# EUROPEAN PATENT APPLICATION

(11) **EP 4 670 720 A1**
(43) Date of publication of application: **31.12.2025**
(21) Application number: 24760456.4
(22) Date of filing: 26.02.2024
(51) Int. Cl.: A61K 31/551, A61K 9/14, A61K 47/20, A61K 47/26, A61K 47/38, A61P 1/04, A61P 1/12, A61P 11/14, A61P 25/00, A61P 25/04, A61P 29/00, A61P 37/08

(54) **SOLID DISPERSION OF COMPOUND HAVING P2X4 RECEPTOR ANTAGONISM**

(30) Priority: 24.02.2023 JP 2023026977
(71) Applicant: Nippon Chemiphar Co., Ltd., Tokyo 101-0032 (JP)
(72) Inventor: ITAI Shigeru, Shizuoka City, Shizuoka 422-8526 (JP); NOGUCHI Syuji, Shizuoka City, Shizuoka 422-8526 (JP); IWAO Yasunori, Shizuoka City, Shizuoka 422-8526 (JP); YAMAKAWA Tomio, Misato City, Saitama 341-0005 (JP)
(74) Representative: Godemeyer Blum Lenze Patentanwälte Partnerschaft mbB - werkpatent
(86) International application number: PCT/JP2024/006678
(87) International publication number: WO 2024/177158

(57) **Abstract**

The present invention provides a solid dispersion comprising a compound represented by a general formula (I) or (II), or a pharmacologically acceptable salt thereof: 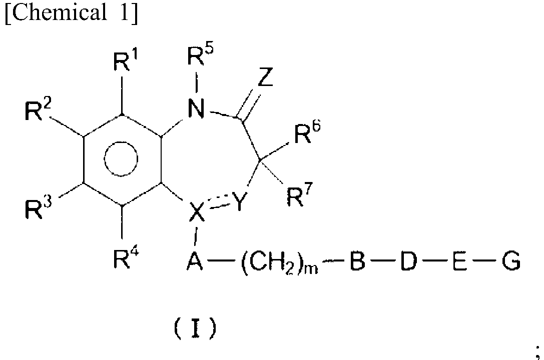 or

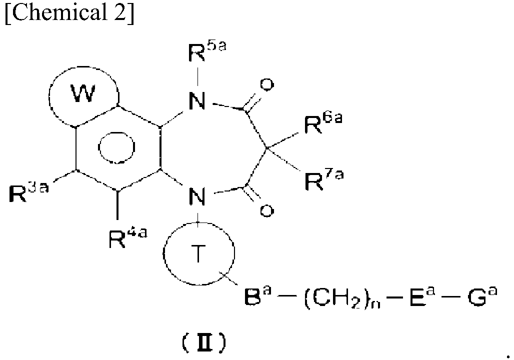

## Description

### Technical Field

The present invention relates to a solid dispersion comprising a compound represented by the general formula (I) or (II) described later (hereinafter referred to as the "present compound") having strong P2X4 receptor antagonistic action or a pharmaceutically acceptable salt thereof. This application claims priority based on Japanese Patent Application No. 2023-26977, filed on February 24, 2022, the contents of which are incorporated herein by reference.

### Background Art

ATP receptors are broadly classified into the P2X family of ion channel receptors and the P2Y family of G protein-coupled receptors, with seven (P2X1 to 7) subtypes and eight (P2Y1, 2, 4, 6, 11 to 14) subtypes reported to date, respectively.

The P2X4 receptor (GenBank No. X87763), a subtype of the P2X family, has been reported to be widely expressed in the central nervous system and other areas (Non-Patent Documents 1 to 5).

The exact mechanism of onset of chronic pain or intractable pain, including neuropathic pain, is not understood, and there is no treatment available when nonsteroidal anti-inflammatory drugs (NSAIDs) or morphine are ineffective. This places a significant burden on the minds and bodies of patients and those around them. Neuropathic pain is often caused by damage to the peripheral or central nervous system. It can be caused by, for example, surgical sequelae, cancer, spinal cord injury, herpes zoster, diabetic neuritis, and trigeminal neuralgia.

The present inventors discovered the present compound that acts as a P2X4 receptor antagonist and filed a patent application (Patent Document 1). Such compounds can be used as therapeutic agents for neuropathic pain, multiple sclerosis, cough suppression, irritable bowel syndrome, inflammatory bowel disease, various allergies, and the like (Patent Documents 2 to 6).

The present inventors have investigated the present compound disclosed in Patent Document 1 and found that it is poorly soluble in water, indicating that there is still room for improvement in terms of its absorbability in the body when administered orally.

Known methods for improving the *in vivo* absorbability of poorly soluble compounds include micronizing the poorly soluble compounds and preparing them into solid dispersions. For example, Patent Document 7 discloses a method for microcrystallizing Istradefylline, and Patent Document 8 discloses a method for preparing a solid dispersion of a thiazolopyrimidine derivative and a hypromellose derivative. To improve the *in vivo* absorbability of poorly soluble compounds by preparing them into solid dispersions, it is necessary to convert the poorly soluble compounds into an amorphous state. However, in general, the amorphous state is unstable in the presence of moisture, and depending on the combination of a poorly soluble compound and a polymer, the poorly soluble compound may transition from an amorphous state to a crystalline state during long-term storage. Therefore, there are problems such as the inability to obtain a certain solubility over time.(Non-Patent Documents 6 to 8).

### Prior Art Documents

### Patent Documents

Patent Document 1: WO2013/105608
Patent Document 2: WO2017/188365
Patent Document 3: WO2019/177117
Patent Document 4: WO2020/050253
Patent Document 5: WO2021/198745
Patent Document 6: WO2022/030428
Patent Document 7: WO2004/099207
Patent Document 8: WO2019/107412

### Non-Patent Documents

Non-Patent Document 1: Buell et al.(1996) EMBO J.15:55-62
Non-Patent Document 2: Seguela et al.(1996) J.Neurosci.16:448-455
Non-Patent Document 3: Bo et al.(1995) FEBS Lett.375:129-133
Non-Patent Document 4: Soto et al.(1996) Proc Natl. Acad. Sci. USA 93:3684-3788
Non-Patent Document 5: Wang et al.(1996) Biochem. Res. Commun. 220:196-202
Non-Patent Document 6: Shibata Y. Yakuzaigaku., 71(1), 50-54 (2011)
Non-Patent Document 7: Imaizumi H. Chem. Pharm. Bull., 31, 2510-2512 (1983)
Non-Patent Document 8: Konno H. Yakuzaigaku., 71(2), 109-113(2011)

### Summary of Invention

### Problem to be Solved by the Invention

An object of the present invention is to improve the *in vivo* absorbability of the present compound or a pharmaceutically acceptable salt thereof disclosed in Patent Document 1, and to provide a pharmaceutical composition which is a solid dispersion comprising the compound or a pharmaceutically acceptable salt thereof as an active ingredient.

### Means for Solving the Problem

The present inventors have conducted various studies on improving the absorbability of the present compound or a pharmaceutically acceptable salt thereof disclosed in Patent Document 1, and as a result, have found that the in vivo absorbability can be improved by forming a solid dispersion with a specific polymer or the like. This finding has led to the completion of the present invention.

Specifically, the present invention has the following aspects.
[1] A solid dispersion comprising a compound represented by the following general formula (I) or (II), or a pharmacologically acceptable salt thereof: 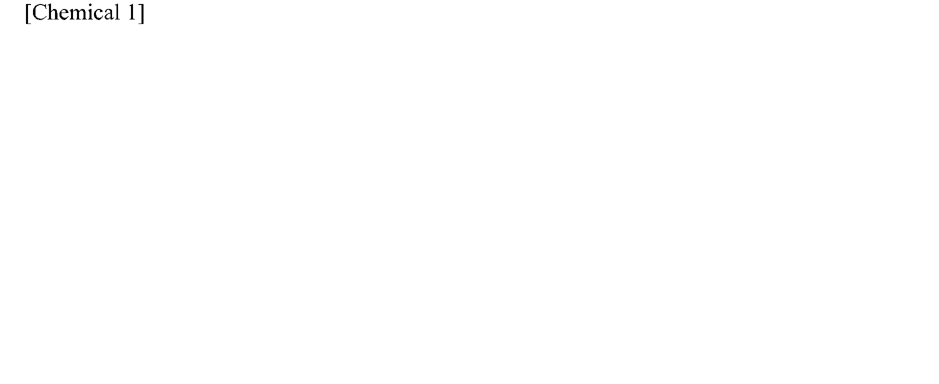 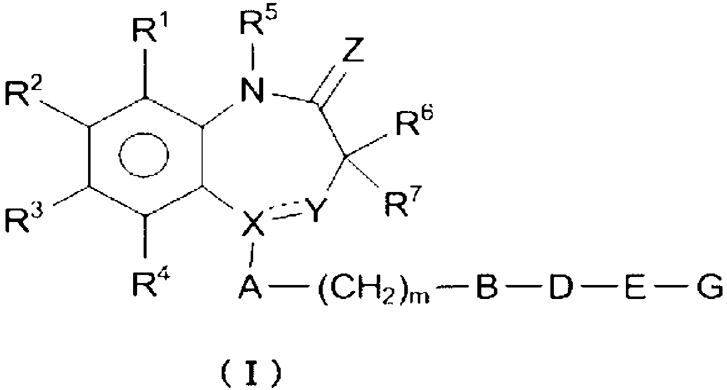 (in the formula, R¹ and R² may be the same or different and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms),a phenyl group which may have a substituent, a pyridyl group which may have a substituent, or an aralkyl group (with an aryl moiety having 6 to 10 carbon atoms and an alkylene moiety having 1 to 8 carbon atoms),
   or
   R¹ and R² may be taken together with a benzene ring to which they are bonded to form a fused ring selected from a naphthalene ring, a quinoline ring, an isoquinoline ring, a tetrahydronaphthalene ring, an indane ring, a tetrahydroquinoline ring, or a tetrahydroisoquinoline ring, and the ring, together with R¹ and R², which consists of carbon atoms to which R¹ and R² are respectively bonded, may be substituted with 1 to 4 substituents that may be the same or different, which are selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
   R³ and R⁴ may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
   R⁵ represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   R⁶ and R⁷ may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group;
   X represents C, CH, or N, and
   Y represents N, NH, or C(=O),
   provided that when X is N, Y is not N or NH, and
   when X is C or CH, Y is not C(=O),
   a double line consisting of a solid line and a dashed line represents a single bond or a double bond;
   Z represents an oxygen atom or a sulfur atom;
   A represents a benzene ring, a pyridine ring, a thiophene ring, a pyrimidine ring, a naphthalene ring, a quinoline ring, or an indole ring which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group, or represents a bond;
   B represents N(R⁸)C(=O), NHCONH, CON(R⁹), NHC(=S)NH, N(R¹⁰)SO₂, SO₂N(R¹¹), or OSO₂,
   wherein R⁸, R⁹, R¹⁰ and R¹¹ represent a hydrogen atom, an alkyl group having 1 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   D represents an alkylene chain having 1 to 6 carbon atoms which may have 1 to 4 substituents that may be the same or different, which are selected from an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms), and which may further have a double bond, or represents a bond;
   E represents O, S, NR¹², or a bond,
   wherein R¹² represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   G represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (with an aryl moiety having 6 to 10 carbon atoms and an alkylene moiety having 1 to 8 carbon atoms), a phenyl group which may have a substituent, a pyridyl group which may have a substituent, an imidazolyl group which may have a substituent, an oxazolyl group which may have a substituent, or a thiazolyl group which may have a substituent; and
   m represents an integer from 0 to 5,
   wherein provided that a case in which when R¹ and R² do not together form a ring, X is C, Y is N, a double line consisting of a solid line and a dashed line is a double bond, Z is an oxygen atom, A is a benzene ring, m is 0, B is C(=O)NH, E is a bond, and G is a phenyl group,
   a case in which R¹, R², R⁴, R⁵, R⁶ and R⁷ are hydrogen atoms, R³ is a chlorine atom, X is C, Y is N, a double line consisting of a solid line and a dashed line is a double bond, Z is an oxygen atom, A is a bond, m is 1, B is NHC(=O), D is a methylene group or a bond, E is a bond, and G is a phenyl group,
   and a case in which R¹, R², R³, R⁴, R⁵, R⁶ and R⁷ are hydrogen atoms, X is N, Y is C(=O), a double line consisting of a solid line and a dashed line is a single bond, Z is an oxygen atom, A is a bond, m is 1, B is CON(R⁹), R⁹ is an isopropyl group, E is a bond, G is a phenyl group with an optional substitution of a methoxy group are excluded). 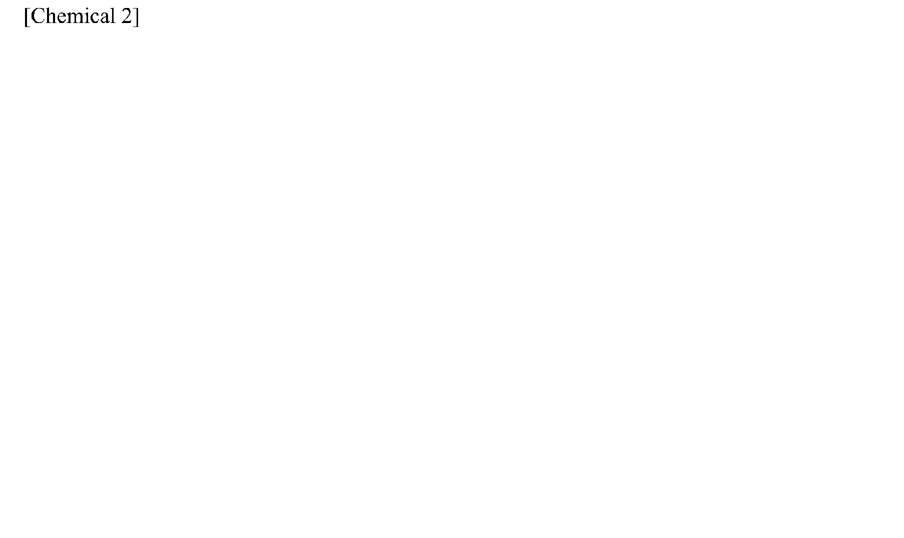 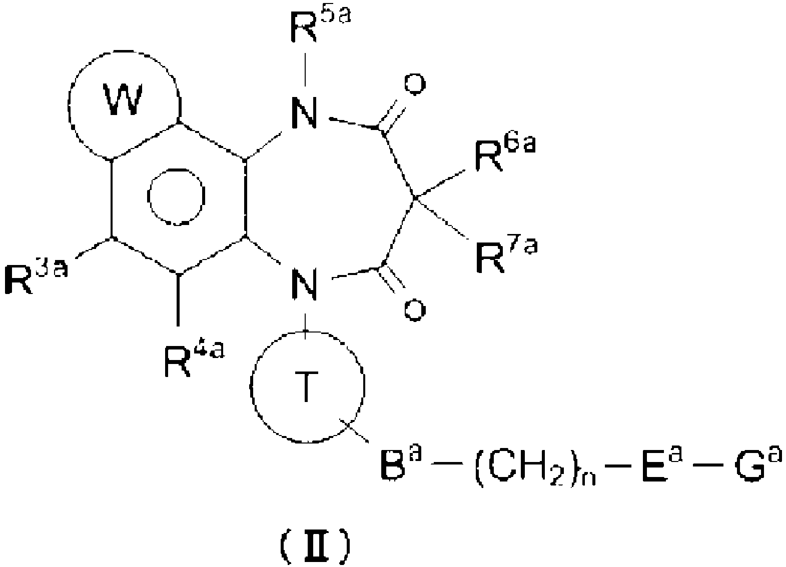 (in the formula, 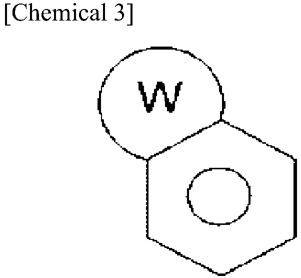 represents a naphthalene ring, a quinoline ring, an isoquinoline ring, a tetrahydronaphthalene ring, an indane ring, a tetrahydroquinoline ring, or a tetrahydroisoquinoline ring;
   these rings may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
   R^{3a} and R^{4a} may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
   R^{5a} represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   R^{6a} and R^{7a} may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group; 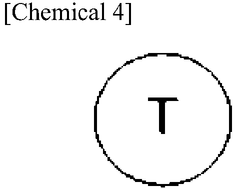 represents a benzene ring, a pyridine ring, a thiophene ring, a pyrimidine ring, a naphthalene ring, a quinoline ring, or an indole ring which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group, or represents a bond;
   B^{a} represents N(R^{8a})C(=O), NHCONH, CON(R^{9a}), NHC(=S)NH, N(R^{10a})SO₂, SO₂N(R^{11a}), or OSO₂,
   wherein R^{8a}, R^{9a}, R^{10a} and R^{11a} represent a hydrogen atom, an alkyl group having 1 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   E^{a} represents O, S, NR^{12a}, or a bond,
   wherein R^{12a} represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
   G^{a} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms,
      an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (with an aryl moiety having 6 to 10 carbon atoms and an alkylene moiety having 1 to 8 carbon atoms), a phenyl group which may have a substituent, a pyridyl group which may have a substituent, an imidazolyl group which may have a substituent, an oxazolyl group which may have a substituent, or a thiazolyl group which may have a substituent; and
   n represents an integer from 0 to 5).
[2] The solid dispersion according to item [1], comprising a combination of hydroxypropylmethylcellulose (HPMC) and sodium lauryl sulfate (SLS), or aminoalkyl methacrylate copolymer E.
[3] The solid dispersion according to item [2], comprising a combination of hydroxypropylmethylcellulose (HPMC) and sodium lauryl sulfate (SLS).
[4] The solid dispersion according to any one of items [1] to [3], wherein the compound is the compound represented by the general formula (II), in which B^{a} is an NHCO group or an NHSO₂ group bonded to a 3rd or 4th position of a benzene ring, n is 0 to 2, E^{a} is a bond, and G^{a} is a substituted phenyl group or a substituted pyridyl group.
[5] The solid dispersion according to any one of items [1] to [4], wherein B^{a} is an NHCO group that binds to a 4th position of a benzene ring, n is 0, and G^{a} is a substituted phenyl group.
[6] The solid dispersion according to any one of items [1] to [5], wherein G^{a} is a trifluoromethyl group, a hydroxyl group, an alkyl group, or a phenyl group in which G^{a} is substituted with a halogen atom.
[7] The solid dispersion according to any one of items [1] to [3], wherein the compound or a pharmacologically acceptable salt thereof is selected from the following group of (1) to (214):
   (1) 5-(4-benzoylaminophenyl)-1H-naphtho [1,2-b] [1,4] diazepine-2,4 (3H,5H)-dione;
   (2) 5-[4-[(2-(trifluoromethyl) benzoyl] aminophenyl]-1H-naphtho [1,2-b] [1,4] diazepine-2,4 (3H,5H)-dione;
   (3) 5-[4-(3-bromobenzoyl) aminophenyl]-1H-naphtho [1,2-b] [1,4] diazepine-2,4 (3H,5H)-dione;
   (4) 5-[4-[4-(trifluoromethyl) benzoyl] aminophenyl]-1H-naphtho [1,2-b] [1,4] diazepine-2,4 (3H,5H)-dione;
   (5) 5-[4-(2-methylbenzoyl) aminophenyl]-1H-naphtho [1,2-b] [1,4] diazepine-2,4 (3H,5H)-dione;
   (6) 5-[4-(2,6-dimethylbenzoylamino) phenyl]-1H-naphtho [1,2-b] [1,4] diazepine-2,4 (3H,5H)-dione;
   (7) 5-[4-(2,6-dichlorobenzoyl) aminophenyl]-1H-naphtho [1,2-b] [1,4] diazepine-2,4 (3H,5H)-dione;
   (8) 5-[4-(3-chlorobenzoyl) aminophenyl]-1H-naphtho [1,2-b] [1,4] diazepine-2,4 (3H,5H)-dione;
   (9) 5 [4-(2-phenylacetylamino) phenyl]-1H-naphtho [1,2-b] [1,4] diazepine-2,4 (3H,5H)-dione;
   (10) 1-[4-(2,4-dioxo-1,2,3,4-tetrahydronaphtho [1,2-b] [1,4] diazepin-5-yl) phenyl]-3-phenylthiourea;
   (11) 5-[4-(2,3-dimethoxybenzoylamino) phenyl]-1H-naphtho [1,2-b] [1,4] diazepine-2,4 (3H,5H)-dione;
   (12) 5-[4-(2-methoxybenzoylamino) phenyl]-1H-naphtho [1,2-b] [1,4] diazepine-2,4 (3H,5H)-dione;
   (13) 5-[4-[(2-chlorophenylacetyl) amino] phenyl]-1H-naphtho [1,2-b] [1,4] diazepine-2,4 (3H,5H)-dione;
   (14) 5-[4-(2,3-dimethylbenzoylamino) phenyl]-1H-naphtho [1,2-b] [1,4] diazepine-2,4 (3H,5H)-dione;
   (15) 5-[4-(2,5-dimethylbenzoylamino) phenyl]-1H-naphtho [1,2-b] [1,4] diazepine-2,4 (3H,5H)-dione;
   (16) 5-[4-(5-bromo-2-chlorobenzoylamino) phenyl]-1H-naphtho [1,2-b] [1,4] diazepine-2,4 (3H,5H)-dione;
   (17) 5-[4-(2,4-dichlorobenzoylamino) phenyl]-1H-naphtho [1,2-b] [1,4] diazepine-2,4 (3H,5H)-dione;
   (18) 5-[4-(2-hydroxybenzoylamino) phenyl]-1H-naphtho [1,2-b] [1,4] diazepine-2,4 (3H,5H)-dione;
   (19) 5-[4-(2,3-dihydroxybenzoylamino) phenyl]-1H-naphtho [1,2-b] [1,4] diazepine-2,4 (3H,5H)-dione;
   (20) 1-[4-(2,4-dioxo-1,2,3,4-tetrahydronaphtho [1,2-b] [1,4] diazepin-5-yl) phenyl]-3-phenylurea;
   (21) 5-[4-[(2,6-dichlorophenylacetyl) amino] phenyl]-1H-naphtho [1,2-b] [1,4] diazepine-2,4 (3H,5H)-dione;
   (22) 5-[4-[(2-methoxyphenylacetyl) amino] phenyl]-1H-naphtho [1,2-b] [1,4] diazepine-2,4 (3H,5H)-dione;
   (23) 5-[4-[(2-hydroxyphenylacetyl) amino] phenyl]-1H-naphtho [1,2-b] [1,4] diazepine-2,4 (3H,5H)-dione;
   (24) 1-(2-chlorophenyl)-3-[4-(2,4-dioxo-1,2,3,4-tetrahydronaphtho [1,2-b] [1,4] diazepin-5-yl) phenyl] thiourea;
   (25) 5-[4-[3-(trifluoromethyl) benzoylamino] phenyl]-1H-naphtho [1,2-b] [1,4] diazepine-2,4 (3H,5H)-dione;
   (26) 5-[4-[2-[(2-trifluoromethyl) phenyl] acetylamino] phenyl]-1H-naphtho [1,2-b] [1,4] diazepine-2,4 (3H,5H)-dione;
   (27) 1-(2-chlorophenyl)-3-[4-(2,4-dioxo-1,2,3,4-tetrahydronaphtho [1,2-b] [1,4] diazepin-5-yl) phenyl] urea;
   (28) 5-[4-[(2-phenylpropionyl) amino] phenyl]-1H-naphtho [1,2-b] [1,4] diazepine-2,4 (3H,5H)-dione;
   (29) 5-[4-(2-chloro-3-methoxybenzoylamino) phenyl]-1H-naphtho [1,2-b] [1,4] diazepine-2,4 (3H,5H)-dione;
   (30) 5-[4-(3-phenylpropionylamino) phenyl]-1H-naphtho [1,2-b] [1,4] diazepine-2,4 (3H,5H)-dione;
   (31) 5-[4-[(1H-indole-3-carbonyl) amino] phenyl]-1H-naphtho [1,2-b] [1,4] diazepine-2,4 (3H,5H)-dione;
   (32) 5-[4-(2-chloro-3-hydroxybenzoylamino) phenyl]-1H-naphtho [1,2-b] [1,4] diazepine-2,4 (3H,5H)-dione;
   (33) 5-[4-[(2-methyl-2-phenylpropionyl) amino] phenyl]-1H-naphtho [1,2-b] [1,4] diazepine-2,4 (3H,5H)-dione;
   (34) 5-[4-(2-phenoxyacetylamino) phenyl]-1H-naphtho [1,2-b] [1,4] diazepine-2,4 (3H,5H)-dione;
   (35) 5-[4-[2-(2-chloro-4-methoxyphenyl) acetylamino] phenyl]-1H-naphtho [1,2-b] [1,4] diazepine-2,4 (3H,5H)-dione;
   (36) 5-[4-[(1-methyl-1H-imidazole-2-carbonyl) amino] phenyl]-1H-naphtho [1,2-b] [1,4] diazepine-2,4 (3H,5H)-dione;
   (37) 5-[4-[2-(2,4-dichlorophenyl) acetylamino] phenyl]-1H-naphtho [1,2-b] [1,4] diazepine-2,4 (3H,5H)-dione;
   (38) 5-[4-[2-(2-chloro-4-hydroxyphenyl) acetylamino] phenyl]-1H-naphtho [1,2-b] [1,4] diazepine-2,4 (3H,5H)-dione;
   (39) 5-[4-(3-phenylpropenylamino) phenyl]-1H-naphtho [1,2-b] [1,4] diazepine-2,4 (3H,5H)-dione;
   (40) 5-[4-[(3-pyridylacetyl) amino] phenyl]-1H-naphtho [1,2-b] [1,4] diazepine-2,4 (3H,5H)-dione hydrochloride;
   (41) 5-[4-(1H-benzimidazole-2-carbonylamino) phenyl]-1H-naphtho [1,2-b] [1,4] diazepine-2,4 (3H,5H)-dione;
   (42) 1-[4-(2,3-dimethylbenzoylamino) phenyl]-7-methoxy-1H-1,5-benzodiazepine-2,4 (3H,5H)-dione;
   (43) 5-[4-[(benzoylamino) methyl] phenyl]-1H-naphtho [1,2-b] [1,4] diazepine-2,4 (3H,5H)-dione;
   (44) 5-[4-[(2-chlorobenzoylamino) methyl] phenyl]-1H-naphtho [1,2-b] [1,4] diazepine-2,4 (3H,5H)-dione;
   (45) 1-[4-(2,3-dimethylbenzoylamino) phenyl]-7-hydroxy-1H-1,5-benzodiazepine-2,4 (3H,5H)-dione;
   (46) 5-[4-(2-chlorobenzoylamino) phenyl]-1H-naphtho [1,2-b] [1,4] diazepine-2,4 (3H,5H)-dione;
   (47) 5-[4-(2-bromobenzoylamino) phenyl]-1H-naphtho [1,2-b] [1,4] diazepine-2,4 (3H,5H)-dione;
   (48) 5-[4-(2-iodobenzoylamino) phenyl]-1H-naphtho [1,2-b] [1,4] diazepine-2,4 (3H,5H)-dione;
   (49) 5-[4-(2,3-dimethylbenzoylamino)-3-fluorophenyl]-1H-naphtho [1,2-b] [1,4] diazepine-2,4 (3H,5H)-dione;
   (50) 5-[4-[2-(2-methylphenyl) acetylamino] phenyl]-1H-naphtho [1,2-b] [1,4] diazepine-2,4 (3H,5H)-dione;
   (51) 5-[4-[(quinoxalin-2yl) carbonylamino] phenyl]-1H-naphtho [1,2-b] [1,4] diazepine-2,4 (3H,5H)-dione;
   (52) 5-[4-[(5-methylthiophen-2yl) carbonylamino] phenyl]-1H-naphtho [1,2-b] [1,4] diazepine-2,4 (3H,5H)-dione;
   (53) 5-[3-[(2-chlorophenylacetyl) amino] phenyl]-1H-naphtho [1,2-b] [1,4] diazepine-2,4 (3H,5H)-dione;
   (54) 5-[4-[(2,4,6-trimethylbenzoyl) amino] phenyl]-1H-naphtho [1,2-b] [1,4] diazepine-2,4 (3H,5H)-dione;
   (55) 5-[4-(cyclohexylcarbonylamino) phenyl]-1,2,3,4,8,9,10,11-octahydronaphtho [1,2-b] [1,4] diazepine-2, 4 (3H,5H)-dione;
   (56) 1-[4-(2,3-dimethylbenzoyl) aminophenyl]-6-methyl-1H-1,5-benzodiazepine-2,4 (3H,5H)-dione;
   (57) 5-[4-[(2-ethylbenzoyl) amino] phenyl]-1H-naphtho [1,2-b] [1,4] diazepine-2,4 (3H,5H)-dione;
   (58) 5-[4-[(6-methylpyridin-2-yl) carbonylamino] phenyl]-1,2,3,4,8,9,10,11-octahydronaphtho [1,2-b ] [1,4] diazepine-2,4 (3H,5H)-dione;
   (59) 5-[4-[(2-methylpyridin-3-yl) carbonylamino] phenyl]-1,2,3,4,8,9,10,11-octahydronaphtho [1,2-b] [1,4] diazepine-2,4 (3H,5H)-dione;
   (60) 1-[4-(2,4-dioxo-1,2,3,4-tetrahydronaphtho [1,2-b] [1,4] diazepin-5-yl) phenyl]-3-(2-methylphenyl) thiourea;
   (61) 5-[4-(2-methoxy-3-methylbenzoyl) aminophenyl]-1,2,3,4,8,9,10,11-octahydronaphtho [1,2-b] [1,4] diazepine-2,4 (3H,5H)-dione;
   (62) 5-[4-(2,3-dichlorobenzoyl) aminophenyl]-1,2,3,4,8,9,10,11-octahydronaphtho [1,2-b] [1,4] diazepine-2,4 (3H,5H)-dione;
   (63) 5-[4-(2,3-dimethylbenzoylamino)-3-hydroxyphenyl]-1H-naphtho [1,2-b] [1,4] diazepine-2,4 (3H,5H)-dione;
   (64) 5-[4-(2-chloro-3-methoxybenzoylamino) phenyl]-1,3-dihydronaphtho [1,2-e]-1,4-diazepin-2-one;
   (65) 5-[4-[(4-dimethylaminobenzoyl) amino] phenyl]-1H-naphtho [1,2-b] [1,4] diazepine-2,4 (3H,5H)-dione;
   (66) 5-[4-[2-(2,4-dichlorophenoxy) acetylamino] phenyl]-1H-naphtho [1,2-b] [1,4] diazepine-2,4 (3H,5H)-dione;
   (67) 5-[4-[2-(2-methylphenoxy) acetylamino] phenyl]-1H-naphtho [1,2-b] [1,4] diazepine-2,4 (3H,5H)-dione;
   (68) N-[4-(2,4-dioxo-1,2,3,4-tetrahydronaphtho [1,2-b] [1,4] diazepin-5-yl) butyl]-2-chloro -3-methoxybenzamide;
   (69) 5-[4-(2-chloro-3-hydroxybenzoylamino) phenyl]-1,3-dihydronaphtho [1,2-e]-1,4-diazepin-2-one;
   (70) 5-[4-(2-acetylbenzoylamino) phenyl]-1H-naphtho [1,2-b] [1,4] diazepine-2,4 (3H,5H)-dione;
   (71) 5-[4-(2-tert-butylbenzoylamino) phenyl]-1H-naphtho [1,2-b] [1,4] diazepine-2,4 (3H,5H)-dione;
   (72) 5-[2-(2-iodobenzoyl) aminoethyl]-1H-naphtho [1,2-b] [1,4] diazepine-2,4 (3H,5H)-dione;
   (73) 5-[3-[(2-iodobenzoyl) amino] phenyl]-1H-naphtho [1,2-b] [1,4] diazepine-2,4 (3H,5H)-dione;
   (74) 6,7-dimethyl-1-[4-(2-iodobenzoyl) aminophenyl]-1H-1,5-benzodiazepine-2,4 (3H,5H)-dione;
   (75) 5-[4-[(1-methylpiperidin-4-yl) carbonylamino] phenyl]-1H-naphtho [1,2-b] [1,4] diazepine-2,4 (3H,5H)-dione hydrochloride;
   (76) 5 [4-[(benzofuran-2-yl) carbonylamino] phenyl]-1H-naphtho [1,2-b] [1,4] diazepine-2,4 (3H,5H)-dione;
   (77) 5-[4-[(1-methyl-1H-indol-3-yl) carbonylamino] phenyl]-1H-naphtho [1,2-b] [1,4] diazepine-2,4 (3H,5H)-dione;
   (78) 5-[4-(2-propenylbenzoylamino) phenyl]-1H-naphtho [1,2-b] [1,4] diazepine-2,4 (3H,5H)-dione;
   (79) 5-[4-(2-propylbenzoylamino) phenyl]-1H-naphtho [1,2-b] [1,4] diazepine-2,4 (3H,5H)-dione;
   (80) 5-[3-fluoro-4-(2-iodobenzoylamino) phenyl]-1H-naphtho [1,2-b] [1,4] diazepine-2,4 (3H,5H)-dione;
   (81) 5-[4-(2-hydroxy-3-methylbenzoyl) aminophenyl]-1,2,3,4,8,9,10,11-octahydronaphtho [1,2-b] [1,4] diazepine-2,4 (3H,5H)-dione;
   (82) 5-[4-[(2-isopropoxybenzoyl) amino] phenyl]-1H-naphtho [1,2-b] [1,4] diazepine-2,4 (3H,5H)-dione;
   (83) 5-[4-[(3-methylthiophen-2-yl) carbonylamino] phenyl]-1H-naphtho [1,2-b] [1,4] diazepine-2,4 (3H,5H)-dione;
   (84) 5-[4-(2-phenoxypropionylamino) phenyl]-1H-naphtho [1,2-b] [1,4] diazepine-2,4 (3H,5H)-dione;
   (85) 5-[4-[2-(4-chloro-2-methylphenoxy) acetylamino] phenyl]-1H-naphtho [1,2-b] [1,4] diazepine-2,4 (3H,5H)-dione;
   (86) 5-[4-(4-fluoro-2-trifluoromethylbenzoyl) aminophenyl]-1H-naphtho [1,2-b] [1,4] diazepine-2,4 (3H,5H)-dione;
   (87) 5-[4-(4-fluoro-2-methoxybenzoyl) aminophenyl]-1H-naphtho [1,2-b] [1,4] diazepine-2,4 (3H,5H)-dione;
   (88) 5-[4-(4-fluoro-2-hydroxybenzoyl) aminophenyl]-1H-naphtho [1,2-b] [1,4] diazepine-2,4 (3H,5H)-dione;
   (89) 5-[3-[(2-iodophenylacetyl) amino] phenyl]-1H-naphtho [1,2-b] [1,4] diazepine-2,4 (3H,5H)-dione;
   (90) 5-[4-(2-methyl-2-phenoxypropionylamino) phenyl]-1H-naphtho [1,2-b] [1,4] diazepine-2,4 (3H,5H)-dione;
   (91) 5-[4-(2-tert-butylbenzoylamino) phenyl]-1,3-dihydronaphtho [1,2-e]-1,4-diazepin-2-one;
   (92) 5-[4-[(3-dimethylaminobenzoyl) amino] phenyl]-1H-naphtho [1,2-b] [1,4] diazepine-2,4 (3H,5H)-dione;
   (93) 5-[4-(4-iodo-2-methoxybenzoylamino) phenyl]-1H-naphtho [1,2-b] [1,4] diazepine-2,4 (3H,5H)-dione;
   (94) 5-[4-(6-fluoro-2-methoxybenzoylamino) phenyl]-1H-naphtho [1,2-b] [1,4] diazepine-2,4 (3H,5H)-dione;
   (95) 5-[4-(2-hydroxy-4-iodobenzoylamino) phenyl]-1H-naphtho [1,2-b] [1,4] diazepine-2,4 (3H,5H)-dione;
   (96) 5-[4-(6-fluoro-2-hydroxyamino) phenyl]-1H-naphtho [1,2-b] [1,4] diazepine-2,4 (3H,5H)-dione;
   (97) 5-[4-(2-fluorobenzoyl) aminophenyl]-1H-naphtho [1,2-b] [1,4] diazepine-2,4 (3H,5H)-dione;
   (98) 5-[4-[(2-dimethylaminobenzoyl) amino] phenyl]-1H-naphtho [1,2-b] [1,4] diazepine-2,4 (3H,5H)-dione;
   (99) 5-[4-(2-methoxy-6-methylbenzoylamino) phenyl]-1H-naphtho [1,2-b] [1,4] diazepine-2,4 (3H,5H)-dione;
   (100) 5-[4-(2-hydroxy-6-methylbenzoylamino) phenyl]-1H-naphtho [1,2-b] [1,4] diazepine-2,4 (3H,5H)-dione;
   (101) 5-[4-[3-(2-methylphenyl) propionylamino] phenyl]-1H-naphtho [1,2-b] [1,4] diazepine-2,4 (3H,5H)-dione;
   (102) 5-(4-phenylcarbamoylphenyl) -1H-naphtho [1,2-b] [1,4] diazepine-2,4 (3H,5H)-dione;
   (103) 5-(4-benzylcarbamoylphenyl)-1H-naphtho [1,2-b] [1,4] diazepine-2,4 (3H,5H)-dione;
   (104) 5-[4-[3-(2-methylphenyl) propenoylamino] phenyl]-1H-naphtho [1,2-b] [1,4] diazepine-2,4 (3H,5H)-dione;
   (105) 5-[4-[3-(2-chlorophenyl) propionylamino] phenyl]-1H-naphtho [1,2-b] [1,4] diazepine-2,4 (3H,5H)-dione;
   (106) 5-[4-(2-iodobenzoyl) aminophenyl]-1,2,3,4,8,9,10,11-octahydronaphtho [1,2-b] [1,4] diazepine-2,4 (3H,5H)-dione;
   (107) 5-[4-[(1-methyl-1H-pyrrol-2-ylacetyl) amino] phenyl]-1,2,3,4,8,9,10,11-octahydronaphtho [1,2-b] [1,4] diazepine-2,4 (3H,5H)-dione;
   (108) 5-[4-(2-chlorobenzyl) carbamoylphenyl]-1H-naphtho [1,2-b] [1,4] diazepine-2,4 (3H,5H)-dione;
   (109) 5-[4-[3-(2-chlorophenyl) propenoylamino] phenyl]-1H-naphtho [1,2-b] [1,4] diazepine-2,4 (3H,5H)-dione;
   (110) 5-[4-(2-chlorophenyl) carbamoylphenyl]-1H-naphtho [1,2-b] [1,4] diazepine-2,4 (3H,5H)-dione;
   (111) 5-[4-(6-bromo-2,3-methylenedioxybenzoylamino) phenyl]-1H-naphtho [1,2-b] [1,4] diazepine-2,4 (3H,5H)-dione;
   (112) 5-[4-(6-bromo-2-methoxybenzoylamino) phenyl]-1H-naphtho [1,2-b] [1,4] diazepine-2,4 (3H,5H)-dione;
   (113) 5-[4-[(2-tert-butylbenzoyl) amino] phenyl]-1,2,3,4,8,9,10,11-octahydronaphtho [1,2-b] [1,4] diazepine-2,4 (3H,5H)-dione;
   (114) 5-[2-(2-iodobenzoyl) aminopyridin-5-yl]-1H-naphtho [1,2-b] [1,4] diazepine-2,4 (3H,5H)-dione;
   (115) 5-[4-(6-bromo-2-hydroxybenzoylamino) phenyl]-1H-naphtho [1,2-b] [1,4] diazepine-2,4 (3H,5H)-dione;
   (116) 5-[4-(6-chloro-2-methoxybenzoylamino) phenyl]-1H-naphtho [1,2-b] [1,4] diazepine-2,4 (3H,5H)-dione;
   (117) 5-[4-(2-iodobenzoylamino) phenyl]-1H-[1,4] diazepino [2,3-h] quinoline-2,4 (3H,5H)-dione;
   (118) 5-[4-(6-chloro-2-hydroxybenzoylamino) phenyl]-1H-naphtho [1,2-b] [1,4] diazepine-2,4 (3H,5H)-dione;
   (119) 5-[4-(2-hydroxy-6-methoxybenzoylamino) phenyl]-1H-naphtho [1,2-b] [1,4] diazepine-2,4 (3H,5H)-dione;
   (120) 5-[4-[2-methoxy-6-(trifluoromethyl) benzoylamino] phenyl]-1H-naphtho [1,2-b] [1,4] diazepine-2,4 (3H,5H)-dione;
   (121) 5-[4-[2-hydroxy-6-(trifluoromethyl) benzoylamino] phenyl]-1H-naphtho [1,2-b] [1,4] diazepine-2,4 (3H,5H)-dione;
   (122) 5-[4-[(2-isopropenylbenzoyl) amino] phenyl]-1,2,3,4,8,9,10,11-octahydronaphtho [1,2-b] [1,4] diazepine-2,4 (3H,5H)-dione;
   (123) 5-[4-[(2-isopropylbenzoyl) amino] phenyl]-1,2,3,4,8,9,10,11-octahydronaphtho [1,2-b] [1,4] diazepine-2,4 (3H,5H)-dione;
   (124) 5-[4-[2-chloro-5-(methylthio) benzoylamino] phenyl]-1H-naphtho [1,2-b] [1,4] diazepine-2,4 (3H,5H)-dione;
   (125) 5-[4-[2-(methylthio) benzoylamino] phenyl]-1H-naphtho [1,2-b] [1,4] diazepine-2,4 (3H,5H)-dione;
   (126) 5-[4-[3-(methylthio) benzoylamino] phenyl]-1H-naphtho [1,2-b] [1,4] diazepine-2,4 (3H,5H)-dione;
   (127) 5-[4-[2-ethyl-6-methoxybenzoylamino] phenyl]-1H-naphtho [1,2-b] [1,4] diazepine-2,4 (3H,5H)-dione;
   (128) 5-[4-(3-methanesulfonylbenzoylamino) phenyl]-1H-naphtho [1,2-b] [1,4] diazepine-2,4 (3H,5H)-dione;
   (129) 6-ethyl-1-[4-(2-iodobenzoyl) aminophenyl]-1H-1,5-benzodiazepine-2,4 (3H,5H)-dione;
   (130) 5-[4-[2-ethyl-6-hydroxybenzoylamino] phenyl]-1H-naphtho [1,2-b] [1,4] diazepine-2,4 (3H,5H)-dione;
   (131) 5-[4-(3-methanesulfinylbenzoylamino) phenyl]-1H-naphtho [1,2-b] [1,4] diazepine-2,4 (3H,5H)-dione;
   (132) 5-[4-(2-chloro-5-methanesulfinylbenzoylamino) phenyl]-1H-naphtho [1,2-b] [1,4] diazepine-2,4 (3H,5H)-dione;
   (133) 5-[4-(2-methanesulfinylbenzoylamino) phenyl]-1H-naphtho [1,2-b] [1,4] diazepine-2,4 (3H,5H)-dione;
   (134) 5-[4-[[2-(4-morpholinyl) acetyl] amino] phenyl]-1,2,3,4,8,9,10,11-octahydronaphtho [1,2-b] [1,4] diazepine-2,4 (3H,5H)-dione hydrochloride;
   (135) 5-[4-(2-chloro-6-methoxybenzoylamino) phenyl]-1,3-dihydronaphtho [1,2-e]-1,4-diazepin-2-one;
   (136) 5-[4-[[(3-chloropyridin-2-yl) carbonyl] amino] phenyl]-1H-naphtho [1,2-b] [1,4] diazepine-2,4 (3H,5H)-dione;
   (137) 5-[4-(2-chloro-6-hydroxybenzoylamino) phenyl]-1,3-dihydronaphtho [1,2-e]-1,4-diazepin-2-one;
   (138) 5-[4-(3-chloro-2-methoxybenzoylamino) phenyl]-1H-naphtho [1,2-b] [1,4] diazepine-2,4 (3H,5H)-dione;
   (139) 5-[4-[(3-methylpyridin-2-yl) carbonylamino] phenyl]-1H-naphtho [1,2-b] [1,4] diazepine-2,4 (3H,5H)-dione;
   (140) 5-[4-[[(3-chloropyridin-2-yl) carbonyl] amino] phenyl]-1,2,3,4,8,9,10,11-octahydronaphtho [1,2-b] [1,4] diazepine-2,4 (3H,5H)-dione;
   (141) 5-[4-(3-chloro-2-hydroxybenzoylamino) phenyl]-1H-naphtho [1,2-b] [1,4] diazepine-2,4 (3H,5H)-dione;
   (142) 5-[4-[[(3-hydroxypyridin-2-yl) carbonyl] amino] phenyl]-1H-naphtho [1,2-b] [1,4] diazepine-2,4 (3H,5H)-dione;
   (143) 5-[4-[(3-vinylpyridin-2-yl) carbonylamino] phenyl]-1H-naphtho [1,2-b] [1,4] diazepine-2,4 (3H,5H)-dione;
   (144) 5-[4-[(3-ethylpyridin-2-yl) carbonylamino] phenyl]-1H-naphtho [1,2-b] [1,4] diazepine-2,4 (3H,5H)-dione;
   (145) N-[4-(2,4-dioxo-1,2,3,4-tetrahydronaphtho-[1,2-b] [1,4]-diazepin-5-yl)phenyl]-2-nitrobenzenesulfonamide;
   (146) N-[4-(2,4-dioxo-1,2,3,4-tetrahydronaphtho [1,2-b] [1,4] diazepin-5-yl) phenyl] benzenesulfonamide;
   (147) 3-bromo-N-[4-(2,4-dioxo-1,2,3,4-tetrahydronaphtho [1,2-b] [1,4] diazepin-5-yl) phenyl] benzenesulfonamide;
   (148) N-[4-(2,4-dioxo-1,2,3,4-tetrahydronaphtho [1,2-b] [1,4] diazepin-5-yl) phenyl]-3-methoxybenzenesulfonamide;
   (149) N-[3-(2-oxo-2,3-dihydro-1H-naphtho [1,2-e] [1,4] diazepin-5-yl) phenyl] benzenesulfonamide;
   (150) N-[3-(2,4-dioxo-1,2,3,4-tetrahydronaphtho-[1,2-b] [1,4]-diazepin-5-yl) phenyl]-2-nitrobenzenesulfonamide;
   (151) N-[4-(2,4-dioxo-1,2,3,4,8,9,10,11-octahydro-naphtho [1,2-b] [1,4]-diazepin-5-yl) phenyl]-2-nitro-benzenesulfonamide;
   (152) N-[4-(2,4-dioxo-1,2,3,4,8,9,10,11-octahydro-naphtho [1,2-b] [1,4]-diazepin-5-yl) phenyl]-N-methyl-2-nitrobenzenesulfonamide;
   (153) N-[3-(2,4-dioxo-1,2,3,4-tetrahydronaphtho-[1,2-b] [1,4]-diazepin-5-yl) phenyl]-N-methyl-2-nitrobenzenesulfonamide;
   (154) 4-(2,4-dioxo-1,2,3,4,8,9,10,11-octahydronaphtho [1,2-b] [1,4] diazepin-5-yl)-N-phenylbenzenesulfonamide;
   (155) N-[3-(2,4-dioxo-1,2,3,4-tetrahydronaphtho [1,2-b]-[1,4] diazepin-5-yl) phenyl]-2-naphthalenesulfonamide;
   (156) N-[3-(2,4-dioxo-1,2,3,4-tetrahydronaphtho [1,2-b]-[1,4] diazepin-5-yl) phenyl]-1-naphthalenesulfonamide;
   (157) N-[4-(2,4-dioxo-1,2,3,4-tetrahydronaphtho [1,2-b] [1,4] diazepin-5yl) phenyl] cyclohexanesulfonamide;
   (158) N-[4-(2,4-dioxo-1,2,3,4-tetrahydronaphtho [1,2-b] [1,4] diazepin-5yl) phenyl]-3-pyridinesulfonamide hydrochloride;
   (159) N-[4-(2,4-dioxo-1,2,3,4-tetrahydronaphtho [1,2-b] [1,4] diazepin-5-yl) phenyl]-4-isopropylbenzenesulfonamide;
   (160) N-[4-(2,4-dioxo-1,2,3,4-tetrahydronaphtho [1,2-b] [1,4] diazepin-5-yl) phenyl] phenylmethanesulfonamide;
   (161) N-[4-(2,4-dioxo-1,2,3,4-tetrahydronaphtho [1,2-b] [1,4] diazepin-5yl) phenyl]-3-pyridinesulfonamide;
   (162) N-[4-(2,4-dioxo-1,2,3,4-tetrahydronaphtho [1,2-b] [1,4] diazepin-5yl) phenyl]-2-naphthalenesulfonamide;
   (163) 4-(2,4-dioxo-1,2,3,4,8,9,10,11-octahydronaphtho-[1,2-b] [1,4] diazepin-5-yl) phenyl 3-bromobenzene-sulfonate;
   (164) N-benzyl-N-[4-(1-benzyl-2,4-dioxo-1,2,3,4-tetrahydronaphtho [1,2-b] [1,4] diazepin-5yl) phenyl]-2-nitrobenzenesulfonamide;
   (165) N-benzyl-N-[4-(2,4-dioxo-1,2,3,4-tetrahydronaphtho [1,2-b] [1,4] diazepin-5yl) phenyl]-2-nitrobenzenesulfonamide;
   (166) 3-bromo-N-[4-(2,4-dioxo-1,2,3,4-tetrahydronaphtho [1,2-b] [1,4] diazepin-5-yl) phenyl]-N-methylbenzenesulfonamide;
   (167) N-[4-(2,4-dioxo-1,2,3,4-tetrahydronaphtho-[1,2-b] [1,4]-diazepin-5-yl) phenyl]-N-methyl-2-nitrobenzenesulfonamide;
   (168) N-[4-(2,4-dioxo-1,2,3,4-tetrahydronaphtho-[1,2-b] [1,4]-diazepin-5-yl) phenyl]-N-(2-hydroxyethyl)-2-nitrobenzenesulfonamide;
   (169) N-[4-(7-chloro-2,4-dioxo-2,3,4,5-tetrahydro-1H-benzo [b] [1,4] diazepin-1-yl) phenyl] benzenesulfonamide;
   (170) N-[4-(7-bromo-2,4-dioxo-2,3,4,5-tetrahydro-1H-benzo [b] [1,4] diazepin-1-yl) phenyl] benzenesulfonamide;
   (171) N-[4-[(2,4-dioxo-7-(trifluoromethyl)-2,3,4,5-tetrahydro-1H-benzo [b] [1,4] diazepin-1-yl)] phenyl] benzenesulfonamide;
   (172) N-[4-(2,4-dioxo-2,3,4,5-tetrahydro-1H-benzo [b] [1,4] diazepin-1-yl) phenyl] benzenesulfonamide;
   (173) 1-(2-chlorophenyl)-N-[4-(2,4-dioxo-1,2,3,4-tetrahydronaphtho [1,2-b] [1,4] diazepin-5-yl) phenyl] methanesulfonamide;
   (174) 1-(3-bromophenyl)-N-[4-(2,4-dioxo-1,2,3,4-tetrahydronaphtho [1,2-b] [1,4] diazepin-5-yl) phenyl] methanesulfonamide;
   (175) N-[4-(2,4-dioxo-1,2,3,4-tetrahydronaphtho-[1,2-b] [1,4]-diazepin-5-yl)phenyl]-2-trifluoromethylbenzenesulfonamide;
   (176) N-[4-(7-bromo-6-methyl-2,4-dioxo-2,3,4,5-tetrahydro-1H-benzo [b] [1,4] diazepin-1-yl) phenyl] benzenesulfonamide;
   (177) 1-(2-chlorophenyl)-N-[4-(2,4-dioxo-1,2,3,4,8,9,10,11-octahydronaphtho [1,2-b] [ 1,4] diazepin-5-yl) phenyl] methanesulfonamide;
   (178) 3-bromo-N-[4-(2,4-dioxo-1,2,3,4,8,9,10,11-octahydronaphtho [1,2-b] [1,4] diazepin-5-yl) phenyl] benzenesulfonamide;
   (179) N-[4-(2,4-dioxo-1,2,3,4,8,9,10,11-octahydronaphtho [1,2-b] [1,4] diazepin-5-yl) phenyl]-3-methoxybenzenesulfonamide;
   (180) 1-(2-bromophenyl)-N-[4-(2,4-dioxo-1,2,3,4-tetrahydronaphtho [1,2-b] [1,4] diazepin-5-yl) phenyl] methanesulfonamide;
   (181) N-[4-(2,4-dioxo-1,2,3,4-tetrahydronaphtho [1,2-b] [1,4] diazepin-5-yl) phenyl]-1-(2-methylphenyl) methanesulfonamide;
   (182) N-[4-(2,4-dioxo-1,2,3,4-tetrahydronaphtho [1,2-b] [1,4] diazepin-5-yl) phenyl]-1-(2-nitrophenyl) methanesulfonamide;
   (183) N-[4-(2,4-dioxo-1,2,3,4-tetrahydronaphtho [1,2-b] [1,4] diazepin-5-yl) phenyl]-2-phenylethanesulfonamide;
   (184) 1-(2,3-dichlorophenyl)-N-[4-(2,4-dioxo-1,2,3,4,8,9,10,11-octahydronaphtho [1,2-b] [1,4] diazepin-5-yl) phenyl] methanesulfonamide;
   (185) 1-(2-chlorophenyl)-N-[4-(2,4-dioxo-7-methoxy-1H-benzo [1,2-b] [1,4] diazepin-1-yl) phenyl] methanesulfonamide;
   (186) 1-(2-chlorophenyl)-N-[4-(2,4-dioxo-7-hydroxy-1H-benzo [1,2-b] [1,4] diazepin-1-yl) phenyl] methanesulfonamide;
   (187) 1-(4-chlorophenyl)-N-[4-(2,4-dioxo-1,2,3,4-tetrahydronaphtho [1,2-b] [1,4] diazepin-5-yl) phenyl] methanesulfonamide;
   (188) 1-(2-chlorophenyl)-N-[4-(2,4-dioxo-1,2,3,4-tetrahydronaphtho [1,2-b] [1,4] diazepin-5-yl) benzyl] methanesulfonamide;
   (189) 1-(2-chlorophenyl)-N-[4-(2,4-dioxo-1,2,3,4-tetrahydronaphtho [1,2-b] [1,4] diazepin-5-yl)-2-methoxyphenyl] methanesulfonamide;
   (190) 1-(2-chlorophenyl)-N-[4-(2,4-dioxo-1,2,3,4-tetrahydronaphtho [1,2-b] [1,4] diazepin-5-yl)-2-hydroxyphenyl] methanesulfonamide;
   (191) 1-(2,6-dichlorophenyl)-N-[4-(2,4-dioxo-1,2,3,4,8,9,10,11-octahydronaphtho [1,2-b] [1,4] diazepin-5-yl) phenyl] methanesulfonamide;
   (192) 1-(2-chlorophenyl)-N-[4-(2,4-dioxo-6-methyl-1H-benzo [1,2-b] [1,4] diazepin-1-yl) phenyl] methanesulfonamide;
   (193) 1-(2-chlorophenyl)-N-[4-(2,4-dioxy-1,2,3,4-tetrahydronaphtho [1,2-b] [1,4] diazepin-5-yl) propyl] methanesulfonamide;
   (194) 1-(2-chlorophenyl)-N-[2-(2,4-dioxo-1,2,3,4-tetrahydronaphtho [1,2-b] [1,4] diazepin-5-yl) ethyl] methanesulfonamide;
   (195) N-[4-(2,4-dioxo-1,2,3,4,8,9,10,11-octahydronaphtho [1,2-b] [1,4] diazepin-5-yl) phenyl]-1-(2-iodophenyl) methanesulfonamide;
   (196) 1-(2-chlorophenyl)-N-[4-(2,4-dioxo-1,2,3,4,8,9,10,11-octahydronaphtho [1,2-b] [1,4] diazepin-5-yl) phenyl]-N-methylmethanesulfonamide;
   (197) 1-(2-chlorophenyl)-N-[4-(2-oxo-2,3-dihydro-1H-naphtho [1,2-e] [1,4] diazepin-5-yl) phenyl] methanesulfonamide;
   (198) 1-[(2-trifluoromethyl) phenyl]-N-[4-(2,4-dioxo-1,2,3,4-tetrahydronaphtho [1,2-b] [1,4] diazepin-5-yl) phenyl] phenyl-N-methylmethanesulfonamide;
   (199) 1-(2-ethylphenyl)-N-[4-(2,4-dioxo-1,2,3,4-tetrahydronaphtho [1,2-b] [1,4] diazepin-5-yl) phenyl] phenyl-N-methylmethanesulfonamide;
   (200) 1-(2,3-dimethylphenyl)-N-[4-(2,4-dioxo-1,2,3,4-tetrahydronaphtho [1,2-b] [1,4] diazepin-5-yl) phenyl] phenyl-N-methylmethanesulfonamide;
   (201) 2-(2-chlorophenyl)-N-[4-(2,4-dioxo-1,2,3,4-tetrahydronaphtho [1,2-b] [1,4] diazepin-5-yl) phenyl] phenyl-N-methylethanesulfonamide;
   (202) 1-(2-nitrophenyl)-N-[4-(2,4-dioxo-1,2,3,4-tetrahydronaphtho [1,2-b] [1,4] diazepin-5-yl) phenyl] phenyl-N-methylmethanesulfonamide;
   (203) 1-(2-aminophenyl)-N-[4-(2,4-dioxo-1,2,3,4-tetrahydronaphtho [1,2-b] [1,4] diazepin-5-yl) phenyl] phenyl-N-methylmethanesulfonamide;
   (204) 1-(2-dimethylaminophenyl)-N-[4-(2,4-dioxo-1,2,3,4-tetrahydronaphtho [1,2-b] [1,4] diazepin-5-yl) phenyl] phenyl-N-methylmethanesulfonamide;
   (205) 5-[4-[(pyridin-4-yl) carbonylamino] phenyl]-1H-naphtho [1,2-b] [1,4] diazepine-2,4 (3H,5H)-dione hydrochloride;
   (206) 5-[4-[2-[(pyridin-3-yl) oxy] acetylamino] phenyl]-1H-naphtho [1,2-b] [1,4] diazepine-2,4 (3H,5H)-dione hydrochloride;
   (207) 5-[4-[(pyridin-3-yl) carbonylamino] phenyl]-1H-naphtho [1,2-b] [1,4] diazepine-2,4 (3H,5H)-dione hydrochloride;
   (208) 5-[4-[(2-methylpyridin-3-yl) carbonylamino] phenyl]-1H-naphtho [1,2-b] [1,4] diazepine-2,4 (3H,5H)-dione hydrochloride;
   (209) 5-[4-[(2-chloropyridin-3-yl) carbonylamino] phenyl]-1H-naphtho [1,2-b] [1,4] diazepine-2,4 (3H,5H)-dione;
   (210) 5-[4-[2-[(pyridin-2-yl) oxy] acetylamino] phenyl]-1H-naphtho [1,2-b] [1,4] diazepine-2,4 (3H,5H)-dione;
   (211) 5-[4-[[4-(trifluoromethyl) pyridin-3-yl] carbonylamino] phenyl]-1H-naphtho [1,2-b] [1,4] diazepine-2,4 (3H,5H)-dione;
   (212) 5-[4-[(2-chloropyridin-3-yl) carbonylamino] phenyl]-1H-[1,4] diazepino [2,3-f] isoquinoline-2,4 (3H,5H)-dione;
   (213) 5-[4-[(2-chloropyridin-3-yl) carbonylamino] phenyl]-8,9,10,11-tetrahydro-1H-[1,4] diazepino [2,3-f] Isoquinoline-2,4 (3H,5H)-dione; and
   (214) 5-[4-[(2-isopropylbenzoyl) amino] phenyl]-1H-naphtho [1,2-b] [1,4] diazepine-2,4 (3H,5H)-dione.
[8] The solid dispersion according to any one of items [1] to [7], wherein the compound or a pharmacologically acceptable salt thereof is amorphous.
[9] The solid dispersion according to any one of items [1] to [8], which has a carrier ratio of 1 to 25.
[10] The solid dispersion according to any one of items [3] to [9] which has an SLS ratio of 0.1 to 10.
[11] A method for producing the solid dispersion according to any one of items [1] to [10], characterized by producing the solid dispersion using a spray drying method.
[12] A pharmaceutical composition comprising the solid dispersion according to any one of items [1] to [11].
[13] The pharmaceutical composition according to item [12], which is a solid preparation.

### Advantageous Effects of Invention

The solid dispersion of the present invention is helpful as a therapeutic agent for neuropathic pain and the like, as the active ingredient, the present compound, exhibits high *in vivo* absorbability. Furthermore, the pharmaceutical composition of the present invention, which is a solid dispersion, has excellent storage stability.

### Brief Description of Drawings

[Fig. 1] Dissolution properties of various solid dispersions of a compound F48 (1): The results of the dissolution test of solid dispersions (A to J) of the present compound produced using 10 types of carriers comprising polymers are shown.
[Fig.2] Dissolution properties of various solid dispersions of the compound F48 (2): The results of the dissolution test of various solid dispersions (B1 to B5, E1 to E3) with different carrier ratios and SLS ratios are shown.
[Fig. 3] The results of powder X-ray diffraction of a solid dispersion comprising the compound F48 are shown.
[Fig. 4] The analgesic effect of a solid dispersion comprising the compound F48 in the modified Chung model is shown. Significant difference compared to the control group (Placebo) is found (mean ± standard error (n=5 to-12); #: p<0.05, ##: p<0.01, ###: p<0.001 (30 mg/kg); *: p<0.05, **: p<0.01, ***: p<0.001 (10 mg/kg); $: p<0.05 (3 mg/kg) (two-way analysis of variance/Bonferroni test)).
[Fig. 5] The results of powder X-ray diffraction of a solid dispersion of a compound F2 (A) and a solid dispersion of a compound F57 (B) are shown.

### Embodiment of Carrying out the Invention

The present invention will be described in detail below with reference to specific embodiments. However, the present invention is not limited to the following embodiments, and can be carried out in any form without departing from the spirit of the present invention.

All patent publications, published patent applications, non-patent documents, and the like cited in the present disclosure are hereby incorporated by reference in their entirety into the present disclosure for all purposes.

In the present disclosure, when applied to a numerical value, "to" refers to a range of values that is equal to or greater than the identified standard value and is equal to or less than the identified standard value.

### (A) Present Compound

In the present specification, the present compound is a compound described in Patent Document 1, specifically a compound represented by the general formula (I) or (II).

As the compound of the present invention represented by the general formula (I), the following compounds are preferred.
(1) The compound represented by the general formula (I) where R¹ and R² may be the same or different and are a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, a phenyl group which may have a substituent, a pyridyl group which may have a substituent or an aralkyl group (with an aryl moiety having 6 to 10 carbon atoms and an alkylene moiety having 1 to 8 carbon atoms), or a pharmacologically acceptable salt thereof.
(2) The compound represented by the general formula (I) where R¹ and R² are taken together with a benzene ring to which they are bonded to form a naphthalene ring or a tetrahydronaphthalene ring, and the benzene ring or cyclohexene ring, together with R¹ and R², which is formed from carbon atoms to which R¹ and R² are respectively bonded, may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms), or a pharmacologically acceptable salt thereof.
(3) The compound represented by the general formula (I) where R¹ and R² are taken together with a benzene ring to which they are bonded to form a naphthalene ring, and the benzene ring, together with R¹ and R², which is formed from carbon atoms to which R¹ and R² are respectively bonded, may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group, or a pharmacologically acceptable salt thereof.
(4) The compound represented by the general formula (I) where R³ and R⁴ may be the same or different and are a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, or an aralkyl group (with aryl moiety having 6 to 10 carbon atoms and an alkylene moiety having 1 to 8 carbon atoms) or the compound according to any one of (1) to (3) above, or a pharmacologically acceptable salt thereof.
(5) The compound represented by the general formula (I) where R⁵ is a hydrogen atom, an alkyl group having 1 to 8 carbon atoms, or an aralkyl group (with an aryl moiety having 6 to 10 carbon atoms and an alkylene moiety having 1 to 8 carbon atoms) or the compound according to any one of (1) to (4) above, or a pharmacologically acceptable salt thereof.
(6) The compound represented by the general formula (I) where R⁵ is a hydrogen atom or the compound according to any one of (1) to (4) or a pharmacologically acceptable salt thereof.
(7) The compound represented by the general formula (I) where R⁶ and R⁷ may be the same or different and are a hydrogen atom, an alkyl group having 1 to 8 carbon atoms, an alkoxy group having 1 to 8 carbon atoms, an alkyl group having 1 to 8 carbon atoms substituted with 1 to 3 halogen atoms, or an alkoxy group having 1 to 8 carbon atoms substituted with 1 to 3 halogen atoms or the compound according to any one of (1) to (6) above, or a pharmacologically acceptable salt thereof.
(8) The compound represented by the general formula (I) where R⁶ and R⁷ are both a hydrogen atom or the compound according to any one of (1) to (6) above, or a pharmacologically acceptable salt thereof.
(9) The compound represented by the general formula (I) where X is N, Y is C(=O), and a double line consisting of a solid line and a dashed line is a single bond or the compound according to any one of (1) to (8) above, or a pharmacologically acceptable salt thereof.
(10) The compound represented by the general formula (I) where X is C, Y is N, and a double line consisting of a solid line and a dashed line is a double bond or the compound according to any one of (1) to (8) above, or a pharmacologically acceptable salt thereof.
(11) The compound represented by the general formula (I) where Z is an oxygen atom or the compound according to any one of (1) to (10) or a pharmacologically acceptable salt thereof.
(12) The compound represented by the general formula (I) where A is a phenyl group or a pyridyl group which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group or the compound according to any one of (1) to (11) above, or a pharmacologically acceptable salt thereof.
(13) The compound represented by the general formula (I) where A is a phenyl group which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, or an amino group or the compound according to any one of (1) to (11) above, or a pharmacologically acceptable salt thereof.
(14) The compound represented by the general formula (I) where A is a bond or the compound according to any one of (1) to (11) above, or a pharmacologically acceptable salt thereof.
(15) The compound represented by the general formula (I) where B is NHC(=O), NHCONH, CONH, NHC(=S)NH, NHSO₂, SO₂NH, or OSO₂ or the compound according to any one of (1) to (14) above, or a pharmacologically acceptable salt thereof.
(16) The compound represented by the general formula (I) where B is NHC(=O), NHCONH, or NHSO₂ or the compound according to any one of (1) to (14) above, or a pharmacologically acceptable salt thereof.
(17) The compound represented by the general formula (I) where D is an alkylene chain having 1 to 6 carbon atoms which may have 1 to 4 substituents that may be the same or different, which are selected from an alkyl group having 1 to 8 carbon atoms or an alkyl group having 1 to 8 carbon atoms substituted with 1 to 3 halogen atoms, and which may further have a double bond or the compound according to any one of (1) to (16) above, or a pharmacologically acceptable salt thereof.
(18) The compound represented by the general formula (I) where D is a bond or the compound according to any one of (1) to (16) above, or a pharmacologically acceptable salt thereof.
(19) The compound represented by the general formula (I) where E is a bond or the compound according to any one of (1) to (16) above, or a pharmacologically acceptable salt thereof.
(20) The compound represented by the general formula (I) where G is piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, or an alkylsulfonyl group having 1 to 6 carbon atoms or the compound according to any one of (1) to (19) above, or a pharmacologically acceptable salt thereof.
(21) The compound represented by the general formula (I) where G is benzene which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, or an alkylsulfonyl group having 1 to 6 carbon atoms or the compound according to any one of (1) to (19) above, or a pharmacologically acceptable salt thereof.
(22) The compound represented by the general formula (I) where m is 0, the compound according to any one of (1) to (21) above, or a pharmacologically acceptable salt thereof,

As the compound of the present invention represented by the general formula (II), the following compounds are preferred.
(23)
   The compound represented by the general formula (II) where 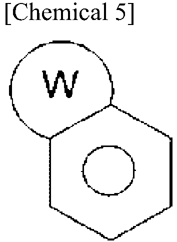 is a naphthalene ring or a tetrahydronaphthalene ring which may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms), or a pharmacologically acceptable salt thereof.
(24)
   The compound represented by the general formula (II) where 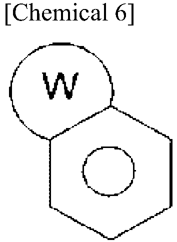 is a naphthalene ring which may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group, or a pharmacologically acceptable salt thereof.
(25)
   The compound represented by the general formula (II) where R^{3a} and R^{4a} may be the same or different and are a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, or an aralkyl group (with aryl moiety having 6 to 10 carbon atoms and an alkylene moiety having 1 to 8 carbon atoms) or the compound according to any one of (23) or (24) above, or a pharmacologically acceptable salt thereof.
(26)
   The compound represented by the general formula (II) where R^{5a} is a hydrogen atom, an alkyl group having 1 to 8 carbon atoms, or an aralkyl group (with an aryl moiety having 6 to 10 carbon atoms and an alkylene moiety having 1 to 8 carbon atoms) or the compound according to any one of (23) to (25) above, or a pharmacologically acceptable salt thereof.
(27)
   The compound represented by the general formula (II) where R^{5a} is a hydrogen atom or the compound according to any one of (23) to (25) above, or a pharmacologically acceptable salt thereof.
(28)
   The compound represented by the general formula (II) where R^{6a} and R^{7a} may be the same or different and are a hydrogen atom, an alkyl group having 1 to 8 carbon atoms, an alkoxy group having 1 to 8 carbon atoms, an alkyl group having 1 to 8 carbon atoms substituted with 1 to 3 halogen atoms, or an alkoxy group having 1 to 8 carbon atoms substituted with 1 to 3 halogen atoms or the compound according to any one of (23) to (27) above, or a pharmacologically acceptable salt thereof.
(29)
   The compound represented by the general formula (II) where R^{6a} and R^{7a} are both a hydrogen atom or the compound according to any one of (23) to (27) above, or a pharmacologically acceptable salt thereof.
(30)
   The compound represented by the general formula (II) where 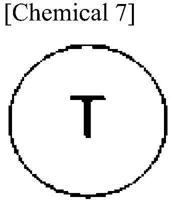 is a phenyl group or a pyridyl group which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group or the compound according to any one of(23) to (29) above, or a pharmacologically acceptable salt thereof.
(31)
   The compound represented by the general formula (II) where 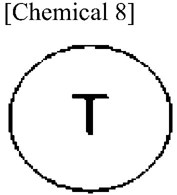 is a phenyl group which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, or an amino group or the compound according to any one of (23) to (29) above, or a pharmacologically acceptable salt thereof.
(32)
   The compound represented by the general formula (II) where 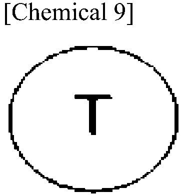 is a bond or the compound according to any one of (23) to (29) above, or a pharmacologically acceptable salt thereof.
(33)
   The compound represented by the general formula (II) where B^{a} is NHC(=O), NHCONH, CONH, NHC(=S)NH, NHSO₂, SO₂NH, or OSO₂ or the compound according to any one of (23) to (32) above, or a pharmacologically acceptable salt thereof.
(34)
   The compound represented by the general formula (II) where B^{a} is NHC(=O), NHCONH, or NHSO₂ or the compound according to any one of (23) to (32) above, or a pharmacologically acceptable salt thereof.
(35)
   The compound represented by the general formula (II) where E^{a} is a bond or the compound according to any one of (23) to (34) above, or a pharmacologically acceptable salt thereof.
(36)
   The compound represented by the general formula (II) where G^{a} is piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, or an alkylsulfonyl group having 1 to 6 carbon atoms or the compound according to any one of (23) to (35) above, or a pharmacologically acceptable salt thereof.
(37)
   The compound represented by the general formula (II) where G^{a} is benzene which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, or an alkylsulfonyl group having 1 to 6 carbon atoms or the compound according to any one of (23) to (35) above, or a pharmacologically acceptable salt thereof.
(38)
   The compound represented by the general formula (II) where n is 0 or the compound according to any one of (23) to (37) above, or a pharmacologically acceptable salt thereof.

As the compound of the present invention represented by the general formula (II), the compounds listed in the following table are preferred.

Of the compounds of the general formula (II) used in the solid dispersion of the present invention, preferred compounds include the compounds in Table 1.

**[Table 1]**

| B^{a} (substitution position) | n | E^{a} | G^{a} | Compound No. |
|---|---|---|---|---|
| NHCO(4) | 0 | Bond | Phenyl | F1 |
| NHCO(4) | 0 | Bond | (2-CF₃)Phenyl | F2 |
| NHCO(4) | 0 | Bond | (2-I) Phenyl | F48 |
| NHCO(4) | 0 | Bond | (2-F)Phenyl | F97 |
| NHCO(4) | 0 | Bond | (2-Br)Phenyl | F47 |
| NHCO(4) | 0 | Bond | (2-Cl)Phenyl | F46 |
| NHCO(4) | 0 | Bond | (2,3-Me)Phenyl | F14 |
| NHCO(4) | 0 | Bond | (2-Et)Phenyl | F57 |
| NHCO(4) | 0 | Bond | (2-iPr)Phenyl | F214 |
| NHCO(4) | 0 | Bond | (2-tBu)Phenyl | F71 |
| NHCO(4) | 0 | Bond | (6-Cl,2-OH)Phenyl | F118 |
| NHCO(4) | 0 | Bond | (2-Cl,3-OH)Phenyl | F32 |
| NHSO₂(4) | 0 | Bond | Phenyl | F146 |
| NHSO₂(4) | 0 | Bond | (3-Br)Phenyl | F147 |
| NHSO₂(4) | 1 | Bond | Phenyl | F160 |
| NHSO₂(4) | 1 | Bond | (2-Cl))Phenyl | F173 |
| NHSO₂(4) | 1 | Bond | (2-Br)Phenyl | F180 |

As the compound of the present invention represented by the general formula (I) or (II), the following compounds are particularly preferred.
(Compound F1) 5-(4-benzoylaminophenyl)-1H-naphtho [1,2-b] [1,4] diazepine-2,4 (3H,5H)-dione;
(Compound F2) 5-[4-[2-(trifluoromethyl) benzoyl] aminophenyl]-1H-naphtho [1,2-b] [1,4] diazepine-2,4 (3H,5H)-dione;
(Compound F3) 5-[4-(3-bromobenzoyl) aminophenyl]-1H-naphtho [1,2-b] [1,4] diazepine-2,4 (3H,5H)-dione;
(Compound F4) 5-[4-[4-(trifluoromethyl) benzoyl] aminophenyl]-1H-naphtho [1,2-b] [1,4] diazepine-2,4 (3H,5H)-dione;
(Compound F5) 5-[4-(2-methylbenzoyl) aminophenyl]-1H-naphtho [1,2-b] [1,4] diazepine-2,4 (3H,5H)-dione;
(Compound F6) 5-[4-(2,6-dimethylbenzoylamino) phenyl]-1H-naphtho [1,2-b] [1,4] diazepine-2,4 (3H,5H)-dione;
(Compound F7) 5-[4-(2,6-dichlorobenzoyl) aminophenyl]-1H-naphtho [1,2-b] [1,4] diazepine-2,4 (3H,5H)-dione;
(Compound F8) 5-[4-(3-chlorobenzoyl) aminophenyl]-1H-naphtho [1,2-b] [1,4] diazepine-2,4 (3H,5H)-dione;
(Compound F9) 5 [4-(2-phenylacetylamino) phenyl]-1H-naphtho [1,2-b] [1,4] diazepine-2,4 (3H,5H)-dione;
(Compound F10)1-[4-(2,4-dioxo-1,2,3,4-tetrahydronaphtho [1,2-b] [1,4] diazepin-5-yl) phenyl]-3-phenylthiourea;
(Compound F11)5-[4-(2,3-dimethoxybenzoylamino) phenyl]-1H-naphtho [1,2-b] [1,4] diazepine-2,4 (3H,5H)-dione;
(Compound F12)5-[4-(2-methoxybenzoylamino) phenyl]-1H-naphtho [1,2-b] [1,4] diazepine-2,4 (3H,5H)-dione;
(Compound F13)5-[4-[(2-chlorophenylacetyl) amino] phenyl]-1H-naphtho [1,2-b] [1,4] diazepine-2,4 (3H,5H)-dione;
(Compound F14)5-[4-(2,3-dimethylbenzoylamino) phenyl]-1H-naphtho [1,2-b] [1,4] diazepine-2,4 (3H,5H)-dione;
(Compound F15)5-[4-(2,5-dimethylbenzoylamino) phenyl]-1H-naphtho [1,2-b] [1,4] diazepine-2,4 (3H,5H)-dione;
(Compound F16)5-[4-(5-bromo-2-chlorobenzoylamino) phenyl]-1H-naphtho [1,2-b] [1,4] diazepine-2,4 (3H,5H)-dione;
(Compound F17)5-[4-(2,4-dichlorobenzoylamino) phenyl]-1H-naphtho [1,2-b] [1,4] diazepine-2,4 (3H,5H)-dione;
(Compound F18)5-[4-(2-hydroxybenzoylamino) phenyl]-1H-naphtho [1,2-b] [1,4] diazepine-2,4 (3H,5H)-dione;
(Compound F19)5-[4-(2,3-dihydroxybenzoylamino) phenyl]-1H-naphtho [1,2-b] [1,4] diazepine-2,4 (3H,5H)-dione;
(Compound F20)1-[4-(2,4-dioxo-1,2,3,4-tetrahydronaphtho [1,2-b] [1,4] diazepin-5-yl) phenyl]-3-phenylurea;
(Compound F21)5-[4-[(2,6-dichlorophenylacetyl) amino] phenyl]-1H-naphtho [1,2-b] [1,4] diazepine-2,4 (3H,5H)-dione;
(Compound F22)5-[4-[(2-methoxyphenylacetyl) amino] phenyl]-1H-naphtho [1,2-b] [1,4] diazepine-2,4 (3H,5H)-dione;
(Compound F23)5-[4-[(2-hydroxyphenylacetyl) amino] phenyl]-1H-naphtho [1,2-b] [1,4] diazepine-2,4 (3H,5H)-dione;
(Compound F24)1-(2-chlorophenyl)-3-[4-(2,4-dioxo-1,2,3,4-tetrahydronaphtho [1,2-b] [1,4] diazepin-5-yl) phenyl] thiourea;
(Compound F25)5-[4-[3-(trifluoromethyl) benzoylamino] phenyl]-1H-naphtho [1,2-b] [1,4] diazepine-2,4 (3H,5H)-dione;
(Compound F26)5-[4-[2-[2-(trifluoromethyl) phenyl] acetylamino] phenyl]-1H-naphtho [1,2-b] [1,4] diazepine-2,4 (3H,5H)-dione;
(Compound F27)1-(2-chlorophenyl)-3-[4-(2,4-dioxo-1,2,3,4-tetrahydronaphtho [1,2-b] [1,4] diazepin-5-yl) phenyl] urea;
(Compound F28)5-[4-[(2-phenylpropionyl) amino] phenyl]-1H-naphtho [1,2-b] [1,4] diazepine-2,4 (3H,5H)-dione;
(Compound F29)5-[4-(2-chloro-3-methoxybenzoylamino) phenyl]-1H-naphtho [1,2-b] [1,4] diazepine-2,4 (3H,5H)-dione;
(Compound F30)5-[4-(3-phenylpropionylamino) phenyl]-1H-naphtho [1,2-b] [1,4] diazepine-2,4 (3H,5H)-dione;
(Compound F31)5-[4-[(1H-indole-3-carbonyl) amino] phenyl]-1H-naphtho [1,2-b] [1,4] diazepine-2,4 (3H,5H)-dione;
(Compound F32)5-[4-(2-chloro-3-hydroxybenzoylamino) phenyl]-1H-naphtho [1,2-b] [1,4] diazepine-2,4 (3H,5H)-dione;
(Compound F33)5-[4-[(2-methyl-2-phenylpropionyl) amino] phenyl]-1H-naphtho [1,2-b] [1,4] diazepine-2,4 (3H,5H)-dione;
(Compound F34)5-[4-(2-phenoxyacetylamino) phenyl]-1H-naphtho [1,2-b] [1,4] diazepine-2,4 (3H,5H)-dione;
(Compound F35)5-[4-[2-(2-chloro-4-methoxyphenyl) acetylamino] phenyl]-1H-naphtho [1,2-b] [1,4] diazepine-2,4 (3H,5H)-dione;
(Compound F36)5-[4-[(1-methyl-1H-imidazole-2-carbonyl) amino] phenyl]-1H-naphtho [1,2-b] [1,4] diazepine-2,4 (3H,5H)-dione;
(Compound F37)5-[4-[2-(2,4-dichlorophenyl) acetylamino] phenyl]-1H-naphtho [1,2-b] [1,4] diazepine-2,4 (3H,5H)-dione;
(Compound F38)5-[4-[2-(2-chloro-4-hydroxyphenyl) acetylamino] phenyl]-1H-naphtho [1,2-b] [1,4] diazepine-2,4 (3H,5H)-dione;
(Compound F39)5-[4-(3-phenylpropenylamino) phenyl]-1H-naphtho [1,2-b] [1,4] diazepine-2,4 (3H,5H)-dione;
(Compound F40)5-[4-[(3-pyridylacetyl) amino] phenyl]-1H-naphtho [1,2-b] [1,4] diazepine-2,4 (3H,5H)-dione hydrochloride;
(Compound F41)5-[4-(1H-benzimidazole-2-carbonylamino) phenyl]-1H-naphtho [1,2-b] [1,4] diazepine-2,4 (3H,5H)-dione;
(Compound F42) 1-[4-(2,3-dimethylbenzoylamino) phenyl]-7-methoxy-1H-1,5-benzodiazepine-2,4 (3H,5H)-dione;
(Compound F43)5-[4-[(benzoylamino) methyl] phenyl]-1H-naphtho [1,2-b] [1,4] diazepine-2,4 (3H,5H)-dione;
(Compound F44)5-[4-[(2-chlorobenzoylamino) methyl] phenyl]-1H-naphtho [1,2-b] [1,4] diazepine-2,4 (3H,5H)-dione;
(Compound F45)1-[4-(2,3-dimethylbenzoylamino) phenyl]-7-hydroxy-1H-1,5-benzodiazepine-2,4 (3H,5H)-dione;
(Compound F46)5-[4-(2-chlorobenzoylamino) phenyl]-1H-naphtho [1,2-b] [1,4] diazepine-2,4 (3H,5H)-dione;
(Compound F47)5-[4-(2-bromobenzoylamino) phenyl]-1H-naphtho [1,2-b] [1,4] diazepine-2,4 (3H,5H)-dione;
(Compound F48)5-[4-(2-iodobenzoylamino) phenyl]-1H-naphtho [1,2-b] [1,4] diazepine-2,4 (3H,5H)-dione;
(Compound F49)5-[4-(2,3-dimethylbenzoylamino)-3-fluorophenyl]-1H-naphtho [1,2-b] [1,4] diazepine-2,4 (3H,5H)-dione;
(Compound F50)5-[4-[2-(2-methylphenyl) acetylamino] phenyl]-1H-naphtho [1,2-b] [1,4] diazepine-2,4 (3H,5H)-dione;
(Compound F51)5-[4-[(quinoxalin-2yl) carbonylamino] phenyl]-1H-naphtho [1,2-b] [1,4] diazepine-2,4 (3H,5H)-dione;
(Compound F52)5-[4-[(5-methylthiophen-2yl) carbonylamino] phenyl]-1H-naphtho [1,2-b] [1,4] diazepine-2,4 (3H,5H)-dione;
(Compound F53)5-[3-[(2-chlorophenylacetyl) amino] phenyl]-1H-naphtho [1,2-b] [1,4] diazepine-2,4 (3H,5H)-dione;
(Compound F54)5-[4-[(2,4,6-trimethylbenzoyl) amino] phenyl]-1H-naphtho [1,2-b] [1,4] diazepine-2,4 (3H,5H)-dione;
(Compound F55)5-[4-(cyclohexylcarbonylamino) phenyl]-1,2,3,4,8,9,10,11-octahydronaphtho [1,2-b] [1,4] diazepine-2, 4 (3H,5H)-dione;
(Compound F56) 1-[4-(2,3-dimethylbenzoyl) aminophenyl]-6-methyl-1H-1,5-benzodiazepine-2,4 (3H,5H)-dione;
(Compound F57)5-[4-[(2-ethylbenzoyl) amino] phenyl]-1H-naphtho [1,2-b] [1,4] diazepine-2,4 (3H,5H)-dione;
(Compound F58)5-[4-[(6-methylpyridin-2-yl) carbonylamino] phenyl]-1,2,3,4,8,9,10,11-octahydronaphtho [1,2-b ] [1,4] diazepine-2,4 (3H,5H)-dione;
(Compound F59)5-[4-[(2-methylpyridin-3-yl) carbonylamino] phenyl]-1,2,3,4,8,9,10,11-octahydronaphtho [1,2-b] [1,4] diazepine-2,4 (3H,5H)-dione;
(Compound F60)1-[4-(2,4-dioxo-1,2,3,4-tetrahydronaphtho [1,2-b] [1,4] diazepin-5-yl) phenyl]-3-(2-methylphenyl) thiourea;
(Compound F61)5-[4-(2-methoxy-3-methylbenzoyl) aminophenyl]-1,2,3,4,8,9,10,11-octahydronaphtho [1,2-b] [1,4] diazepine-2,4 (3H,5H)-dione;
(Compound F62)5-[4-(2,3-dichlorobenzoyl) aminophenyl]-1,2,3,4,8,9,10,11-octahydronaphtho [1,2-b] [1,4] diazepine-2,4 (3H,5H)-dione;
(Compound F63)5-[4-(2,3-dimethylbenzoylamino)-3-hydroxyphenyl]-1H-naphtho [1,2-b] [1,4] diazepine-2,4 (3H,5H)-dione;
(Compound F64)5-[4-(2-chloro-3-methoxybenzoylamino) phenyl]-1,3-dihydronaphtho [1,2-e]-1,4-diazepin-2-one;
(Compound F65)5-[4-[(4-dimethylaminobenzoyl) amino] phenyl]-1H-naphtho [1,2-b] [1,4] diazepine-2,4 (3H,5H)-dione;
(Compound F66)5-[4-[2-(2,4-dichlorophenoxy) acetylamino] phenyl]-1H-naphtho [1,2-b] [1,4] diazepine-2,4 (3H,5H)-dione;
(Compound F67)5-[4-[2-(2-methylphenoxy) acetylamino] phenyl]-1H-naphtho [1,2-b] [1,4] diazepine-2,4 (3H,5H)-dione;
(Compound F68)N-[4-(2,4-dioxo-1,2,3,4-tetrahydronaphtho [1,2-b] [1,4] diazepin-5-yl) butyl]-2-chloro -3-methoxybenzamide;
(Compound F69)5-[4-(2-chloro-3-hydroxybenzoylamino) phenyl]-1,3-dihydronaphtho [1,2-e]-1,4-diazepin-2-one;
(Compound F70)5-[4-(2-acetylbenzoylamino) phenyl]-1H-naphtho [1,2-b] [1,4] diazepine-2,4 (3H,5H)-dione;
(Compound F71)5-[4-(2-tert-butylbenzoylamino) phenyl]-1H-naphtho [1,2-b] [1,4] diazepine-2,4 (3H,5H)-dione;
(Compound F72)5-[2-(2-iodobenzoyl) aminoethyl]-1H-naphtho [1,2-b] [1,4] diazepine-2,4 (3H,5H)-dione;
(Compound F73)5-[3-[(2-iodobenzoyl) amino] phenyl]-1H-naphtho [1,2-b] [1,4] diazepine-2,4 (3H,5H)-dione;
(Compound F74)6,7-dimethyl-1-[4-(2-iodobenzoyl) aminophenyl]-1H-1,5-benzodiazepine-2,4 (3H,5H)-dione;
(Compound F75)5-[4-[(1-methylpiperidin-4-yl) carbonylamino] phenyl]-1H-naphtho [1,2-b] [1,4] diazepine-2,4 (3H,5H)-dione hydrochloride;
(Compound F76)5 [4-[(benzofuran-2-yl) carbonylamino] phenyl]-1H-naphtho [1,2-b] [1,4] diazepine-2,4 (3H,5H)-dione;
(Compound F77)5-[4-[(1-methyl-1H-indol-3-yl) carbonylamino] phenyl]-1H-naphtho [1,2-b] [1,4] diazepine-2,4 (3H,5H)-dione;
(Compound F78)5-[4-(2-propenylbenzoylamino) phenyl]-1H-naphtho [1,2-b] [1,4] diazepine-2,4 (3H,5H)-dione;
(Compound F79)5-[4-(2-propylbenzoylamino) phenyl]-1H-naphtho [1,2-b] [1,4] diazepine-2,4 (3H,5H)-dione;
(Compound F80)5-[3-fluoro-4-(2-iodobenzoylamino) phenyl]-1H-naphtho [1,2-b] [1,4] diazepine-2,4 (3H,5H)-dione;
(Compound F81)5-[4-(2-hydroxy-3-methylbenzoyl) aminophenyl]-1,2,3,4,8,9,10,11-octahydronaphtho [1,2-b] [1,4] diazepine-2,4 (3H,5H)-dione;
(Compound F82)5-[4-[(2-isopropoxybenzoyl) amino] phenyl]-1H-naphtho [1,2-b] [1,4] diazepine-2,4 (3H,5H)-dione;
(Compound F83)5-[4-[(3-methylthiophen-2-yl) carbonylamino] phenyl]-1H-naphtho [1,2-b] [1,4] diazepine-2,4 (3H,5H)-dione;
(Compound F84)5-[4-(2-phenoxypropionylamino) phenyl]-1H-naphtho [1,2-b] [1,4] diazepine-2,4 (3H,5H)-dione;
(Compound F85)5-[4-[2-(4-chloro-2-methylphenoxy) acetylamino] phenyl]-1H-naphtho [1,2-b] [1,4] diazepine-2,4 (3H,5H)-dione;
(Compound F86)5-[4-[(4-fluoro-2-trifluoromethyl)benzoyl ] aminophenyl]-1H-naphtho [1,2-b] [1,4] diazepine-2,4 (3H,5H)-dione;
(Compound F87)5-[4-(4-fluoro-2-methoxybenzoyl) aminophenyl]-1H-naphtho [1,2-b] [1,4] diazepine-2,4 (3H,5H)-dione;
(Compound F88)5-[4-(4-fluoro-2-hydroxybenzoyl) aminophenyl]-1H-naphtho [1,2-b] [1,4] diazepine-2,4 (3H,5H)-dione;
(Compound F89)5-[3-[(2-iodophenylacetyl) amino] phenyl]-1H-naphtho [1,2-b] [1,4] diazepine-2,4 (3H,5H)-dione;
(Compound F90)5-[4-(2-methyl-2-phenoxypropionylamino) phenyl]-1H-naphtho [1,2-b] [1,4] diazepine-2,4 (3H,5H)-dione;
(Compound F91)5-[4-(2-tert-butylbenzoylamino) phenyl]-1,3-dihydronaphtho [1,2-e]-1,4-diazepin-2-one;
(Compound F92)5-[4-[(3-dimethylaminobenzoyl) amino] phenyl]-1H-naphtho [1,2-b] [1,4] diazepine-2,4 (3H,5H)-dione;
(Compound F93)5-[4-(4-iodo-2-methoxybenzoylamino) phenyl]-1H-naphtho [1,2-b] [1,4] diazepine-2,4 (3H,5H)-dione;
(Compound F94)5-[4-(6-fluoro-2-methoxybenzoylamino) phenyl]-1H-naphtho [1,2-b] [1,4] diazepine-2,4 (3H,5H)-dione;
(Compound F95)5-[4-(2-hydroxy-4-iodobenzoylamino) phenyl]-1H-naphtho [1,2-b] [1,4] diazepine-2,4 (3H,5H)-dione;
(Compound F96)5-[4-(6-fluoro-2-hydroxybenzoylamino) phenyl]-1H-naphtho [1,2-b] [1,4] diazepine-2,4 (3H,5H)-dione;
(Compound F97)5-[4-(2-fluorobenzoyl) aminophenyl]-1H-naphtho [1,2-b] [1,4] diazepine-2,4 (3H,5H)-dione;
(Compound F98)5-[4-[(2-dimethylaminobenzoyl) amino] phenyl]-1H-naphtho [1,2-b] [1,4] diazepine-2,4 (3H,5H)-dione;
(Compound F99)5-[4-(2-methoxy-6-methylbenzoylamino) phenyl]-1H-naphtho [1,2-b] [1,4] diazepine-2,4 (3H,5H)-dione;
(Compound F100) 5-[4-(2-hydroxy-6-methylbenzoylamino) phenyl]-1H-naphtho [1,2-b] [1,4] diazepine-2,4 (3H,5H)-dione;
(Compound F101) 5-[4-[3-(2-methylphenyl) propionylamino] phenyl]-1H-naphtho [1,2-b] [1,4] diazepine-2,4 (3H,5H)-dione;
(Compound F102) 5-(4-phenylcarbamoylphenyl) -1H-naphtho [1,2-b] [1,4] diazepine-2,4 (3H,5H)-dione;
(Compound F103) 5-(4-benzylcarbamoylphenyl)-1H-naphtho [1,2-b] [1,4] diazepine-2,4 (3H,5H)-dione;
(Compound F104) 5-[4-[3-(2-methylphenyl) propenoylamino] phenyl]-1H-naphtho [1,2-b] [1,4] diazepine-2,4 (3H,5H)-dione;
(Compound F105) 5-[4-[3-(2-chlorophenyl) propionylamino] phenyl]-1H-naphtho [1,2-b] [1,4] diazepine-2,4 (3H,5H)-dione;
(Compound F106) 5-[4-(2-iodobenzoyl) aminophenyl]-1,2,3,4,8,9,10,11-octahydronaphtho [1,2-b] [1,4] diazepine-2,4 (3H,5H)-dione;
(Compound F107) 5-[4-[(1-methyl-1H-pyrrol-2-ylacetyl) amino] phenyl]-1,2,3,4,8,9,10,11-octahydronaphtho [1,2-b] [1,4] diazepine-2,4 (3H,5H)-dione;
(Compound F108) 5-[4-(2-chlorobenzyl) carbamoylphenyl]-1H-naphtho [1,2-b] [1,4] diazepine-2,4 (3H,5H)-dione;
(Compound F109) 5-[4-[3-(2-chlorophenyl) propenoylamino] phenyl]-1H-naphtho [1,2-b] [1,4] diazepine-2,4 (3H,5H)-dione;
(Compound F110) 5-[4-(2-chlorophenyl) carbamoylphenyl]-1H-naphtho [1,2-b] [1,4] diazepine-2,4 (3H,5H)-dione;
(Compound F111) 5-[4-(6-bromo-2,3-methylenedioxybenzoylamino) phenyl]-1H-naphtho [1,2-b] [1,4] diazepine-2,4 (3H,5H)-dione;
(Compound F112) 5-[4-(6-bromo-2-methoxybenzoylamino) phenyl]-1H-naphtho [1,2-b] [1,4] diazepine-2,4 (3H,5H)-dione;
(Compound F113) 5-[4-[(2-tert-butylbenzoyl) amino] phenyl]-1,2,3,4,8,9,10,11-octahydronaphtho [1,2-b] [1,4] diazepine-2,4 (3H,5H)-dione;
(Compound F114) 5-[2-(2-iodobenzoyl) aminopyridin-5-yl]-1H-naphtho [1,2-b] [1,4] diazepine-2,4 (3H,5H)-dione;
(Compound F115) 5-[4-(6-bromo-2-hydroxybenzoylamino) phenyl]-1H-naphtho [1,2-b] [1,4] diazepine-2,4 (3H,5H)-dione;
(Compound F116) 5-[4-(6-chloro-2-methoxybenzoylamino) phenyl]-1H-naphtho [1,2-b] [1,4] diazepine-2,4 (3H,5H)-dione;
(Compound F117) 5-[4-(2-iodobenzoylamino) phenyl]-1H-[1,4] diazepino [2,3-h] quinoline-2,4 (3H,5H)-dione;
(Compound F118) 5-[4-(6-chloro-2-hydroxybenzoylamino) phenyl]-1H-naphtho [1,2-b] [1,4] diazepine-2,4 (3H,5H)-dione;
(Compound F119) 5-[4-(2-hydroxy-6-methoxybenzoylamino) phenyl]-1H-naphtho [1,2-b] [1,4] diazepine-2,4 (3H,5H)-dione;
(Compound F120) 5-[4-[2-methoxy-6-(trifluoromethyl) benzoylamino] phenyl]-1H-naphtho [1,2-b] [1,4] diazepine-2,4 (3H,5H)-dione;
(Compound F121) 5-[4-[2-hydroxy-6-(trifluoromethyl) benzoylamino] phenyl]-1H-naphtho [1,2-b] [1,4] diazepine-2,4 (3H,5H)-dione;
(Compound F122) 5-[4-[(2-isopropenylbenzoyl) amino] phenyl]-1,2,3,4,8,9,10,11-octahydronaphtho [1,2-b] [1,4] diazepine-2,4 (3H,5H)-dione;
(Compound F123) 5-[4-[(2-isopropylbenzoyl) amino] phenyl]-1,2,3,4,8,9,10,11-octahydronaphtho [1,2-b] [1,4] diazepine-2,4 (3H,5H)-dione;
(Compound F124) 5-[4-[2-chloro-5-(methylthio) benzoylamino] phenyl]-1H-naphtho [1,2-b] [1,4] diazepine-2,4 (3H,5H)-dione;
(Compound F125) 5-[4-[2-(methylthio) benzoylamino] phenyl]-1H-naphtho [1,2-b] [1,4] diazepine-2,4 (3H,5H)-dione;
(Compound F126) 5-[4-[3-(methylthio) benzoylamino] phenyl]-1H-naphtho [1,2-b] [1,4] diazepine-2,4 (3H,5H)-dione;
(Compound F127) 5-[4-[2-ethyl-6-methoxybenzoylamino] phenyl]-1H-naphtho [1,2-b] [1,4] diazepine-2,4 (3H,5H)-dione;
(Compound F128) 5-[4-(3-methanesulfonylbenzoylamino) phenyl]-1H-naphtho [1,2-b] [1,4] diazepine-2,4 (3H,5H)-dione;
(Compound F129) 6-ethyl-1-[4-(2-iodobenzoyl) aminophenyl]-1H-1,5-benzodiazepine-2,4 (3H,5H)-dione;
(Compound F130) 5-[4-[2-ethyl-6-hydroxybenzoylamino] phenyl]-1H-naphtho [1,2-b] [1,4] diazepine-2,4 (3H,5H)-dione;
(Compound F131) 5-[4-(3-methanesulfinylbenzoylamino) phenyl]-1H-naphtho [1,2-b] [1,4] diazepine-2,4 (3H,5H)-dione;
(Compound F132) 5-[4-(2-chloro-5-methanesulfinylbenzoylamino) phenyl]-1H-naphtho [1,2-b] [1,4] diazepine-2,4 (3H,5H)-dione;
(Compound F133) 5-[4-(2-methanesulfinylbenzoylamino) phenyl]-1H-naphtho [1,2-b] [1,4] diazepine-2,4 (3H,5H)-dione;
(Compound F134) 5-[4-[[2-(4-morpholinyl) acetyl] amino] phenyl]-1,2,3,4,8,9,10,11-octahydronaphtho [1,2-b] [1,4] diazepine-2,4 (3H,5H)-dione hydrochloride;
(Compound F135) 5-[4-(2-chloro-6-methoxybenzoylamino) phenyl]-1,3-dihydronaphtho [1,2-e]-1,4-diazepin-2-one;
(Compound F136) 5-[4-[[(3-chloropyridin-2-yl) carbonyl] amino] phenyl]-1H-naphtho [1,2-b] [1,4] diazepine-2,4 (3H,5H)-dione;
(Compound F137) 5-[4-(2-chloro-6-hydroxybenzoylamino) phenyl]-1,3-dihydronaphtho [1,2-e]-1,4-diazepin-2-one;
(Compound F138) 5-[4-(3-chloro-2-methoxybenzoylamino) phenyl]-1H-naphtho [1,2-b] [1,4] diazepine-2,4 (3H,5H)-dione;
(Compound F139) 5-[4-[(3-methylpyridin-2-yl) carbonylamino] phenyl]-1H-naphtho [1,2-b] [1,4] diazepine-2,4 (3H,5H)-dione;
(Compound F140) 5-[4-[[(3-chloropyridin-2-yl) carbonyl] amino] phenyl]-1,2,3,4,8,9,10,11-octahydronaphtho [1,2-b] [1,4] diazepine-2,4 (3H,5H)-dione;
(Compound F141) 5-[4-(3-chloro-2-hydroxybenzoylamino) phenyl]-1H-naphtho [1,2-b] [1,4] diazepine-2,4 (3H,5H)-dione;
(Compound F142) 5-[4-[[(3-hydroxypyridin-2-yl) carbonyl] amino] phenyl]-1H-naphtho [1,2-b] [1,4] diazepine-2,4 (3H,5H)-dione;
(Compound F143) 5-[4-[(3-vinylpyridin-2-yl) carbonylamino] phenyl]-1H-naphtho [1,2-b] [1,4] diazepine-2,4 (3H,5H)-dione;
(Compound F144) 5-[4-[(3-ethylpyridin-2-yl) carbonylamino] phenyl]-1H-naphtho [1,2-b] [1,4] diazepine-2,4 (3H,5H)-dione;
(Compound F145) N-[4-(2,4-dioxo-1,2,3,4-tetrahydronaphtho-[1,2-b] [1,4]-diazepin-5-yl) phenyl]-2-nitrobenzenesulfonamide;
(Compound F146) N-[4-(2,4-dioxo-1,2,3,4-tetrahydronaphtho [1,2-b] [1,4] diazepin-5-yl) phenyl] benzenesulfonamide;
(Compound F147) 3-bromo-N-[4-(2,4-dioxo-1,2,3,4-tetrahydronaphtho [1,2-b] [1,4] diazepin-5-yl) phenyl] benzenesulfonamide;
(Compound F148) N-[4-(2,4-dioxo-1,2,3,4-tetrahydronaphtho [1,2-b] [1,4] diazepin-5-yl) phenyl]-3-methoxybenzenesulfonamide;
(Compound F149) N-[3-(2-oxo-2,3-dihydro-1H-naphtho [1,2-e] [1,4] diazepin-5-yl) phenyl] benzenesulfonamide;
(Compound F150) N-[3-(2,4-dioxo-1,2,3,4-tetrahydronaphtho-[1,2-b] [1,4]-diazepin-5-yl) phenyl]-2-nitrobenzenesulfonamide;
(Compound F151) N-[4-(2,4-dioxo-1,2,3,4,8,9,10,11-octahydro-naphtho [1,2-b] [1,4]-diazepin-5-yl) phenyl]-2-nitro-benzenesulfonamide;
(Compound F152) N-[4-(2,4-dioxo-1,2,3,4,8,9,10,11-octahydro-naphtho [1,2-b] [1,4]-diazepin-5-yl) phenyl]-N-methyl-2-nitrobenzenesulfonamide;
(Compound F153) N-[3-(2,4-dioxo-1,2,3,4-tetrahydronaphtho-[1,2-b] [1,4]-diazepin-5-yl) phenyl]-N-methyl-2-nitrobenzenesulfonamide;
(Compound F154) 4-(2,4-dioxo-1,2,3,4,8,9,10,11-octahydronaphtho [1,2-b] [1,4] diazepin-5-yl)-N-phenylbenzenesulfonamide;
(Compound F155) N-[3-(2,4-dioxo-1,2,3,4-tetrahydronaphtho [1,2-b]-[1,4] diazepin-5-yl) phenyl]-2-naphthalenesulfonamide;
(Compound F156) N-[3-(2,4-dioxo-1,2,3,4-tetrahydronaphtho [1,2-b]-[1,4] diazepin-5-yl) phenyl]-1-naphthalenesulfonamide;
(Compound F157) N-[4-(2,4-dioxo-1,2,3,4-tetrahydronaphtho [1,2-b] [1,4] diazepin-5yl) phenyl] cyclohexanesulfonamide;
(Compound F158) N-[4-(2,4-dioxo-1,2,3,4-tetrahydronaphtho [1,2-b] [1,4] diazepin-5yl) phenyl]-3-pyridinesulfonamide hydrochloride;
(Compound F159) N-[4-(2,4-dioxo-1,2,3,4-tetrahydronaphtho [1,2-b] [1,4] diazepin-5-yl) phenyl]-4-isopropylbenzenesulfonamide;
(Compound F160) N-[4-(2,4-dioxo-1,2,3,4-tetrahydronaphtho [1,2-b] [1,4] diazepin-5-yl) phenyl] phenylmethanesulfonamide;
(Compound F161) N-[4-(2,4-dioxo-1,2,3,4-tetrahydronaphtho [1,2-b] [1,4] diazepin-5yl) phenyl]-3-pyridinesulfonamide;
(Compound F162) N-[4-(2,4-dioxo-1,2,3,4-tetrahydronaphtho [1,2-b] [1,4] diazepin-5yl) phenyl]-2-naphthalenesulfonamide;
(Compound F163) 4-(2,4-dioxo-1,2,3,4,8,9,10,11-octahydronaphtho-[1,2-b] [1,4] diazepin-5-yl) phenyl 3-bromobenzene-sulfonate;
(Compound F164) N-benzyl-N-[4-(1-benzyl-2,4-dioxo-1,2,3,4-tetrahydronaphtho [1,2-b] [1,4] diazepin-5yl) phenyl]-2-nitrobenzenesulfonamide;
(Compound F165) N-benzyl-N-[4-(2,4-dioxo-1,2,3,4-tetrahydronaphtho [1,2-b] [1,4] diazepin-5yl) phenyl]-2-nitrobenzenesulfonamide;
(Compound F166) 3-bromo-N-[4-(2,4-dioxo-1,2,3,4-tetrahydronaphtho [1,2-b] [1,4] diazepin-5-yl) phenyl]-N-methylbenzenesulfonamide;
(Compound F167) N-[4-(2,4-dioxo-1,2,3,4-tetrahydronaphtho-[1,2-b] [1,4]-diazepin-5-yl) phenyl]-N-methyl-2-nitrobenzenesulfonamide;
(Compound F168) N-[4-(2,4-dioxo-1,2,3,4-tetrahydronaphtho-[1,2-b] [1,4]-diazepin-5-yl) phenyl]-N-(2-hydroxyethyl)-2-nitrobenzenesulfonamide;
(Compound F169) N-[4-(7-chloro-2,4-dioxo-2,3,4,5-tetrahydro-1H-benzo [b] [1,4] diazepin-1-yl) phenyl] benzenesulfonamide;
(Compound F170) N-[4-(7-bromo-2,4-dioxo-2,3,4,5-tetrahydro-1H-benzo [b] [1,4] diazepin-1-yl) phenyl] benzenesulfonamide;
(Compound F171) N-[4-[(2,4-dioxo-7-(trifluoromethyl)-2,3,4,5-tetrahydro-1H-benzo [b] [1,4] diazepin-1-yl)] phenyl] benzenesulfonamide;
(Compound F172) N-[4-(2,4-dioxo-2,3,4,5-tetrahydro-1H-benzo [b] [1,4] diazepin-1-yl) phenyl] benzenesulfonamide;
(Compound F173) 1-(2-chlorophenyl)-N-[4-(2,4-dioxo-1,2,3,4-tetrahydronaphtho [1,2-b] [1,4] diazepin-5-yl) phenyl] methanesulfonamide;
(Compound F174) 1-(3-bromophenyl)-N-[4-(2,4-dioxo-1,2,3,4-tetrahydronaphtho [1,2-b] [1,4] diazepin-5-yl) phenyl] methanesulfonamide;
(Compound F175) N-[4-(2,4-dioxo-1,2,3,4-tetrahydronaphtho-[1,2-b] [1,4]-diazepin-5-yl) phenyl]-2-trifluoromethylbenzenesulfonamide;
(Compound F176) N-[4-(7-bromo-6-methyl-2,4-dioxo-2,3,4,5-tetrahydro-1H-benzo [b] [1,4] diazepin-1-yl) phenyl] benzenesulfonamide;
(Compound F177) 1-(2-chlorophenyl)-N-[4-(2,4-dioxo-1,2,3,4,8,9,10,11-octahydronaphtho [1,2-b] [ 1,4] diazepin-5-yl) phenyl] methanesulfonamide;
(Compound F178) 3-bromo-N-[4-(2,4-dioxo-1,2,3,4,8,9,10,11-octahydronaphtho [1,2-b] [1,4] diazepin-5-yl) phenyl] benzenesulfonamide;
(Compound F179) N-[4-(2,4-dioxo-1,2,3,4,8,9,10,11-octahydronaphtho [1,2-b] [1,4] diazepin-5-yl) phenyl]-3-methoxybenzenesulfonamide;
(Compound F180) 1-(2-bromophenyl)-N-[4-(2,4-dioxo-1,2,3,4-tetrahydronaphtho [1,2-b] [1,4] diazepin-5-yl) phenyl] methanesulfonamide;
(Compound F181) N-[4-(2,4-dioxo-1,2,3,4-tetrahydronaphtho [1,2-b] [1,4] diazepin-5-yl) phenyl]-1-(2-methylphenyl) methanesulfonamide;
(Compound F182) N-[4-(2,4-dioxo-1,2,3,4-tetrahydronaphtho [1,2-b] [1,4] diazepin-5-yl) phenyl]-1-(2-nitrophenyl) methanesulfonamide;
(Compound F183) N-[4-(2,4-dioxo-1,2,3,4-tetrahydronaphtho [1,2-b] [1,4] diazepin-5-yl) phenyl]-2-phenylethanesulfonamide;
(Compound F184) 1-(2,3-dichlorophenyl)-N-[4-(2,4-dioxo-1,2,3,4,8,9,10,11-octahydronaphtho [1,2-b] [1,4] diazepin-5-yl) phenyl] methanesulfonamide;
(Compound F185) 1-(2-chlorophenyl)-N-[4-(2,4-dioxo-7-methoxy-1H-benzo [1,2-b] [1,4] diazepin-1-yl) phenyl] methanesulfonamide;
(Compound F186) 1-(2-chlorophenyl)-N-[4-(2,4-dioxo-7-hydroxy-1H-benzo [1,2-b] [1,4] diazepin-1-yl) phenyl] methanesulfonamide;
(Compound F187) 1-(4-chlorophenyl)-N-[4-(2,4-dioxo-1,2,3,4-tetrahydronaphtho [1,2-b] [1,4] diazepin-5-yl) phenyl] methanesulfonamide;
(Compound F188) 1-(2-chlorophenyl)-N-[4-(2,4-dioxo-1,2,3,4-tetrahydronaphtho [1,2-b] [1,4] diazepin-5-yl) benzyl] methanesulfonamide;
(Compound F189) 1-(2-chlorophenyl)-N-[4-(2,4-dioxo-1,2,3,4-tetrahydronaphtho [1,2-b] [1,4] diazepin-5-yl)-2-methoxyphenyl] methanesulfonamide;
(Compound F190) 1-(2-chlorophenyl)-N-[4-(2,4-dioxo-1,2,3,4-tetrahydronaphtho [1,2-b] [1,4] diazepin-5-yl)-2-hydroxyphenyl] methanesulfonamide;
(Compound F191) 1-(2,6-dichlorophenyl)-N-[4-(2,4-dioxo-1,2,3,4,8,9,10,11-octahydronaphtho [1,2-b] [1,4] diazepin-5-yl) phenyl] methanesulfonamide;
(Compound F192) 1-(2-chlorophenyl)-N-[4-(2,4-dioxo-6-methyl-1H-benzo [1,2-b] [1,4] diazepin-1-yl) phenyl] methanesulfonamide;
(Compound F193) 1-(2-chlorophenyl)-N-[4-(2,4-dioxy-1,2,3,4-tetrahydronaphtho [1,2-b] [1,4] diazepin-5-yl) propyl] methanesulfonamide;
(Compound F194) 1-(2-chlorophenyl)-N-[2-(2,4-dioxo-1,2,3,4-tetrahydronaphtho [1,2-b] [1,4] diazepin-5-yl) ethyl] methanesulfonamide;
(Compound F195) N-[4-(2,4-dioxo-1,2,3,4,8,9,10,11-octahydronaphtho [1,2-b] [1,4] diazepin-5-yl) phenyl]-1-(2-iodophenyl) methanesulfonamide;
(Compound F196) 1-(2-chlorophenyl)-N-[4-(2,4-dioxo-1,2,3,4,8,9,10,11-octahydronaphtho [1,2-b] [1,4] diazepin-5-yl) phenyl]-N-methylmethanesulfonamide;
(Compound F197) 1-(2-chlorophenyl)-N-[4-(2-oxo-2,3-dihydro-1H-naphtho [1,2-e] [1,4] diazepin-5-yl) phenyl] methanesulfonamide;
(Compound F198) 1-[2-(trifluoromethyl) phenyl]-N-[4-(2,4-dioxo-1,2,3,4-tetrahydronaphtho [1,2-b] [1,4] diazepin-5-yl) phenyl] phenyl-N-methylmethanesulfonamide;
(Compound F199) 1-(2-ethylphenyl)-N-[4-(2,4-dioxo-1,2,3,4-tetrahydronaphtho [1,2-b] [1,4] diazepin-5-yl) phenyl] phenyl-N-methylmethanesulfonamide;
(Compound F200) 1-(2,3-dimethylphenyl)-N-[4-(2,4-dioxo-1,2,3,4-tetrahydronaphtho [1,2-b] [1,4] diazepin-5-yl) phenyl] phenyl-N-methylmethanesulfonamide;
(Compound F201) 2-(2-chlorophenyl)-N-[4-(2,4-dioxo-1,2,3,4-tetrahydronaphtho [1,2-b] [1,4] diazepin-5-yl) phenyl] phenyl-N-methylethanesulfonamide;
(Compound F202) 1-(2-nitrophenyl)-N-[4-(2,4-dioxo-1,2,3,4-tetrahydronaphtho [1,2-b] [1,4] diazepin-5-yl) phenyl] phenyl-N-methylmethanesulfonamide;
(Compound F203) 1-(2-aminophenyl)-N-[4-(2,4-dioxo-1,2,3,4-tetrahydronaphtho [1,2-b] [1,4] diazepin-5-yl) phenyl] phenyl-N-methylmethanesulfonamide;
(Compound F204) 1-(2-dimethylaminophenyl)-N-[4-(2,4-dioxo-1,2,3,4-tetrahydronaphtho [1,2-b] [1,4] diazepin-5-yl) phenyl] phenyl-N-methylmethanesulfonamide;
(Compound F205) 5-[4-[(pyridin-4-yl) carbonylamino] phenyl]-1H-naphtho [1,2-b] [1,4] diazepine-2,4 (3H,5H)-dione hydrochloride;
(Compound F206) 5-[4-[2-[(pyridin-3-yl) oxy] acetylamino] phenyl]-1H-naphtho [1,2-b] [1,4] diazepine-2,4 (3H,5H)-dione hydrochloride;
(Compound F207) 5-[4-[(pyridin-3-yl) carbonylamino] phenyl]-1H-naphtho [1,2-b] [1,4] diazepine-2,4 (3H,5H)-dione hydrochloride;
(Compound F208) 5-[4-[(2-methylpyridin-3-yl) carbonylamino] phenyl]-1H-naphtho [1,2-b] [1,4] diazepine-2,4 (3H,5H)-dione hydrochloride;
(Compound F209) 5-[4-[(2-chloropyridin-3-yl) carbonylamino] phenyl]-1H-naphtho [1,2-b] [1,4] diazepine-2,4 (3H,5H)-dione;
(Compound F210) 5-[4-[2-[(pyridin-2-yl) oxy] acetylamino] phenyl]-1H-naphtho [1,2-b] [1,4] diazepine-2,4 (3H,5H)-dione;
(Compound F211) 5-[4-[[4-(trifluoromethyl) pyridin-3-yl] carbonylamino] phenyl]-1H-naphtho [1,2-b] [1,4] diazepine-2,4 (3H,5H)-dione;
(Compound F212) 5-[4-[(2-chloropyridin-3-yl) carbonylamino] phenyl]-1H-[1,4] diazepino [2,3-f] isoquinoline-2,4 (3H,5H)-dione;
(Compound F213) 5-[4-[(2-chloropyridin-3-yl) carbonylamino] phenyl]-8,9,10,11-tetrahydro-1H-[1,4] diazepino [2,3-f] Isoquinoline-2,4 (3H,5H)-dione; and
(Compound F214) 5-[4-[(2-isopropylbenzoyl) amino] phenyl]-1H-naphtho [1,2-b] [1,4] diazepine-2,4 (3H,5H)-dione.

Examples of a pharmacologically acceptable salt of the compound represented by the general formula (I) or (II) include hydrochlorides and salts of alkali metals, such as sodium, potassium, and lithium.

The compound of the present invention may exist in the form of optical isomers such as cis-trans isomers, optically active substances, and racemates, and all of these are included in the present invention.

The compound represented by the general formula (I) or (II) or a pharmacologically acceptable salt thereof can be produced by a known method, for example, the method described in Patent Document 1.

### (B) Solid Dispersion

As used herein, a solid dispersion refers to a dispersion of an active pharmaceutical ingredient in an inert matrix (also referred to as a "carrier"), in which the active pharmaceutical ingredient may exist in a microcrystalline or amorphous state. The matrix in a solid dispersion typically comprises a polymer. As used herein, the active pharmaceutical ingredient is a P2X4 receptor antagonist, specifically, the compound represented by the general formula (I) or (II) or a salt thereof.

Examples of polymers that can be used in a solid dispersion may include cellulose derivatives: hydroxyalkyl celluloses such as hydroxypropyl cellulose and hydroxypropyl methylcellulose (HPMC); cellulose ethers (e.g., cellulose alkyl ethers such as ethyl cellulose, ethyl methyl cellulose, ethyl propyl cellulose, isopropyl cellulose, and butyl cellulose, cellulose aralkyl ethers such as benzyl cellulose, and cellulose cyanoalkyl ethers such as cyanoethyl cellulose); cellulose esters (e.g., cellulose fatty acid esters such as cellulose acetate, cellulose propionate, and hydroxymethylcellulose acetate succinate, and cellulose aromatic carboxylic acid esters such as cellulose acetate phthalate, hydroxypropyl methylcellulose phthalate, and hydroxypropyl methylcellulose acetate succinate (HPMCAS)), as well as polysaccharides such as hyaluronic acid and pullulan. Of these polysaccharides, HPMCAS and HPMC are preferably used, with HPMC being particularly preferably used. As the HPMC, commercially available products can be used. Examples thereof include TC-5R, TC-SE, TC-5M, and PHARMACOAT P-606, manufactured by Shin-Etsu Chemical Co., Ltd., as well as METHOCEL E3 and E5, manufactured by DuPont.

Examples of polymers that can be used in a solid dispersion include (meth)acrylic polymers: homopolymers or copolymers of (meth)acrylic monomers ((meth)acrylic acid or a salt thereof (e.g., ammonium salt, amine salt, alkali metal salt, or the like); (meth)acrylic acid C₁₋₁₀ alkyl ester monomers such as methyl (meth)acrylate and ethyl (meth)acrylate; di-C₁₋₄ alkylamino C₂₋₄ alkyl (meth)acrylate such as N,N-dimethylaminoethyl (meth)acrylate or a salt thereof (e.g., quaternary ammonium salt, hydrochloride, or the like); and copolymers of (meth)acrylic monomers and copolymerizable monomers (e.g., vinyl monomers such as styrene monomers, vinyl ester monomers, heterocyclic vinyl monomers, polymerizable unsaturated dicarboxylic acids, or derivatives thereof). Specific Examples include trade name "EUDRAGIT RSPO" (ammonioalkyl methacrylate copolymer), trade name "EUDRAGIT NE30D" (ethyl acrylate - methyl methacrylate copolymer dispersion liquid), trade name "EUDRAGIT RS30D" (ammonioalkyl methacrylate copolymer dispersion liquid), trade name "EUDRAGIT RL30D" (ammonioalkyl methacrylate copolymer dispersion liquid), trade name "EUDRAGIT EPO" (aminoalkyl methacrylate copolymer E), trade name "EUDRAGIT L100-55" (dry methacrylic acid copolymer LD), trade name "EUDRAGIT S-100" (methacrylic acid copolymer S), trade name "EUDRAGIT E100" (aminoalkyl methacrylate copolymer E) manufactured by Rohm Pharma. Of these, "EUDRAGIT L100-55" and "EUDRAGIT E100" are preferably used, with "EUDRAGIT E100" being particularly preferably used.

Examples of polymers that can be used in a solid dispersion include watersoluble synthetic polymers such as polyvinylpyrrolidone (such as PVP K90), polyvinyl alcohol, copolyvidone, polyvinyl acetate phthalate, polyvinyl acetal diethylaminoacetate, polyvinyl caprolactam-polyvinyl acetate - polyethylene glycol graft copolymer (product name: Soluplus), polyvinylpyrrolidone - vinyl acetate resin (such as Kollidon (registered trademark) SR), carboxyl vinyl polymer, polyethylene glycol (e.g., PEG 6000), and polyoxyethylene/polyoxypropylene block copolymer (e.g., Poloxamer P188).

The "solid dispersion" of the present invention may comprise, in addition to the compound represented by the general formula (I) or (II) or a pharmaceutically acceptable salt thereof, and the polymer, a pharmaceutically acceptable additive as a carrier, optionally. Examples of pharmaceutically acceptable additives that may be comprised optionally include additives selected from a surfactant, a pH adjuster, a sugar, a plasticizer, and the like. These can be appropriately combined and blended in the solid dispersion of the present invention in the required amounts. In the "solid dispersion" of the present invention, the pharmaceutically acceptable additive may constitute either a dispersed phase or a continuous phase of the solid dispersion.

Examples of the surfactant that can be used include cationic surfactants such as sodium bis-(2-ethylhexyl) sulfosuccinate (docusate sodium), alkyltrimethylammonium bromide (e.g., cetyltrimethylammonium bromide (Cetrimide)), anionic surfactants such as sodium lauryl sulfate (SLS), and nonionic surfactants such as polyoxyethylene sorbitan (e.g., Tween (trademark) 20, 40, 60, 80, or 85), sorbitan fatty acid esters (e.g., Span (trademark) 20, 40, 60, 80, or 85). Of these, SLS is preferably used.

Examples of the pH adjuster that can be used include acids such as succinic acid, maleic acid, tartaric acid, citric acid, and aspartic acid, as well as alkalis such as sodium hydroxide, magnesium oxide, silicon dioxide, sodium hydrogen carbonate, and L-arginine.

Examples of the sugar that can be used include lactose, white sugar, glucose, fructose, sucrose, maltose (malt sugar), reduced maltose, maltitol, mannitol, erythritol, sorbitol, and xylitol.

Examples of the plasticizer that can be used include triethyl citrate and triacetin.

The "solid dispersion" of the present invention comprises the compound represented by the general formula (I) or (II) or a pharmaceutically acceptable salt thereof, which is an active pharmaceutical ingredient, and a carrier consisting of the polymer and the additive. The weight ratio of the carrier to the active pharmaceutical ingredient (herein referred to as the "carrier ratio") is in a range of preferably 0.5 to 100, 1 to 50, 1 to 25, 1 to 20, 1 to 15, 1 to 10, 1 to 8, 1 to 5, 2 to 50, 2 to 25, 2 to 20, 2 to 15, 2 to 10, 2 to 8, 2 to 5, 3 to 50, 3 to 25, 3 to 20, 3 to 15, 3 to 10, 3 to 8, or 3 to 5, and more preferably 1 to 25, 1 to 20, 1 to 15, 1 to 10, or 1 to 5. The carrier ratio of the "solid dispersion" of the present invention is preferably low, taking into consideration bulkiness, wettability, and production costs, provided that dissolution properties, absorbability, and stability are sufficient.

One aspect of the "solid dispersion" of the present invention comprises the compound represented by the general formula (I) or (II) or a pharmaceutically acceptable salt thereof as an active pharmaceutical ingredient and EUDRAGIT E100. The carrier ratio is in a range of preferably 0.5 to 100, 1 to 50, 1 to 25, 1 to 20, 1 to 15, 1 to 10, 1 to 8, 1 to 5, 2 to 50, 2 to 25, 2 to 20, 2 to 15, 2 to 10, 2 to 8, 2 to 5, 3 to 50, 3 to 25, 3 to 20, 3 to 15, 3 to 10, 3 to 8 or 3 to 5, and more preferably 1 to 25, 1 to 20, 1 to 15, 1 to 10 or 1 to 5. The carrier ratio of the "solid dispersion" of the present invention is preferably low, taking into consideration bulkiness, wettability, and production costs, provided that dissolution properties, absorbability, and stability are sufficient.

One aspect of the "solid dispersion" of the present invention comprises the compound represented by the general formula (I) or (II) or a pharmaceutically acceptable salt thereof as an active pharmaceutical ingredient, HPMC, and SLS. The carrier ratio is in a range of preferably 0.5 to 100, 1 to 50, 1 to 25, 1 to 20, 1 to 15, 1 to 10, 1 to 8, 1 to 5, 2 to 50, 2 to 25, 2 to 20, 2 to 15, 2 to 10, 2 to 8, 2 to 5, 3 to 50, 3 to 25, 3 to 20, 3 to 15, 3 to 10, 3 to 8 or 3 to 5, and more preferably 1 to 25, 1 to 20, 1 to 15, 1 to 10 or 1 to 5. The carrier ratio of the "solid dispersion" of the present invention is preferably low, taking into consideration bulkiness, wettability, and production costs, provided that dissolution properties, absorbability, and stability are sufficient.

One aspect of the "solid dispersion" of the present invention comprises the compound represented by the general formula (I) or (II) or a pharmaceutically acceptable salt thereof as an active pharmaceutical ingredient, HPMC, and SLS. The weight ratio of SLS to the active pharmaceutical ingredient (herein referred to as the "SLS ratio") is in a range of preferably 0.05 to 50, 0.1 to 50, 0.1 to 25, 0.1 to 20, 0.1 to 15, 0.1 to 10, 0.1 to 8, 0.1 to 5, 0.1 to 4, 0.2 to 50, 0.2 to 25, 0.2 to 20, 0.2 to 15, 0.2 to 10, 0.2 to 8, 0.2 to 5, 0.2 to 4, 0.3 to 50, 0.3 to 25, 0.3 to 20, 0.3 to 15, 0.3 to 10, 0.3 to 8, 0.3 to 5, 0.3 to 4, 0.5 to 50, 0.5 to 25, 0.5 to 20, 0.5 to 15, 0.5 to 10, 0.5 to 8, 0.5 to 5 or 0.5 to 4, and more preferably 0.1 to 10, 0.1 to 8, 0.1 to 5, 0.1 to 4, 0.5 to 10, 0.5 to 8, 0.5 to 5 or 0.5 to 4.

In the "solid dispersion" of the present invention, the compound represented by the general formula (I) or (II) or a pharmaceutically acceptable salt thereof is preferably present in part or in whole as an amorphous substance. Here, an amorphous substance means a substance in a state in which there is short-range order between atoms or molecules of the compound represented by the general formula (I) or (II) or a pharmaceutically acceptable salt thereof. Still, there is no long-range order as in a crystal. In the present invention, an amorphous substance can be identified by the presence of a halo pattern in powder X-ray diffraction.

In the present invention, the compound represented by the general formula (I) or (II) or a pharmaceutically acceptable salt thereof exists as an amorphous substance at a content of preferably 50% by weight or more, more preferably 80% by weight or more, still more preferably 90% by weight or more, even more preferably 95% by weight or more, even more preferably 98% by weight or more, which may be 100% by weight, based on the total weight thereof. The content of the amorphous substance can be determined by X-ray diffraction.

The solid dispersion of the present invention can be produced using a method known per se, such as a mixed grinding method (mechanochemical method), a solvent method, or a melting method.

Here, the mixed grinding method can be carried out by mixing the compound represented by the general formula (I) or (II) or a pharmaceutically acceptable salt thereof with a carrier, and then using a mixer and grinder such as a ball mill or a hammer mill in a conventional manner.

The solvent method refers to a method in which the compound represented by the general formula (I) or (II) or a pharmaceutically acceptable salt thereof and a carrier are dissolved or suspended in a solvent (an organic solvent, water, or a mixture thereof), and then, the solvent is removed to precipitate a solid dispersion, or a solid dispersion is precipitated in the solvent.

The solvent can be removed by a method such as a spray method (which can be classified into a fluidized bed method, a spray drying method (also called a spray-drying method), a rolling bed method, a stirring method, or a supercritical method, depending on the embodiment), a filtration method, an evaporation method, or a freeze-drying method, with a spray method being preferred, and a spray-drying method being particularly preferred.

The solvent that can be used in producing the solid dispersion of the present invention is preferably a pharmaceutically acceptable solvent. Examples thereof include ethanol, methanol, 2-propanol, n-propanol, butanol, acetone, 2-butanone, methyl ethyl ketone, methyl isobutyl ketone, tetrahydrofuran (THF), tetrahydropyran, 1,4-dioxane, diethyl ether, toluene, acetonitrile, methylene chloride, chloroform, 1,1,1-trichloroethane, carbon tetrachloride, methyl acetate, ethyl acetate, butyl acetate, acetic acid, formic acid, N,N-dimethylformamide, N,N-dimethylacetamide or dimethylsulfoxide, any combination of these solvents, and the combination with water. These solvents that can be used may be the same or different for the compound represented by the general formula (I) or (II) or a pharmaceutically acceptable salt thereof and the carrier components. Suitable solvents are ethanol, methanol, acetone, THF, and a mixture of ethyl acetate and water, and mixtures thereof.

In the spray drying method, a solid dispersion can be produced by a method known per se. For example, a compound represented by the general formula (I) or (II) or a pharmaceutically acceptable salt thereof, and carrier components are added to the solvent to prepare a solution or suspension. The solution or suspension is converted into a fine mist by centrifugal spraying using a rotating disk or by pressurized spraying using a pressure nozzle, which is then sprayed into a drying medium (heated air or nitrogen gas) to produce a powdery dried product, thereby obtaining a solid dispersion. Each component constituting the solid dispersion is preferably dissolved in the solvent used.

In the spray drying method, the temperature of the drying medium is, for example, 50°C to 120°C, and preferably 50°C to 90°C. The drying medium may be flowed in a constant direction, for example, at a flow rate (drying air) of 0.2 to 0.6 m³/min, and preferably 0.3 to 0.5 m³/min.

The melting method refers to a method in which the compound represented by the general formula (I) or (II) or a pharmaceutically acceptable salt thereof and the carrier components are dissolved or dispersed by heating to a temperature equal to or higher than their melting points or softening points and stirring or the like, and then rapidly cooled. In this case, additives such as a plasticizer (e.g., triethyl citrate, polyethylene glycol, or triacetin) and a surfactant can be further added, optionally. The production can be carried out using an agitation granulator equipped with a heating device.

The solid dispersion of the present invention produced by the above-described production method can be made into solid dispersion particles having any particle size by a known method, and the solid dispersion particles can be used as they are as a powder or granules.

The pharmaceutical composition comprising the solid dispersion of the present invention comprises the solid dispersion and additional pharmaceutically acceptable additives. The pharmaceutical composition of the present invention can be produced by appropriately combining and blending the required amounts of, for example, a binder, a disintegrant, an excipient, a lubricant, and the like, as the additional additives.

Examples of the binder that can be used in the pharmaceutical composition of the present invention include methylcellulose, hydroxyethylcellulose, hydroxypropylcellulose, hypromellose, polyvinylpyrrolidone, gelatin, agar, alginic acid, sodium alginate, partially saponified polyvinyl alcohol, pullulan, partially pregelatinized starch, dextrin, xanthan gum, and arabic gum powder. Hydroxypropyl cellulose, hypromellose, and polyvinylpyrrolidone are preferred. These may be used singly or in combination of two or more kinds.

Examples of the disintegrant that can be used in the pharmaceutical composition of the present invention include crystalline cellulose, carboxymethylcellulose (carmellose), croscarmellose sodium, carboxymethylcellulose calcium, low-substituted hydroxypropylcellulose, crospovidone, hydroxypropyl starch, starch, partially pregelatinized starch, and sodium starch glycolate, with croscarmellose sodium, sodium starch glycolate, crospovidone being preferred, and crospovidone being more preferred. The blended amount of the disintegrant used is preferably 5% to 30% by weight, more preferably 5% to 15% by weight, based on the total weight of the pharmaceutical composition. When the disintegrant is blended into tablets, the blended amount is preferably 1% to 10% by weight and more preferably 2% to 6% by weight in granules for tableting.

The excipient that can be used in the pharmaceutical composition of the present invention can be blended into a kneaded product, a granulated product, or a powder. Examples thereof include: celluloses such as crystalline cellulose, ethyl cellulose, hydroxypropyl cellulose, low-substituted hydroxypropyl cellulose, and hydroxypropyl methylcellulose (such as hypromellose); starches such as corn starch, potato starch, wheat starch, rice starch, partially pregelatinized starch, and hydroxypropyl starch; sugars such as glucose, lactose, white sugar, refined white sugar, powdered sugar, trehalose, dextran, and dextrin; sugar alcohols such as D-mannitol, xylitol, sorbitol, and erythritol; and inorganic salts such as glycerin fatty acid esters, magnesium aluminometasilicate, synthetic hydrotalcite, anhydrous calcium phosphate, precipitated calcium carbonate, calcium silicate, calcium hydrogen phosphate hydrate, and sodium hydrogen carbonate, with crystalline cellulose being preferred.

Examples of the lubricant that can be used in the pharmaceutical composition of the present invention include stearic acid, sodium stearyl fumarate, magnesium stearate, calcium stearate, sucrose fatty acid ester, polyethylene glycol, light anhydrous silicic acid, hydrogenated oil, glycerin fatty acid ester, and talc. Sodium stearyl fumarate, magnesium stearate, calcium stearate, and sucrose fatty acid ester are preferred. These may be used singly or in combination of two or more kinds.

The pharmaceutical composition of the present invention can be provided in various forms, such as solid preparations (e.g., tablets, capsules, granules, powders, and lozenges) or liquid preparations (e.g., injections), by subjecting the composition to a formulation step known per se. Such an injection may be in a form in which the pharmaceutical composition of the present invention is provided as a solid preparation and is prepared into an injection immediately before use.

Since the present invention also has an effect of suppressing tableting troubles, it is particularly preferable for the solid preparation to be in the form of tablets. In addition, such solid preparations may be coated, optionally.

The content of the solid dispersion in the pharmaceutical composition of the present invention may be 10% to 95% by weight, preferably 30% to 90% by weight, and more preferably 60% to 85% by weight, based on the total weight of the pharmaceutical composition.

The solid dispersion of the present invention, or a pharmaceutical composition comprising the solid dispersion, can be administered orally or parenterally to humans or other mammals and is beneficial as a therapeutic agent for neuropathic pain, multiple sclerosis, cough suppression, irritable bowel syndrome, inflammatory bowel disease, various allergies, and the like. As used herein, "treatment" may encompass the concept of prevention or prophylactic use, in addition to treatment.

One aspect of the present invention is a method for treating neuropathic pain, multiple sclerosis, cough suppression, irritable bowel syndrome, inflammatory bowel disease, various allergies, or the like, comprising administering a therapeutically effective amount of the solid dispersion of the present invention or a pharmaceutical composition comprising the solid dispersion to a subject in need of treatment for neuropathic pain, multiple sclerosis, cough suppression, irritable bowel syndrome, inflammatory bowel disease, various allergies, and the like.

Another aspect of the present invention is the solid dispersion of the present invention or a pharmaceutical composition comprising the solid dispersion for use in the treatment of neuropathic pain, multiple sclerosis, cough suppression, irritable bowel syndrome, inflammatory bowel disease, various allergies, and the like.

Yet another aspect of the present invention is the use of the solid dispersion of the present invention for producing a therapeutic agent for treating neuropathic pain, multiple sclerosis, cough suppression, irritable bowel syndrome, inflammatory bowel disease, various allergies, and the like.

The dosage of the solid dispersion of the present invention or a pharmaceutical composition comprising the solid dispersion can be adjusted depending on the content of the compound represented by the general formula (I) or (II) or a pharmaceutically acceptable salt thereof in the solid dispersion of the present invention or a pharmaceutical composition comprising the solid dispersion. The dosage is appropriately determined based on the method of administration, age, weight, sex, symptoms, drug sensitivity, and other relevant conditions of the subject being administered. The dosage may be adjusted according to the degree of symptom improvement.

The dosage of the solid dispersion of the present invention or a pharmaceutical composition comprising the solid dispersion can be, for example, in an adult, usually administered in terms of the content of the compound represented by the general formula (I) or (II) or a pharmaceutically acceptable salt thereof at about 0.1 mg to 1,000 mg per day for injection or about 1 mg to 20,000 mg per day for oral administration, but can be increased or decreased depending on age, symptoms, or the like. The number of doses may be, for example, 1 to 3 times per day, preferably 1 to 2 times per day.

### Examples

The present invention is more specifically explained with reference to the Examples, Comparative Examples, and Test Examples below; however, the present invention is not limited to the Examples.

### Example 1. Synthesis of Compounds

Naphthodiazepinedione derivatives, a compound F2 (5-[4-[2-(trifluoromethyl)benzoyl]aminophenyl]-1H-naphtho[1,2-b][1,4]diazepine-2,4(3H,5H)-dione), a compound F48 (5-[4-(2-iodobenzoylamino)phenyl]-1H-naphtho[1,2-b][1,4]diazepine-2,4(3H,5H)-dione), and a compound F57 (5-[4-[(2-ethylbenzoyl)amino]phenyl]-1H-naphtho[1,2-b][1,4]diazepine-2,4(3H,5H)-dione), were synthesized by the method described in Patent Document 1. These compounds were poorly soluble in water.

### Example 2. Solid Dispersion (1): Examination of Carriers

### 2-1) Preparation of Solid Dispersion

Solid dispersions of the compound F48 were prepared using the following 10 types of carriers containing polymers (referred to as solid dispersions A to J, respectively, depending on the carrier used). To 125 mg of each carrier, 5 mL of a mixture of methanol/dichloromethane (both from Wako Pure Chemical Industries, Ltd.) (volume ratio 3:10) was added to dissolve it. Then, 250 mg of the compound F48 was dissolved in 100 mL of tetrahydrofuran (Wako Pure Chemical Industries, Ltd.), and 2 mL of the solution was placed in a 10 mm × 10 mm test tube (AS ONE CORPORATION), and 5 mL of the polymer solution was added thereto. The carrier ratio (weight ratio between the compound F48 and the carrier) of each solid dispersion was 25, and the SLS ratio (weight ratio between the compound F48 and SLS) of solid dispersion B was 8.3. The resulting solution was mixed in a vortex mixer until homogenous, and then dispensed into 4 vials (each containing 1.25 mg of the compound F48). The organic solvent was removed by spraying a nitrogen stream onto these samples, and the samples were then dried overnight under reduced pressure (SATO VAC INC., oil rotary vacuum pump), thereby preparing solid dispersions. As a control, a sample to which no carrier was added was also prepared.

### Various carriers used in solid dispersions:

A: Hydroxypropyl methylcellulose (HPMC) (Shin-Etsu Chemical Co., Ltd.; product type: TC-5R)
B: HPMC/sodium lauryl sulfate (SLS) (Tokyo Chemical Industry Co., Ltd. (TCI)) (weight ratio 2:1)
C: Hydroxypropyl methylcellulose acetate succinate (HPMCAS) (Shin-Etsu Chemical Co., Ltd., MF)
D: EUDRAGIT S100 (Evonik)
E: EUDRAGIT E100 (Evonik)
F: EUDRAGIT L100-55 (Evonik)
G: Polyvinylpyrrolidone (PVP) (BASF)
H: PEG6000 (Wako Pure Chemical Industries, Ltd.)
I: Crospovidone (BASF)
J: Poloxamer P188 (BASF)

### 2-2) Dissolution Test

To each of the prepared solid dispersion samples, 5 mL of the 1st fluid for dissolution test of the Japanese Pharmacopoeia (pH 1.2) was added to prepare two bottles, and 5 mL of the 2nd fluid for dissolution test of the Japanese Pharmacopoeia (pH 6.8) was added to prepare two bottles. The solid dispersion was crushed with a glass rod or spatula and then shaken at 37°C for 2 hours (EYELA, INCUBATOR FMS). Each sample solution was filtered through a 0.22 µm filter. To 200 µL of the resulting solution,200 µL of acetonitrile (Wako Pure Chemical Industries, Ltd.) was immediately added. As a control, a similar dissolution test was also carried out on a sample to which no carrier was added. The concentration of the compound F48 in the dissolved solution was quantified by HPLC under the following conditions.

### Quantification conditions

Equipment: LC-2010CHT (manufactured by Shimadzu Corporation)
Column: YMC-Pack ODS-A; particle size: 5 µm; inner diameter: 4.6 mm; length: 15 cm (YMC.CO., LTD.)
Detector: Ultraviolet absorptiometer (measurement wavelength: 250 nm)
Column temperature: 40°C
Mobile phase: 0.1% formic acid/acetonitrile mixture (1:1)

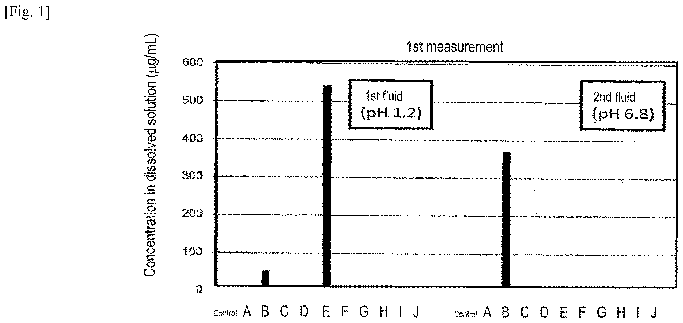

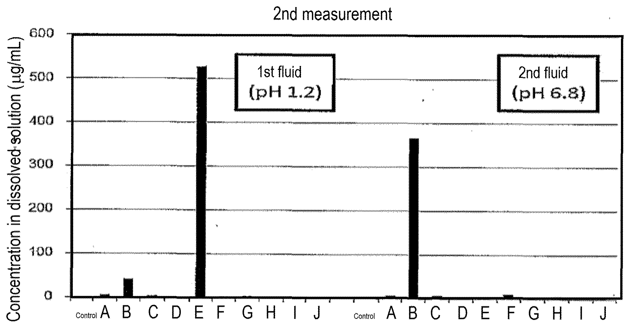

The test, from the preparation of various solid dispersions to the measurement of the concentration of the compound F48 in the dissolved solution, was repeated twice, and the results are shown in Fig. 1. This test system had favorable reproducibility. In the 1st fluid for dissolution test of the Japanese Pharmacopoeia, whose condition was acidic conditions, the dissolution of solid dispersion E using EUDRAGIT E100, a basic polymer, as a carrier was significantly high. Meanwhile, although in the solid dispersion A, using only HPMC as a carrier, the dissolution of the compound F48 was not significantly affected, in the solid dispersion B, using a combination of HPMC and SLS as a carrier, the dissolution of the compound F48 was significantly improved in both 1st and 2nd fluids for dissolution test of the Japanese Pharmacopoeia. Also in a case in which a solid dispersion was prepared by the same procedure as above, except that only SLS was used as a carrier, compared with solid dispersion B using a combination of HPMC and SLS as a carrier, the dissolution of the compound F48 was not significantly affected (when the weight ratios of SLS to compound F48 were 5, 2, and 1, the dissolution concentrations of the compound F48 were 31.4 µg/mL, 15.0 µg/mL, and 8.5 µg/mL, respectively). In both solid dispersions E and B, the concentration of the compound F48 in the dissolved solution reached a maximum value 10 to 20 minutes after the start of dissolution, and this state continued for at least 2 hours.

### 2-3) PK Test

Solid dispersions E and B were prepared using a rotary evaporator according to the following method. The compound F48 was dissolved in THF to result in 2.5 mg/mL. By the same procedure as above, each polymer was prepared to result in 25 mg/mL. Next, the compound F48 solution was added to the polymer solution with stirring such that the weight ratio between the compound F48 and the carrier was 1:25. The resulting mixture was transferred to a recovery flask, and the organic solvent was then removed using a rotary evaporator (Yamato Scientific Co., Ltd., distillation apparatus). The recovery flask was transferred to a desiccator and dried under reduced pressure using a vacuum pump for approximately 16 hours, thereby obtaining solid dispersions E and B.

Next, the solid dispersion E dispersed in a 1% carboxymethylcellulose aqueous solution or the solid dispersion B dissolved in water was, respectively, orally administered once at a dose of 10 mg/kg (in terms of compound F48) to fasted male rats (8 weeks old, Wistar (Crlj:WI), Charles River Laboratories Japan, Inc.) (n=3 per group). As a control, the crystalline form of the compound F48 was crushed in an agate mortar, suspended in a 1% carboxymethylcellulose aqueous solution, and administered in the same manner.

Blood samples were collected in a time-dependent manner before oral administration and at 0.5, 1, 3, 5, 8, and 24 hours after oral administration. The concentration of the compound F48 in the obtained plasma was measured by HPLC (Hitachi High-Technologies Corporation). The time to reach the maximum plasma concentration (Tₘₐₓ), the maximum plasma concentration (Cₘₐₓ), and the area under the plasma concentration-time curve (AUCₗₐₛₜ) were calculated from the plasma concentration transition (Table 2). Compared to the control, the solid dispersions E and B showed a significant improvement in absorption, as indicated by both Cₘₐₓ and AUCₗₐₛₜ. Furthermore, unlike the results of the in vitro dissolution test, the solid dispersion B had superior pharmacokinetics in plasma compared to the solid dispersion E.

From the above, it was demonstrated that the *in vivo* absorbability was improved by preparing a solid dispersion of the present compound using EUDRAGIT E100 or a combination of HPMC and SLS as a carrier.

**[Table 2]**

| Dosing Group | Dosage* (mg/kg) | Cₘₐₓ (ng/mL) | Tₘₐₓ (hr) | AUCₗₐₛₜ (ng · hr/mL) |
|---|---|---|---|---|
| Solid Dispersion E | 10 | 995 ± 293 | 7.0 ± 1.7 | 9167 ± 3812 |
| Solid Dispersion B | 10 | 2773 ± 203 | 6.0 ± 1.7 | 39087 ± 4383 |
| Control | 10 | <25 | Not Applicable | Not Applicable |

| | | | | |
|---|---|---|---|---|
| *: indicates the dosage of the compound F48. | | | | |

### Example 3. Solid Dispersion (2): Examination of Mixing Ratio Through Dissolution Test

In the same manner as in Example 2, various solid dispersions were prepared by changing the mixing ratio based on the solid dispersion E or B (Table 3: solid dispersions E1 to E3, B1 to B5).

**[Table 3]**

| Solid Dispersion | (A) Compound F48 | (B) Carrier (Total) | Carrier Ratio (B/A) | (C) SLS in Carrier | SLS Ratio (C/A) |
|---|---|---|---|---|---|
| E | 5 mg | 125 mg | 25 | - | - |
| E1 | 5 mg | 25 mg | 5 | - | - |
| E2 | 5 mg | 10 mg | 2 | - | - |
| E3 | 5 mg | 5 mg | 1 | - | - |
| B | 5 mg | 125 mg | 25 | 41.7 mg | 8.3 |
| B1 | 5 mg | 25 mg | 5 | 8.3 mg | 1.7 |
| B2 | 5 mg | 10 mg | 2 | 3.3 mg | 0.7 |
| B3 | 5 mg | 5 mg | 1 | 1.7 mg | 0.3 |
| B4 | 5 mg | 25 mg | 5 | 16.7 mg | 3.3 |
| B5 | 5 mg | 25 mg | 5 | 5.0 mg | 1.0 |

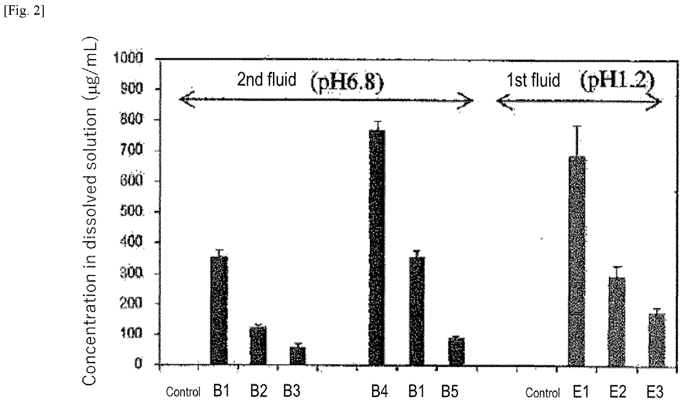

The resulting solid dispersions were subjected to a dissolution test by the method described above. When the carrier ratio was changed from 25 to 1 using the solid dispersion E as a base, the dissolution rate in the 1st fluid for dissolution test of the Japanese Pharmacopoeia (pH 1.2) peaked at a carrier ratio of 5, and then decreased (Figs. 1 and 2: solid dispersions E, E1 to E3). Meanwhile, when the carrier ratio was changed from 25 to 1 using the solid dispersion B as a base, the dissolution rate in the 2nd fluid for dissolution test of the Japanese Pharmacopoeia (pH 6.8) peaked at a carrier ratio of 5, and then decreased (Figs. 1 and 2: solid dispersions B, B1 to B3). In addition, when the SLS ratio, which is the weight ratio of SLS contained in the carrier to the compound F48, was changed from 8.3 to 0.3, the dissolution rate in the 2nd fluid for dissolution test of the Japanese Pharmacopoeia (pH 6.8) peaked at an SLS ratio of 3.3, and then decreased (Figs. 1 and 2: solid dispersions B, B1 to B5).

### Example 4. Solid Dispersion (3): Examination of Mixing Ratio Through PK Test

### 4-1) Preparation of Solid Dispersion

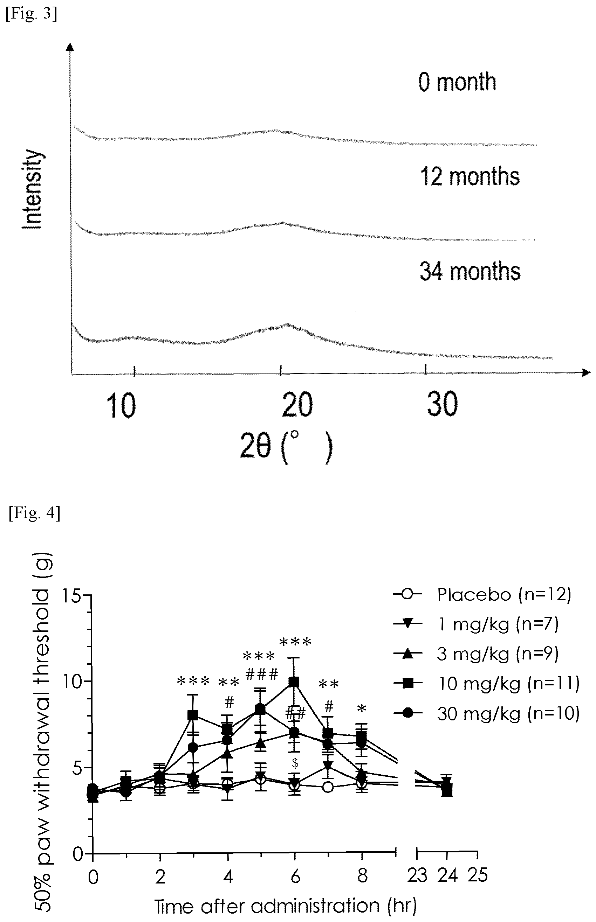

Next, the effects of the carrier ratio and SLS ratio on *in vivo* absorption were confirmed by a PK test. Various solid dispersions were prepared by the spray drying method as follows. 400 mg of the compound F48 was dissolved in 160 mL of THF (containing a stabilizer, KANTO CHEMICAL CO., INC.). Also, 1.6 g of HPMC and 400 mg of SLS (KOKUSAN CHEMICAL Co., Ltd.) were dissolved in an 80% (v/v) ethanol aqueous solution (100 mL). Both solutions were mixed by stirring for 1 hour, thereby preparing a spray solution with a weight ratio of the compound F48/HPMC/SLS of 1:4:1 (carrier ratio of 5; SLS ratio of 1). The spray solution was pumped to a spray dryer (Yamato, Palvis Minibed GB22) using a peristaltic pump. Spray drying was performed from a spray nozzle (SPRAYING SYSTEMS CO., JAPAN; 2050 SS; nozzle inner diameter: 508 µm) under conditions of an inlet temperature of approximately 85°C, an outlet temperature of approximately 60°C, an air volume (drying air) of approximately 0.3 m³/min, and a constant spray pressure. The obtained primary dried product was further subjected to secondary drying (at 40°C for approximately 1 day) in a vacuum dryer, thereby obtaining 1.63 g of solid dispersion B5 as a white powder. Similarly, solid dispersions B1 and B5 to B8, shown in Table 4, were prepared. The solid dispersion B4 prepared by the method using a rotary evaporator described above was used. The prepared solid dispersions were subjected to powder X-ray diffraction analysis under the following measurement conditions. The powder X-ray pattern of the solid dispersion B5 showed a halo pattern as shown in Fig. 3, revealing that the compound F48 was in an amorphous state.
Measurement conditions of powder X-ray diffraction
Equipment: D2Phaser (BRUKER)
Anticathode: Cu
Measurement angle: 2θ = 5° to 40°
Scanning mode: PSD high speed scan
Counting time: 0.3 sec
Step width: 0.02°
Divergence slit: 1.0 mm
Air scatter sink: 3.0 mm

### 4-2) PK Test

Each solid dispersion dissolved or suspended in water was orally administered once at a dose of 100 mg/kg (as the compound F48) to male rats (5 weeks old, CD(SD) (Crl:CD(SD)), Charles River Laboratories Japan, Inc.) under non-fasting conditions (n=3 per group). Blood samples were collected in a time-dependent manner at 0.5, 1, 3, 5, 8, and 24 hours after oral administration. The concentration of the compound F48 in the obtained plasma was measured by HPLC. Tₘₐₓ, Cₘₐₓ, and AUCₗₐₛₜ were calculated from the plasma concentration transition (Table 4: n=3 for each group, mean value ± standard deviation). When the carrier ratio of the solid dispersion was decreased from 5 to 3, the blood kinetics (Cₘₐₓ and AUCₗₐₛₜ) of the compound F48 tended to decline (see the values for the solid dispersions B6 and B5 with an SLS ratio of 1; and the solid dispersions B7 and B8 with an SLS ratio of 0.3). Furthermore, when the SLS ratio was decreased from 3.3 to 0.3, the blood kinetics (Cₘₐₓ and AUCₗₐₛₜ) of the compound F48 tended to decline (see the values for the solid dispersions B4, B1, B5, and B8 with a carrier ratio of 5; and the solid dispersions B6 and B7 with a carrier ratio of 3). When a solid dispersion (weight ratio of compound F48/HPMC/Tween 80: 1:3.3:1.7) using Tween 80 (KANTO CHEMICAL CO., INC.) instead of SLS was prepared using the spray dryer used above, and a PK test was performed, the absorbability was not as sufficient as that of SLS (solid dispersion B1) (Cₘₐₓ: 8,783 ± 572 ng/mL; Tₘₐₓ: 3.7 ± 1.2 hr; AUCₗₐₛₜ: 123,463 ± 6,401 ng·hr/mL).

**[Table 4]**

| Solid Dispersion | Carrier Ratio | SLS Ratio | Cₘₐₓ (ng/mL) | Tₘₐₓ (hr) | AUCₗₐₛₜ (ng· hr/mL) |
|---|---|---|---|---|---|
| B4 | 5 | 3.3 | 21,036 ± 2,633 | 8.0 ± 0.0 | 297,206 ± 23,338 |
| B1 | 5 | 1.7 | 20,987 ± 4,073 | 8.0 ± 0.0 | 290,977 ± 44,364 |
| B6 | 3 | 1 | 17,592 ± 2,854 | 6.0 ± 1.7 | 255,677 ± 49,730 |
| B5 | 5 | 1 | 18,604 ± 1,947 | 6.0 ± 1.7 | 271,801 ± 28,880 |
| B7 | 3 | 0.6 | 17,367 ± 971 | 5.0 ± 0.0 | 239,465 ± 11,684 |
| B8 | 5 | 0.6 | 14,965 ± 1,068 | 4.3 ± 1.2 | 221,250 ± 13,578 |
| B9 | 3 | 0.3 | 14,837 ± 585 | 5.0 ± 0.0 | 198,890 ± 1,893 |
| B10 | 5 | 0.3 | 17,273 ± 1,526 | 5.0 ± 0.0 | 236,585 ± 14,927 |

### Example 5. Solid Dispersion (4): Production by Spray Drying Method 5-1) Preparation of Spray Liquid

50 g of the compound F48 was dissolved in 17.98 kg of THF (containing a stabilizer, KANTO CHEMICAL CO., INC.). In addition, 200 g of HPMC (Shin-Etsu Chemical Co., Ltd.) and 50 g of SLS (KOKUSAN CHEMICAL Co., Ltd.) were successively added to a mixed solvent of 7.89 kg of ethanol (KANTO CHEMICAL CO., INC.) and 2.5 kg of ion-exchanged water under stirring. The mixture was then sieved (using 300- and 500-mesh sieves). The two liquids were mixed and stirred to prepare a spray liquid with a weight ratio of compound F48/HPMC/SLS of 1:4:1.

### 5-2) Spray Drying and Secondary Drying

The prepared spray liquid was pumped into a spray dryer (CL-8i, OHKAWARA KAKOHKI CO., LTD.) via a peristaltic pump. Spray drying was performed under the following conditions: raw liquid processing rate was 4 kg/h, the spray gas pressure of the two-fluid nozzle was 0.2 MPa, the inlet temperature was 120°C, and the outlet temperature was 70°C. The obtained primary dried product (270.18 g) was further subjected to secondary drying (40°C/60 hours) in a vacuum dryer, thereby obtaining 237.4 g of a solid dispersion as a white powder (recovery rate 79.1%). The powder X-ray diffraction pattern of the solid dispersion B5 obtained by the above production showed a halo pattern similar to the powder X-ray diffraction pattern of the solid dispersion B5 prepared in Example 4.

### Example 6. Stability of Solid Dispersion

After storing the solid dispersion B5 produced separately by a spray-drying method under the conditions of room temperature, light shielding, and sealing, the powder X-ray diffraction was measured in the same manner as in Example 4. As shown in Fig 3, there was no change in the powder X-ray diffraction pattern measured at the start of storage (0 month) and the powder X-ray diffraction pattern (halo pattern) measured about 34 months after the start of storage, which revealed that the compound F48 was stable in an amorphous state even when stored for an extended period. In addition, the physical stability was examined when the solid dispersion was stored under light-shielded, open, heated, and humidified conditions (storage at 60 °C and 85% humidity for 1 week). As a result, even after storage, no peaks at diffraction angles characteristic of the crystalline form of the compound F48 were observed in powder X-ray diffraction. Furthermore, the glass transition temperature of the solid dispersion B5 was measured (TA Instruments, Q2000) according to the manual for the equipment and was found to be 100°C or higher, suggesting that it was stable as an amorphous solid dispersion.

### Example 7. Analgesic Effect of Solid Dispersion

The analgesic effect of the solid dispersion was examined using a modified Chung rat model (Patent Document 1), which is a neuropathic pain model. On days 8 and 9 after preparation of the modified Chung model, non-fasted rats (Charles River Laboratories Japan, Inc., Wistar, male, 7 to 8 weeks old) with a sufficiently decreased pain threshold (paw withdrawal threshold ≤ 5 g) were orally administered 1, 3, 10, or 30 mg/kg of the compound F48 by suspending solid dispersion B5 (carrier ratio 5, SLS ratio 1; compound F48/HPMC/SLS = 1:4:1) prepared in the same manner as in Example 4 in water for injection. An HPMC/SLS composition without the compound F48 was administered as a control. After administration, the pain threshold was measured over time by the up-down method using a von Frey filament (STOELTING CO: TOUCH-TEST SENSORY EVALUATOR). As a result, the solid dispersion B5 exhibited a significant analgesic effect in the groups dosed with 3 mg/kg or more of the compound F48, as compared with the control group (Fig. 4). Furthermore, in the 10 mg/kg dosing group, a significant analgesic effect was observed from 3 to 8 hours after administration.

### Example 8. Solid Dispersion of Compound F2

### 8-1) Production of Solid Dispersion

A solid dispersion (carrier ratio: 3; SLS ratio: 0.6; compound F2/HPMC/SLS = 1:2.4:0.6) was produced by the above-described spray drying method using the compound F2 as follows.

### (1) Preparation of Spray Liquid

First, 25.0 g of the compound F2 was dissolved in 7,250 g of THF (containing a stabilizer, KANTO CHEMICAL CO., INC.). In addition, 60.0 g of HPMC (Shin-Etsu Chemical Co., Ltd.) was added and dispersed in 1,776 g of ethanol (KANTO CHEMICAL CO., INC.). Then, 750 g of ion-exchanged water was added, and the mixture was stirred for 30 minutes. After confirming that the HPMC was dissolved, 15.0 g of SLS (KOKUSAN CHEMICAL Co., Ltd.) was added, and the mixture was stirred for 20 minutes. The two liquids were mixed and stirred for 1 hour to prepare a spray liquid with a weight ratio of compound F2/HPMC/SLS of 1:2.4:0.6.

### (2) Spray Drying and Secondary Drying

The prepared spray liquid was pumped into a spray dryer (CL-8i, OHKAWARA KAKOHKI CO., LTD.) via a peristaltic pump. Spray drying was performed under the following conditions: raw liquid processing rate was 3.9 kg/h, the spray pressure of the two-fluid nozzle was 0.2 MPa, the inlet temperature was 120°C, and the outlet temperature was 67°C. The obtained primary dried product (62.05 g) was further subjected to secondary drying (40°C/approximately 8 hours) in a vacuum dryer, thereby obtaining 61.1 g of a solid dispersion as a white powder (recovery rate: 61.1%).

### 8-2) Powder X-ray Diffraction of Solid Dispersion

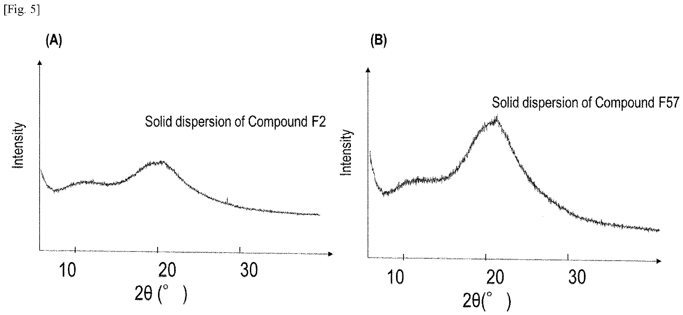

In the same manner as in Example 4, the solid dispersion of the compound F2 was analyzed by powder X-ray diffraction. As shown in Fig. 5, a halo pattern was observed, and it was revealed that the compound F2 was in an amorphous state.

### 8-3) PK Test of Solid Dispersion

Fasted male rats (8 weeks old, Wistar (Crlj:WI), Charles River Laboratories Japan, Inc.) were orally administered a crystalline form of the compound F2 suspended in a 1% methylcellulose aqueous solution or a solid dispersion comprising the compound F2 suspended in water for injection. The dosage of the compound F2 in each group was 10 mg/kg (n=3 in each group). Blood samples were collected in a time-dependent manner at 0.5, 1, 2, 4, 6, 8, and 24 hours after oral administration. The concentration of the compound F2 in the obtained plasma was measured by LC-MS/MS (MS/MS: API4000, SCIEX, LC: LC-20AD series, Shimadzu Corporation). Tₘₐₓ, Cₘₐₓ, and AUCₗₐₛₜ were calculated from the plasma concentration transition (Table 5). As shown in Table 5, the blood kinetics (Cₘₐₓ and AUCₗₐₛₜ) of the compound F2 were significantly improved by preparing a solid dispersion using a combination of HPMC and SLS as a carrier. In addition, the solid dispersion of the compound F2 exhibited a favorable analgesic effect.

**[Table 5]**

| Dosing Group | Dosage* (mg/kg) | Cₘₐₓ (ng/mL) | Tₘₐₓ (h) | AUCₗₐₛₜ (ng·h/mL) |
|---|---|---|---|---|
| Compound F2 | 10 | 386 ± 56 | 4.0 ± 0.0 | 5706 ± 520 |
| Solid Dispersion | 10 | 1900 ± 100 | 4.0 ± 0.0 | 27010 ± 2508 |

| | | | | |
|---|---|---|---|---|
| *: indicates the dosage of the compound F2. | | | | |

### Example 9. Solid Dispersion of Compound F57

### 9-1) Production of Solid Dispersion

A solid dispersion (carrier ratio: 3; SLS ratio: 0.6; compound F57/HPMC/SLS = 1:2.4:0.6) was produced by the above-described spray drying method using the compound F57 as follows.

### (1) Preparation of Spray Liquid

30.0 g of the compound F57 was dissolved in 7,710 g of THF (containing a stabilizer, KANTO CHEMICAL CO., INC.). In addition, 72.0 g of HPMC (Shin-Etsu Chemical Co., Ltd.) was added and dispersed in 2,131 g of ethanol (KANTO CHEMICAL CO., INC.). Then, 900 g of ion-exchanged water was added, and the mixture was stirred for 30 minutes. After confirming that the HPMC was dissolved, 18.0 g of SLS (KOKUSAN CHEMICAL Co., Ltd.) was added, and the mixture was stirred for 20 minutes. The two liquids were mixed and stirred to prepare a spray liquid with a weight ratio of compound F57/HPMC/SLS of 1:2.4:0.6 (i.e., a 1:3 weight ratio of compound F57 to carrier).

### (2) Spray Drying and Secondary Drying

The prepared spray liquid was pumped into a spray dryer (CL-8i, OHKAWARA KAKOHKI CO., LTD.) via a peristaltic pump. Spray drying was performed under the following conditions: raw liquid processing rate was 3.9 kg/h, the spray pressure of the two-fluid nozzle was 0.17 MPa, the inlet temperature was 120°C, and the outlet temperature was 67°C. The obtained primary dried product (62.05 g) was further subjected to secondary drying (40 °C/about 9 hours) in a vacuum dryer, thereby obtaining 83.2 g of a solid dispersion as a white powder (recovery rate: 69.3%).

### 9-2) Powder X-ray Diffraction of Solid Dispersion

In the same manner as in Example 4, the solid dispersion of the compound F57 was analyzed by powder X-ray diffraction. As shown in Fig. 5, a halo pattern was observed, and it was revealed that the compound F57 was in an amorphous state.

### Industrial Applicability

The solid dispersion of the present invention exhibits high *in vivo* absorbability and storage stability. Thus, it is a helpful therapeutic agent for neuropathic pain and the like.

## Claims

1. A solid dispersion comprising a compound represented by the following general formula (I) or (II), or a pharmacologically acceptable salt thereof: 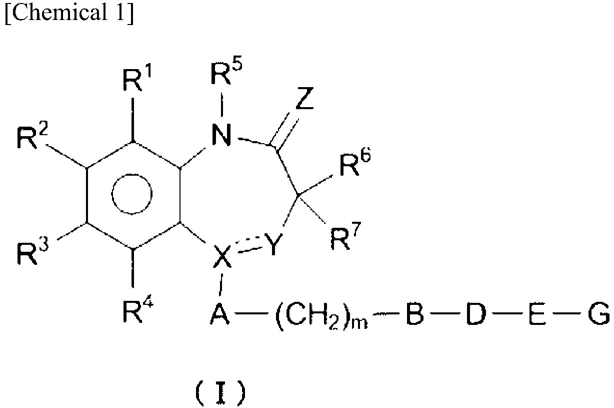 (in the formula, R¹ and R² may be the same or different and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms),a phenyl group which may have a substituent, a pyridyl group which may have a substituent, or an aralkyl group (with an aryl moiety having 6 to 10 carbon atoms and an alkylene moiety having 1 to 8 carbon atoms),
or
R¹ and R² may be taken together with a benzene ring to which they are bonded to form a fused ring selected from a naphthalene ring, a quinoline ring, an isoquinoline ring, a tetrahydronaphthalene ring, an indane ring, a tetrahydroquinoline ring, or a tetrahydroisoquinoline ring, and the ring, together with R¹ and R², which consists of carbon atoms to which R¹ and R² are respectively bonded, may be substituted with 1 to 4 substituents that may be the same or different, which are selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
R³ and R⁴ may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
R⁵ represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
R⁶ and R⁷ may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group;
X represents C, CH, or N, and
Y represents N, NH, or C(=O),
provided that when X is N, Y is not N or NH, and
when X is C or CH, Y is not C(=O),
a double line consisting of a solid line and a dashed line represents a single bond or a double bond;
Z represents an oxygen atom or a sulfur atom;
A represents a benzene ring, a pyridine ring, a thiophene ring, a pyrimidine ring, a naphthalene ring, a quinoline ring, or an indole ring which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group, or represents a bond;
B represents N(R⁸)C(=O), NHCONH, CON(R⁹), NHC(=S)NH, N(R¹⁰)SO₂, SO₂N(R¹¹), or OSO₂,
wherein R⁸, R⁹, R¹⁰ and R¹¹ represent a hydrogen atom, an alkyl group having 1 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
D represents an alkylene chain having 1 to 6 carbon atoms which may have 1 to 4 substituents that may be the same or different, which are selected from an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms), and which may further have a double bond, or represents a bond;
E represents O, S, NR¹², or a bond,
wherein R¹² represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
G represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (with an aryl moiety having 6 to 10 carbon atoms and an alkylene moiety having 1 to 8 carbon atoms), a phenyl group which may have a substituent, a pyridyl group which may have a substituent, an imidazolyl group which may have a substituent, an oxazolyl group which may have a substituent, or a thiazolyl group which may have a substituent; and
m represents an integer from 0 to 5,
wherein provided that a case in which when R¹ and R² do not together form a ring, X is C, Y is N, a double line consisting of a solid line and a dashed line is a double bond, Z is an oxygen atom, A is a benzene ring, m is 0, B is C(=O)NH, E is a bond, and G is a phenyl group,
a case in which R¹, R², R⁴, R⁵, R⁶ and R¹ are hydrogen atoms, R³ is a chlorine atom, X is C, Y is N, a double line consisting of a solid line and a dashed line is a double bond, Z is an oxygen atom, A is a bond, m is 1, B is NHC(=O), D is a methylene group or a bond, E is a bond, and G is a phenyl group,
and a case in which R¹, R², R³, R⁴, R⁵, R⁶ and R¹ are hydrogen atoms, X is N, Y is C(=O), a double line consisting of a solid line and a dashed line is a single bond, Z is an oxygen atom, A is a bond, m is 1, B is CON(R⁹), R⁹ is an isopropyl group, E is a bond, G is a phenyl group with an optional substitution of a methoxy group are excluded). 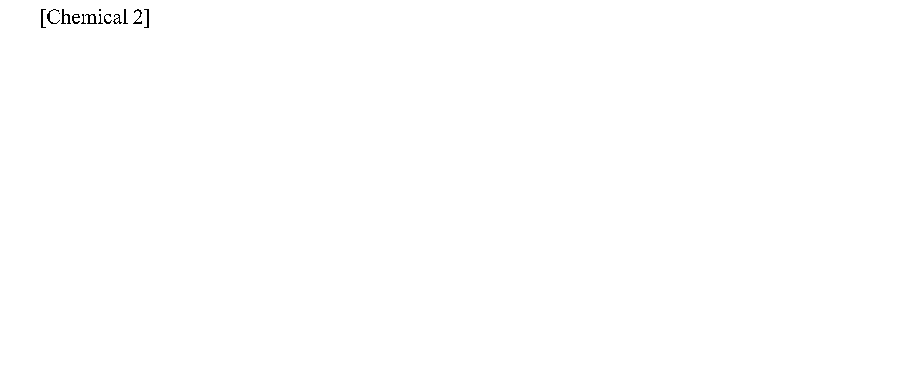 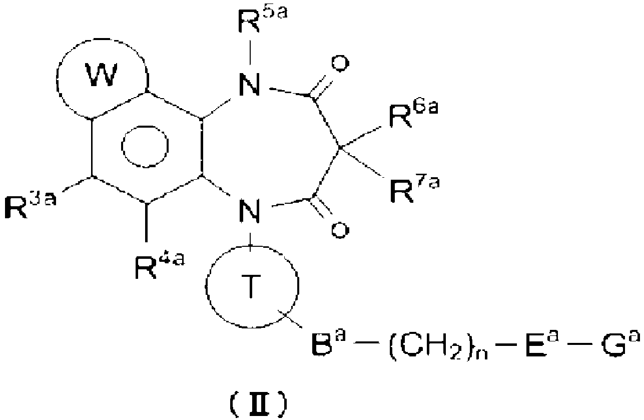 (in the formula, 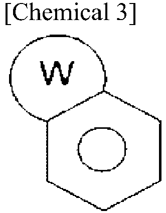 represents a naphthalene ring, a quinoline ring, an isoquinoline ring, a tetrahydronaphthalene ring, an indane ring, a tetrahydroquinoline ring, or a tetrahydroisoquinoline ring;
these rings may be substituted with 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
R^{3a} and R^{4a} may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (with an alkoxy moiety having 1 to 8 carbon atoms), or an aralkyl group (with an aryl moiety having 6 to 10 carbon atoms and an alkylene moiety having 1 to 8 carbon atoms);
R^{5a} represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
R^{6a} and R^{7a} may be the same or different and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, or an amino group; 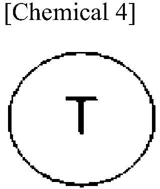 represents a benzene ring, a pyridine ring, a thiophene ring, a pyrimidine ring, a naphthalene ring, a quinoline ring, or an indole ring which may have 1 to 4 substituents that may be the same or different, which are selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (with an aryl moiety having 6 to 10 carbon atoms and an alkylene moiety having 1 to 8 carbon atoms), a phenyl group, or a pyridyl group, or represents a bond;
B^{a} represents N(R^{8a})C(=O), NHCONH, CON(R^{9a}), NHC(=S)NH, N(R^{10a})SO₂, SO₂N(R^{11a}), or OSO₂,
wherein R^{8a}, R^{9a}, R^{10a} and R^{11a} represent a hydrogen atom, an alkyl group having 1 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
E^{a} represents O, S, NR^{12a}, or a bond,
wherein R^{12a} represents a hydrogen atom, an alkyl group having 1 to 8 carbon atoms, an alkenyl group having 2 to 8 carbon atoms, an alkyl group having 1 to 8 carbon atoms substituted with 1 to 3 halogen atoms, an alkyl group having 1 to 8 carbon atoms substituted with hydroxyl groups, or an aralkyl group (with an aryl moiety having 6 to 10 carbon atoms and an alkylene moiety having 1 to 8 carbon atoms);
G^{a} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine which may have 1 to 4 identical or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms substituted with 1 to 3 halogen atoms, an alkoxy group having 1 to 8 carbon atoms substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms,
an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (with an aryl moiety having 6 to 10 carbon atoms and an alkylene moiety having 1 to 8 carbon atoms), a phenyl group which may have a substituent, a pyridyl group which may have a substituent, an imidazolyl group which may have a substituent, an oxazolyl group which may have a substituent, or a thiazolyl group which may have a substituent; and
n represents an integer from 0 to 5).

2. The solid dispersion according to claim 1, comprising a combination of hydroxypropylmethylcellulose (HPMC) and sodium lauryl sulfate (SLS), or aminoalkyl methacrylate copolymer E.

3. The solid dispersion according to claim 2, comprising a combination of hydroxypropylmethylcellulose (HPMC) and sodium lauryl sulfate (SLS).

4. The solid dispersion according to claim 1, wherein the compound is the compound represented by the general formula (II), in which B^{a} is an NHCO group or an NHSO₂ group bonded to a 3rd or 4th position of a benzene ring, n is 0 to 2, E^{a} is a bond, and G^{a} is a substituted phenyl group or a substituted pyridyl group.

5. The solid dispersion according to claim 4, wherein B^{a} is an NHCO group that binds to a 4th position of a benzene ring, n is 0, and G^{a} is a substituted phenyl group.

6. The solid dispersion according to claim 5, wherein G^{a} is a trifluoromethyl group, a hydroxyl group, an alkyl group, or a phenyl group in which G^{a} is substituted with a halogen atom.

7. The solid dispersion according to claim 1, wherein the compound or a pharmacologically acceptable salt thereof is selected from the following group of (1) to (214):
(1) 5-(4-benzoylaminophenyl)-1H-naphtho [1,2-b] [1,4] diazepine-2,4 (3H,5H)-dione;
(2) 5-[4-[(2-(trifluoromethyl) benzoyl] aminophenyl]-1H-naphtho [1,2-b] [1,4] diazepine-2,4 (3H,5H)-dione;
(3) 5-[4-(3-bromobenzoyl) aminophenyl]-1H-naphtho [1,2-b] [1,4] diazepine-2,4 (3H,5H)-dione;
(4) 5-[4-[4-(trifluoromethyl) benzoyl] aminophenyl]-1H-naphtho [1,2-b] [1,4] diazepine-2,4 (3H,5H)-dione;
(5) 5-[4-(2-methylbenzoyl) aminophenyl]-1H-naphtho [1,2-b] [1,4] diazepine-2,4 (3H,5H)-dione;
(6) 5-[4-(2,6-dimethylbenzoylamino) phenyl]-1H-naphtho [1,2-b] [1,4] diazepine-2,4 (3H,5H)-dione;
(7) 5-[4-(2,6-dichlorobenzoyl) aminophenyl]-1H-naphtho [1,2-b] [1,4] diazepine-2,4 (3H,5H)-dione;
(8) 5-[4-(3-chlorobenzoyl) aminophenyl]-1H-naphtho [1,2-b] [1,4] diazepine-2,4 (3H,5H)-dione;
(9) 5 [4-(2-phenylacetylamino) phenyl]-1H-naphtho [1,2-b] [1,4] diazepine-2,4 (3H,5H)-dione;
(10) 1-[4-(2,4-dioxo-1,2,3,4-tetrahydronaphtho [1,2-b] [1,4] diazepin-5-yl) phenyl]-3-phenylthiourea;
(11) 5-[4-(2,3-dimethoxybenzoylamino) phenyl]-1H-naphtho [1,2-b] [1,4] diazepine-2,4 (3H,5H)-dione;
(12) 5-[4-(2-methoxybenzoylamino) phenyl]-1H-naphtho [1,2-b] [1,4] diazepine-2,4 (3H,5H)-dione;
(13) 5-[4-[(2-chlorophenylacetyl) amino] phenyl]-1H-naphtho [1,2-b] [1,4] diazepine-2,4 (3H,5H)-dione;
(14) 5-[4-(2,3-dimethylbenzoylamino) phenyl]-1H-naphtho [1,2-b] [1,4] diazepine-2,4 (3H,5H)-dione;
(15) 5-[4-(2,5-dimethylbenzoylamino) phenyl]-1H-naphtho [1,2-b] [1,4] diazepine-2,4 (3H,5H)-dione;
(16) 5-[4-(5-bromo-2-chlorobenzoylamino) phenyl]-1H-naphtho [1,2-b] [1,4] diazepine-2,4 (3H,5H)-dione;
(17) 5-[4-(2,4-dichlorobenzoylamino) phenyl]-1H-naphtho [1,2-b] [1,4] diazepine-2,4 (3H,5H)-dione;
(18) 5-[4-(2-hydroxybenzoylamino) phenyl]-1H-naphtho [1,2-b] [1,4] diazepine-2,4 (3H,5H)-dione;
(19) 5-[4-(2,3-dihydroxybenzoylamino) phenyl]-1H-naphtho [1,2-b] [1,4] diazepine-2,4 (3H,5H)-dione;
(20) 1-[4-(2,4-dioxo-1,2,3,4-tetrahydronaphtho [1,2-b] [1,4] diazepin-5-yl) phenyl]-3-phenylurea;
(21) 5-[4-[(2,6-dichlorophenylacetyl) amino] phenyl]-1H-naphtho [1,2-b] [1,4] diazepine-2,4 (3H,5H)-dione;
(22) 5-[4-[(2-methoxyphenylacetyl) amino] phenyl]-1H-naphtho [1,2-b] [1,4] diazepine-2,4 (3H,5H)-dione;
(23) 5-[4-[(2-hydroxyphenylacetyl) amino] phenyl]-1H-naphtho [1,2-b] [1,4] diazepine-2,4 (3H,5H)-dione;
(24) 1-(2-chlorophenyl)-3-[4-(2,4-dioxo-1,2,3,4-tetrahydronaphtho [1,2-b] [1,4] diazepin-5-yl) phenyl] thiourea;
(25) 5-[4-[3-(trifluoromethyl) benzoylamino] phenyl]-1H-naphtho [1,2-b] [1,4] diazepine-2,4 (3H,5H)-dione;
(26) 5-[4-[2-[(2-trifluoromethyl) phenyl] acetylamino] phenyl]-1H-naphtho [1,2-b] [1,4] diazepine-2,4 (3H,5H)-dione;
(27) 1-(2-chlorophenyl)-3-[4-(2,4-dioxo-1,2,3,4-tetrahydronaphtho [1,2-b] [1,4] diazepin-5-yl) phenyl] urea;
(28) 5-[4-[(2-phenylpropionyl) amino] phenyl]-1H-naphtho [1,2-b] [1,4] diazepine-2,4 (3H,5H)-dione;
(29) 5-[4-(2-chloro-3-methoxybenzoylamino) phenyl]-1H-naphtho [1,2-b] [1,4] diazepine-2,4 (3H,5H)-dione;
(30) 5-[4-(3-phenylpropionylamino) phenyl]-1H-naphtho [1,2-b] [1,4] diazepine-2,4 (3H,5H)-dione;
(31) 5-[4-[(1H-indole-3-carbonyl) amino] phenyl]-1H-naphtho [1,2-b] [1,4] diazepine-2,4 (3H,5H)-dione;
(32) 5-[4-(2-chloro-3-hydroxybenzoylamino) phenyl]-1H-naphtho [1,2-b] [1,4] diazepine-2,4 (3H,5H)-dione;
(33) 5-[4-[(2-methyl-2-phenylpropionyl) amino] phenyl]-1H-naphtho [1,2-b] [1,4] diazepine-2,4 (3H,5H)-dione;
(34) 5-[4-(2-phenoxyacetylamino) phenyl]-1H-naphtho [1,2-b] [1,4] diazepine-2,4 (3H,5H)-dione;
(35) 5-[4-[2-(2-chloro-4-methoxyphenyl) acetylamino] phenyl]-1H-naphtho [1,2-b] [1,4] diazepine-2,4 (3H,5H)-dione;
(36) 5-[4-[(1-methyl-1H-imidazole-2-carbonyl) amino] phenyl]-1H-naphtho [1,2-b] [1,4] diazepine-2,4 (3H,5H)-dione;
(37) 5-[4-[2-(2,4-dichlorophenyl) acetylamino] phenyl]-1H-naphtho [1,2-b] [1,4] diazepine-2,4 (3H,5H)-dione;
(38) 5-[4-[2-(2-chloro-4-hydroxyphenyl) acetylamino] phenyl]-1H-naphtho [1,2-b] [1,4] diazepine-2,4 (3H,5H)-dione;
(39) 5-[4-(3-phenylpropenylamino) phenyl]-1H-naphtho [1,2-b] [1,4] diazepine-2,4 (3H,5H)-dione;
(40) 5-[4-[(3-pyridylacetyl) amino] phenyl]-1H-naphtho [1,2-b] [1,4] diazepine-2,4 (3H,5H)-dione hydrochloride;
(41) 5-[4-(1H-benzimidazole-2-carbonylamino) phenyl]-1H-naphtho [1,2-b] [1,4] diazepine-2,4 (3H,5H)-dione;
(42) 1-[4-(2,3-dimethylbenzoylamino) phenyl]-7-methoxy-1H-1,5-benzodiazepine-2,4 (3H,5H)-dione;
(43) 5-[4-[(benzoylamino) methyl] phenyl]-1H-naphtho [1,2-b] [1,4] diazepine-2,4 (3H,5H)-dione;
(44) 5-[4-[(2-chlorobenzoylamino) methyl] phenyl]-1H-naphtho [1,2-b] [1,4] diazepine-2,4 (3H,5H)-dione;
(45) 1-[4-(2,3-dimethylbenzoylamino) phenyl]-7-hydroxy-1H-1,5-benzodiazepine-2,4 (3H,5H)-dione;
(46) 5-[4-(2-chlorobenzoylamino) phenyl]-1H-naphtho [1,2-b] [1,4] diazepine-2,4 (3H,5H)-dione;
(47) 5-[4-(2-bromobenzoylamino) phenyl]-1H-naphtho [1,2-b] [1,4] diazepine-2,4 (3H,5H)-dione;
(48) 5-[4-(2-iodobenzoylamino) phenyl]-1H-naphtho [1,2-b] [1,4] diazepine-2,4 (3H,5H)-dione;
(49) 5-[4-(2,3-dimethylbenzoylamino)-3-fluorophenyl]-1H-naphtho [1,2-b] [1,4] diazepine-2,4 (3H,5H)-dione;
(50) 5-[4-[2-(2-methylphenyl) acetylamino] phenyl]-1H-naphtho [1,2-b] [1,4] diazepine-2,4 (3H,5H)-dione;
(51) 5-[4-[(quinoxalin-2yl) carbonylamino] phenyl]-1H-naphtho [1,2-b] [1,4] diazepine-2,4 (3H,5H)-dione;
(52) 5-[4-[(5-methylthiophen-2yl) carbonylamino] phenyl]-1H-naphtho [1,2-b] [1,4] diazepine-2,4 (3H,5H)-dione;
(53) 5-[3-[(2-chlorophenylacetyl) amino] phenyl]-1H-naphtho [1,2-b] [1,4] diazepine-2,4 (3H,5H)-dione;
(54) 5-[4-[(2,4,6-trimethylbenzoyl) amino] phenyl]-1H-naphtho [1,2-b] [1,4] diazepine-2,4 (3H,5H)-dione;
(55) 5-[4-(cyclohexylcarbonylamino) phenyl]-1,2,3,4,8,9,10,11-octahydronaphtho [1,2-b][1,4] diazepine-2, 4 (3H,5H)-dione;
(56) 1-[4-(2,3-dimethylbenzoyl) aminophenyl]-6-methyl-1H-1,5-benzodiazepine-2,4 (3H,5H)-dione;
(57) 5-[4-[(2-ethylbenzoyl) amino] phenyl]-1H-naphtho [1,2-b] [1,4] diazepine-2,4 (3H,5H)-dione;
(58) 5-[4-[(6-methylpyridin-2-yl) carbonylamino] phenyl]-1,2,3,4,8,9,10,11-octahydronaphtho [1,2-b] [1,4] diazepine-2,4 (3H,5H)-dione;
(59) 5-[4-[(2-methylpyridin-3-yl) carbonylamino] phenyl]-1,2,3,4,8,9,10,11-octahydronaphtho [1,2-b] [1,4] diazepine-2,4 (3H,5H)-dione;
(60) 1-[4-(2,4-dioxo-1,2,3,4-tetrahydronaphtho [1,2-b] [1,4] diazepin-5-yl) phenyl]-3-(2-methylphenyl) thiourea;
(61) 5-[4-(2-methoxy-3-methylbenzoyl) aminophenyl]-1,2,3,4,8,9,10,11-octahydronaphtho [1,2-b] [1,4] diazepine-2,4 (3H,5H)-dione;
(62) 5-[4-(2,3-dichlorobenzoyl) aminophenyl]-1,2,3,4,8,9,10,11-octahydronaphtho [1,2-b][1,4] diazepine-2,4 (3H,5H)-dione;
(63) 5-[4-(2,3-dimethylbenzoylamino)-3-hydroxyphenyl]-1H-naphtho [1,2-b] [1,4] diazepine-2,4 (3H,5H)-dione;
(64) 5-[4-(2-chloro-3-methoxybenzoylamino) phenyl]-1,3-dihydronaphtho [1,2-e]-1,4-diazepin-2-one;
(65) 5-[4-[(4-dimethylaminobenzoyl) amino] phenyl]-1H-naphtho [1,2-b] [1,4] diazepine-2,4 (3H,5H)-dione;
(66) 5-[4-[2-(2,4-dichlorophenoxy) acetylamino] phenyl]-1H-naphtho [1,2-b] [1,4] diazepine-2,4 (3H,5H)-dione;
(67) 5-[4-[2-(2-methylphenoxy) acetylamino] phenyl]-1H-naphtho [1,2-b] [1,4] diazepine-2,4 (3H,5H)-dione;
(68) N-[4-(2,4-dioxo-1,2,3,4-tetrahydronaphtho [1,2-b] [1,4] diazepin-5-yl) butyl]-2-chloro -3-methoxybenzamide;
(69) 5-[4-(2-chloro-3-hydroxybenzoylamino) phenyl]-1,3-dihydronaphtho [1,2-e]-1,4-diazepin-2-one;
(70) 5-[4-(2-acetylbenzoylamino) phenyl]-1H-naphtho [1,2-b] [1,4] diazepine-2,4 (3H,5H)-dione;
(71) 5-[4-(2-tert-butylbenzoylamino) phenyl]-1H-naphtho [1,2-b] [1,4] diazepine-2,4 (3H,5H)-dione;
(72) 5-[2-(2-iodobenzoyl) aminoethyl]-1H-naphtho [1,2-b] [1,4] diazepine-2,4 (3H,5H)-dione;
(73) 5-[3-[(2-iodobenzoyl) amino] phenyl]-1H-naphtho [1,2-b] [1,4] diazepine-2,4 (3H,5H)-dione;
(74) 6,7-dimethyl-1-[4-(2-iodobenzoyl) aminophenyl]-1H-1,5-benzodiazepine-2,4 (3H,5H)-dione;
(75) 5-[4-[(1-methylpiperidin-4-yl) carbonylamino] phenyl]-1H-naphtho [1,2-b] [1,4] diazepine-2,4 (3H,5H)-dione hydrochloride;
(76) 5 [4-[(benzofuran-2-yl) carbonylamino] phenyl]-1H-naphtho [1,2-b] [1,4] diazepine-2,4 (3H,5H)-dione;
(77) 5-[4-[(1-methyl-1H-indol-3-yl) carbonylamino] phenyl]-1H-naphtho [1,2-b] [1,4] diazepine-2,4 (3H,5H)-dione;
(78) 5-[4-(2-propenylbenzoylamino) phenyl]-1H-naphtho [1,2-b] [1,4] diazepine-2,4 (3H,5H)-dione;
(79) 5-[4-(2-propylbenzoylamino) phenyl]-1H-naphtho [1,2-b] [1,4] diazepine-2,4 (3H,5H)-dione;
(80) 5-[3-fluoro-4-(2-iodobenzoylamino) phenyl]-1H-naphtho [1,2-b] [1,4] diazepine-2,4 (3H,5H)-dione;
(81) 5-[4-(2-hydroxy-3-methylbenzoyl) aminophenyl]-1,2,3,4,8,9,10,11-octahydronaphtho [1,2-b] [1,4] diazepine-2,4 (3H,5H)-dione;
(82) 5-[4-[(2-isopropoxybenzoyl) amino] phenyl]-1H-naphtho [1,2-b] [1,4] diazepine-2,4 (3H,5H)-dione;
(83) 5-[4-[(3-methylthiophen-2-yl) carbonylamino] phenyl]-1H-naphtho [1,2-b] [1,4] diazepine-2,4 (3H,5H)-dione;
(84) 5-[4-(2-phenoxypropionylamino) phenyl]-1H-naphtho [1,2-b] [1,4] diazepine-2,4 (3H,5H)-dione;
(85) 5-[4-[2-(4-chloro-2-methylphenoxy) acetylamino] phenyl]-1H-naphtho [1,2-b] [1,4] diazepine-2,4 (3H,5H)-dione;
(86) 5-[4-(4-fluoro-2-trifluoromethylbenzoyl) aminophenyl]-1H-naphtho [1,2-b] [1,4] diazepine-2,4 (3H,5H)-dione;
(87) 5-[4-(4-fluoro-2-methoxybenzoyl) aminophenyl]-1H-naphtho [1,2-b] [1,4] diazepine-2,4 (3H,5H)-dione;
(88) 5-[4-(4-fluoro-2-hydroxybenzoyl) aminophenyl]-1H-naphtho [1,2-b] [1,4] diazepine-2,4 (3H,5H)-dione;
(89) 5-[3-[(2-iodophenylacetyl) amino] phenyl]-1H-naphtho [1,2-b] [1,4] diazepine-2,4 (3H,5H)-dione;
(90) 5-[4-(2-methyl-2-phenoxypropionylamino) phenyl]-1H-naphtho [1,2-b] [1,4] diazepine-2,4 (3H,5H)-dione;
(91) 5-[4-(2-tert-butylbenzoylamino) phenyl]-1,3-dihydronaphtho [1,2-e]-1,4-diazepin-2-one;
(92) 5-[4-[(3-dimethylaminobenzoyl) amino] phenyl]-1H-naphtho [1,2-b] [1,4] diazepine-2,4 (3H,5H)-dione;
(93) 5-[4-(4-iodo-2-methoxybenzoylamino) phenyl]-1H-naphtho [1,2-b] [1,4] diazepine-2,4 (3H,5H)-dione;
(94) 5-[4-(6-fluoro-2-methoxybenzoylamino) phenyl]-1H-naphtho [1,2-b] [1,4] diazepine-2,4 (3H,5H)-dione;
(95) 5-[4-(2-hydroxy-4-iodobenzoylamino) phenyl]-1H-naphtho [1,2-b] [1,4] diazepine-2,4 (3H,5H)-dione;
(96) 5-[4-(6-fluoro-2-hydroxyamino) phenyl]-1H-naphtho [1,2-b] [1,4] diazepine-2,4 (3H,5H)-dione;
(97) 5-[4-(2-fluorobenzoyl) aminophenyl]-1H-naphtho [1,2-b] [1,4] diazepine-2,4 (3H,5H)-dione;
(98) 5-[4-[(2-dimethylaminobenzoyl) amino] phenyl]-1H-naphtho [1,2-b] [1,4] diazepine-2,4 (3H,5H)-dione;
(99) 5-[4-(2-methoxy-6-methylbenzoylamino) phenyl]-1H-naphtho [1,2-b] [1,4] diazepine-2,4 (3H,5H)-dione;
(100) 5-[4-(2-hydroxy-6-methylbenzoylamino) phenyl]-1H-naphtho [1,2-b] [1,4] diazepine-2,4 (3H,5H)-dione;
(101) 5-[4-[3-(2-methylphenyl) propionylamino] phenyl]-1H-naphtho [1,2-b] [1,4] diazepine-2,4 (3H,5H)-dione;
(102) 5-(4-phenylcarbamoylphenyl) -1H-naphtho [1,2-b] [1,4] diazepine-2,4 (3H,5H)-dione;
(103) 5-(4-benzylcarbamoylphenyl)-1H-naphtho [1,2-b] [1,4] diazepine-2,4 (3H,5H)-dione;
(104) 5-[4-[3-(2-methylphenyl) propenoylamino] phenyl]-1H-naphtho [1,2-b] [1,4] diazepine-2,4 (3H,5H)-dione;
(105) 5-[4-[3-(2-chlorophenyl) propionylamino] phenyl]-1H-naphtho [1,2-b] [1,4] diazepine-2,4 (3H,5H)-dione;
(106) 5-[4-(2-iodobenzoyl) aminophenyl]-1,2,3,4,8,9,10,11-octahydronaphtho [1,2-b] [1,4] diazepine-2,4 (3H,5H)-dione;
(107) 5-[4-[(1-methyl-1H-pyrrol-2-ylacetyl) amino] phenyl]-1,2,3,4,8,9,10,11-octahydronaphtho [1,2-b] [1,4] diazepine-2,4 (3H,5H)-dione;
(108) 5-[4-(2-chlorobenzyl) carbamoylphenyl]-1H-naphtho [1,2-b] [1,4] diazepine-2,4 (3H,5H)-dione;
(109) 5-[4-[3-(2-chlorophenyl) propenoylamino] phenyl]-1H-naphtho [1,2-b] [1,4] diazepine-2,4 (3H,5H)-dione;
(110) 5-[4-(2-chlorophenyl) carbamoylphenyl]-1H-naphtho [1,2-b] [1,4] diazepine-2,4 (3H,5H)-dione;
(111) 5-[4-(6-bromo-2,3-methylenedioxybenzoylamino) phenyl]-1H-naphtho [1,2-b] [1,4] diazepine-2,4 (3H,5H)-dione;
(112) 5-[4-(6-bromo-2-methoxybenzoylamino) phenyl]-1H-naphtho [1,2-b] [1,4] diazepine-2,4 (3H,5H)-dione;
(113) 5-[4-[(2-tert-butylbenzoyl) amino] phenyl]-1,2,3,4,8,9,10,11-octahydronaphtho [1,2-b][1,4] diazepine-2,4 (3H,5H)-dione;
(114) 5-[2-(2-iodobenzoyl) aminopyridin-5-yl]-1H-naphtho [1,2-b] [1,4] diazepine-2,4 (3H,5H)-dione;
(115) 5-[4-(6-bromo-2-hydroxybenzoylamino) phenyl]-1H-naphtho [1,2-b] [1,4] diazepine-2,4 (3H,5H)-dione;
(116) 5-[4-(6-chloro-2-methoxybenzoylamino) phenyl]-1H-naphtho [1,2-b] [1,4] diazepine-2,4 (3H,5H)-dione;
(117) 5-[4-(2-iodobenzoylamino) phenyl]-1H-[1,4] diazepino [2,3-h] quinoline-2,4 (3H,5H)-dione;
(118) 5-[4-(6-chloro-2-hydroxybenzoylamino) phenyl]-1H-naphtho [1,2-b] [1,4] diazepine-2,4 (3H,5H)-dione;
(119) 5-[4-(2-hydroxy-6-methoxybenzoylamino) phenyl]-1H-naphtho [1,2-b] [1,4] diazepine-2,4 (3H,5H)-dione;
(120) 5-[4-[2-methoxy-6-(trifluoromethyl) benzoylamino] phenyl]-1H-naphtho [1,2-b] [1,4] diazepine-2,4 (3H,5H)-dione;
(121) 5-[4-[2-hydroxy-6-(trifluoromethyl) benzoylamino] phenyl]-1H-naphtho [1,2-b] [1,4] diazepine-2,4 (3H,5H)-dione;
(122) 5-[4-[(2-isopropenylbenzoyl) amino] phenyl]-1,2,3,4,8,9,10,11-octahydronaphtho [1,2-b] [1,4] diazepine-2,4 (3H,5H)-dione;
(123) 5-[4-[(2-isopropylbenzoyl) amino] phenyl]-1,2,3,4,8,9,10,11-octahydronaphtho [1,2-b][1,4] diazepine-2,4 (3H,5H)-dione;
(124) 5-[4-[2-chloro-5-(methylthio) benzoylamino] phenyl]-1H-naphtho [1,2-b] [1,4] diazepine-2,4 (3H,5H)-dione;
(125) 5-[4-[2-(methylthio) benzoylamino] phenyl]-1H-naphtho [1,2-b] [1,4] diazepine-2,4 (3H,5H)-dione;
(126) 5-[4-[3-(methylthio) benzoylamino] phenyl]-1H-naphtho [1,2-b] [1,4] diazepine-2,4 (3H,5H)-dione;
(127) 5-[4-[2-ethyl-6-methoxybenzoylamino] phenyl]-1H-naphtho [1,2-b][1,4] diazepine-2,4 (3H,5H)-dione;
(128) 5-[4-(3-methanesulfonylbenzoylamino) phenyl]-1H-naphtho [1,2-b] [1,4] diazepine-2,4 (3H,5H)-dione;
(129) 6-ethyl-1-[4-(2-iodobenzoyl) aminophenyl]-1H-1,5-benzodiazepine-2,4 (3H,5H)-dione;
(130) 5-[4-[2-ethyl-6-hydroxybenzoylamino] phenyl]-1H-naphtho [1,2-b] [1,4] diazepine-2,4 (3H,5H)-dione;
(131) 5-[4-(3-methanesulfinylbenzoylamino) phenyl]-1H-naphtho [1,2-b] [1,4] diazepine-2,4 (3H,5H)-dione;
(132) 5-[4-(2-chloro-5-methanesulfinylbenzoylamino) phenyl]-1H-naphtho [1,2-b] [1,4] diazepine-2,4 (3H,5H)-dione;
(133) 5-[4-(2-methanesulfinylbenzoylamino) phenyl]-1H-naphtho [1,2-b] [1,4] diazepine-2,4 (3H,5H)-dione;
(134) 5-[4-[[2-(4-morpholinyl) acetyl] amino] phenyl]-1,2,3,4,8,9,10,11-octahydronaphtho [1,2-b] [1,4] diazepine-2,4 (3H,5H)-dione hydrochloride;
(135) 5-[4-(2-chloro-6-methoxybenzoylamino) phenyl]-1,3-dihydronaphtho [1,2-e]-1,4-diazepin-2-one;
(136) 5-[4-[[(3-chloropyridin-2-yl) carbonyl] amino] phenyl]-1H-naphtho [1,2-b] [1,4] diazepine-2,4 (3H,5H)-dione;
(137) 5-[4-(2-chloro-6-hydroxybenzoylamino) phenyl]-1,3-dihydronaphtho [1,2-e]-1,4-diazepin-2-one;
(138) 5-[4-(3-chloro-2-methoxybenzoylamino) phenyl]-1H-naphtho [1,2-b] [1,4] diazepine-2,4 (3H,5H)-dione;
(139) 5-[4-[(3-methylpyridin-2-yl) carbonylamino] phenyl]-1H-naphtho [1,2-b] [1,4] diazepine-2,4 (3H,5H)-dione;
(140) 5-[4-[[(3-chloropyridin-2-yl) carbonyl] amino] phenyl]-1,2,3,4,8,9,10,11-octahydronaphtho [1,2-b] [1,4] diazepine-2,4 (3H,5H)-dione;
(141) 5-[4-(3-chloro-2-hydroxybenzoylamino) phenyl]-1H-naphtho [1,2-b] [1,4] diazepine-2,4 (3H,5H)-dione;
(142) 5-[4-[[(3-hydroxypyridin-2-yl) carbonyl] amino] phenyl]-1H-naphtho [1,2-b] [1,4] diazepine-2,4 (3H,5H)-dione;
(143) 5-[4-[(3-vinylpyridin-2-yl) carbonylamino] phenyl]-1H-naphtho [1,2-b] [1,4] diazepine-2,4 (3H,5H)-dione;
(144) 5-[4-[(3-ethylpyridin-2-yl) carbonylamino] phenyl]-1H-naphtho [1,2-b] [1,4] diazepine-2,4 (3H,5H)-dione;
(145) N-[4-(2,4-dioxo-1,2,3,4-tetrahydronaphtho-[1,2-b] [1,4]-diazepin-5-yl)phenyl]-2-nitrobenzenesulfonamide;
(146) N-[4-(2,4-dioxo-1,2,3,4-tetrahydronaphtho [1,2-b] [1,4] diazepin-5-yl) phenyl] benzenesulfonamide;
(147) 3-bromo-N-[4-(2,4-dioxo-1,2,3,4-tetrahydronaphtho [1,2-b] [1,4] diazepin-5-yl) phenyl] benzenesulfonamide;
(148) N-[4-(2,4-dioxo-1,2,3,4-tetrahydronaphtho [1,2-b] [1,4] diazepin-5-yl) phenyl]-3-methoxybenzenesulfonamide;
(149) N-[3-(2-oxo-2,3-dihydro-1H-naphtho [1,2-e] [1,4] diazepin-5-yl) phenyl] benzenesulfonamide;
(150) N-[3-(2,4-dioxo-1,2,3,4-tetrahydronaphtho-[1,2-b][1,4]-diazepin-5-yl) phenyl]-2-nitrobenzenesulfonamide;
(151) N-[4-(2,4-dioxo-1,2,3,4,8,9,10,11-octahydro-naphtho [1,2-b] [1,4]-diazepin-5-yl) phenyl]-2-nitro-benzenesulfonamide;
(152) N-[4-(2,4-dioxo-1,2,3,4,8,9,10,11-octahydro-naphtho [1,2-b] [1,4]-diazepin-5-yl) phenyl]-N-methyl-2-nitrobenzenesulfonamide;
(153) N-[3-(2,4-dioxo-1,2,3,4-tetrahydronaphtho-[1,2-b] [1,4]-diazepin-5-yl) phenyl]-N-methyl-2-nitrobenzenesulfonamide;
(154) 4-(2,4-dioxo-1,2,3,4,8,9,10,11-octahydronaphtho [1,2-b] [1,4] diazepin-5-yl)-N-phenylbenzenesulfonamide;
(155) N-[3-(2,4-dioxo-1,2,3,4-tetrahydronaphtho [1,2-b]-[1,4] diazepin-5-yl) phenyl]-2-naphthalenesulfonamide;
(156) N-[3-(2,4-dioxo-1,2,3,4-tetrahydronaphtho [1,2-b]-[1,4] diazepin-5-yl) phenyl]-1-naphthalenesulfonamide;
(157) N-[4-(2,4-dioxo-1,2,3,4-tetrahydronaphtho [1,2-b] [1,4] diazepin-5yl) phenyl] cyclohexanesulfonamide;
(158) N-[4-(2,4-dioxo-1,2,3,4-tetrahydronaphtho [1,2-b] [1,4] diazepin-5yl) phenyl]-3-pyridinesulfonamide hydrochloride;
(159) N-[4-(2,4-dioxo-1,2,3,4-tetrahydronaphtho [1,2-b] [1,4] diazepin-5-yl) phenyl]-4-isopropylbenzenesulfonamide;
(160) N-[4-(2,4-dioxo-1,2,3,4-tetrahydronaphtho [1,2-b] [1,4] diazepin-5-yl) phenyl] phenylmethanesulfonamide;
(161) N-[4-(2,4-dioxo-1,2,3,4-tetrahydronaphtho [1,2-b] [1,4] diazepin-5yl) phenyl]-3-pyridinesulfonamide;
(162) N-[4-(2,4-dioxo-1,2,3,4-tetrahydronaphtho [1,2-b] [1,4] diazepin-5yl) phenyl]-2-naphthalenesulfonamide;
(163) 4-(2,4-dioxo-1,2,3,4,8,9,10,11-octahydronaphtho-[1,2-b] [1,4] diazepin-5-yl) phenyl 3-bromobenzene-sulfonate;
(164) N-benzyl-N-[4-(1-benzyl-2,4-dioxo-1,2,3,4-tetrahydronaphtho [1,2-b] [1,4] diazepin-5yl) phenyl]-2-nitrobenzenesulfonamide;
(165) N-benzyl-N-[4-(2,4-dioxo-1,2,3,4-tetrahydronaphtho [1,2-b] [1,4] diazepin-5yl) phenyl]-2-nitrobenzenesulfonamide;
(166) 3-bromo-N-[4-(2,4-dioxo-1,2,3,4-tetrahydronaphtho [1,2-b] [1,4] diazepin-5-yl) phenyl]-N-methylbenzenesulfonamide;
(167) N-[4-(2,4-dioxo-1,2,3,4-tetrahydronaphtho-[1,2-b][1,4]-diazepin-5-yl)phenyl]-N-methyl-2-nitrobenzenesulfonamide;
(168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(169) N-[4-(7-chloro-2,4-dioxo-2,3,4,5-tetrahydro-1H-benzo [b] [1,4] diazepin-1-yl) phenyl] benzenesulfonamide;
(170) N-[4-(7-bromo-2,4-dioxo-2,3,4,5-tetrahydro-1H-benzo [b] [1,4] diazepin-1-yl) phenyl] benzenesulfonamide;
(171) N-[4-[(2,4-dioxo-7-(trifluoromethyl)-2,3,4,5-tetrahydro-1H-benzo [b] [1,4] diazepin-1-yl)] phenyl] benzenesulfonamide;
(172) N-[4-(2,4-dioxo-2,3,4,5-tetrahydro-1H-benzo [b] [1,4] diazepin-1-yl) phenyl] benzenesulfonamide;
(173) 1-(2-chlorophenyl)-N-[4-(2,4-dioxo-1,2,3,4-tetrahydronaphtho [1,2-b] [1,4] diazepin-5-yl) phenyl] methanesulfonamide;
(174) 1-(3-bromophenyl)-N-[4-(2,4-dioxo-1,2,3,4-tetrahydronaphtho [1,2-b] [1,4] diazepin-5-yl) phenyl] methanesulfonamide;
(175) N-[4-(2,4-dioxo-1,2,3,4-tetrahydronaphtho-[1,2-b][1,4]-diazepin-5-yl)phenyl]-2-trifluoromethylbenzenesulfonamide;
(176) N-[4-(7-bromo-6-methyl-2,4-dioxo-2,3,4,5-tetrahydro-1H-benzo [b] [1,4] diazepin-1-yl) phenyl] benzenesulfonamide;
(177) 1-(2-chlorophenyl)-N-[4-(2,4-dioxo-1,2,3,4,8,9,10,11-octahydronaphtho [1,2-b] [1,4] diazepin-5-yl) phenyl] methanesulfonamide;
(178) 3-bromo-N-[4-(2,4-dioxo-1,2,3,4,8,9,10,11-octahydronaphtho [1,2-b] [1,4] diazepin-5-yl) phenyl] benzenesulfonamide;
(179) N-[4-(2,4-dioxo-1,2,3,4,8,9,10,11-octahydronaphtho [1,2-b] [1,4] diazepin-5-yl) phenyl]-3-methoxybenzenesulfonamide;
(180) 1-(2-bromophenyl)-N-[4-(2,4-dioxo-1,2,3,4-tetrahydronaphtho [1,2-b] [1,4] diazepin-5-yl) phenyl] methanesulfonamide;
(181) N-[4-(2,4-dioxo-1,2,3,4-tetrahydronaphtho [1,2-b] [1,4] diazepin-5-yl) phenyl]-1-(2-methylphenyl) methanesulfonamide;
(182) N-[4-(2,4-dioxo-1,2,3,4-tetrahydronaphtho [1,2-b] [1,4] diazepin-5-yl) phenyl]-1-(2-nitrophenyl) methanesulfonamide;
(183) N-[4-(2,4-dioxo-1,2,3,4-tetrahydronaphtho [1,2-b] [1,4] diazepin-5-yl) phenyl]-2-phenylethanesulfonamide;
(184) 1-(2,3-dichlorophenyl)-N-[4-(2,4-dioxo-1,2,3,4,8,9,10,11-octahydronaphtho [1,2-b][1,4] diazepin-5-yl) phenyl] methanesulfonamide;
(185) 1-(2-chlorophenyl)-N-[4-(2,4-dioxo-7-methoxy-1H-benzo [1,2-b] [1,4] diazepin-1-yl) phenyl] methanesulfonamide;
(186) 1-(2-chlorophenyl)-N-[4-(2,4-dioxo-7-hydroxy-1H-benzo [1,2-b] [1,4] diazepin-1-yl) phenyl] methanesulfonamide;
(187) 1-(4-chlorophenyl)-N-[4-(2,4-dioxo-1,2,3,4-tetrahydronaphtho [1,2-b] [1,4] diazepin-5-yl) phenyl] methanesulfonamide;
(188) 1-(2-chlorophenyl)-N-[4-(2,4-dioxo-1,2,3,4-tetrahydronaphtho [1,2-b] [1,4] diazepin-5-yl) benzyl] methanesulfonamide;
(189) 1-(2-chlorophenyl)-N-[4-(2,4-dioxo-1,2,3,4-tetrahydronaphtho [1,2-b] [1,4] diazepin-5-yl)-2-methoxyphenyl] methanesulfonamide;
(190) 1-(2-chlorophenyl)-N-[4-(2,4-dioxo-1,2,3,4-tetrahydronaphtho [1,2-b] [1,4] diazepin-5-yl)-2-hydroxyphenyl] methanesulfonamide;
(191) 1-(2,6-dichlorophenyl)-N-[4-(2,4-dioxo-1,2,3,4,8,9,10,11-octahydronaphtho [1,2-b][1,4] diazepin-5-yl) phenyl] methanesulfonamide;
(192) 1-(2-chlorophenyl)-N-[4-(2,4-dioxo-6-methyl-1H-benzo [1,2-b] [1,4] diazepin-1-yl) phenyl] methanesulfonamide;
(193) 1-(2-chlorophenyl)-N-[4-(2,4-dioxy-1,2,3,4-tetrahydronaphtho [1,2-b] [1,4] diazepin-5-yl) propyl] methanesulfonamide;
(194) 1-(2-chlorophenyl)-N-[2-(2,4-dioxo-1,2,3,4-tetrahydronaphtho [1,2-b] [1,4] diazepin-5-yl) ethyl] methanesulfonamide;
(195) N-[4-(2,4-dioxo-1,2,3,4,8,9,10,11-octahydronaphtho [1,2-b] [1,4] diazepin-5-yl) phenyl]-1-(2-iodophenyl) methanesulfonamide;
(196) 1-(2-chlorophenyl)-N-[4-(2,4-dioxo-1,2,3,4,8,9,10,11-octahydronaphtho [1,2-b] [1,4] diazepin-5-yl) phenyl]-N-methylmethanesulfonamide;
(197) 1-(2-chlorophenyl)-N-[4-(2-oxo-2,3-dihydro-1H-naphtho [1,2-e] [1,4] diazepin-5-yl) phenyl] methanesulfonamide;
(198) 1-[(2-trifluoromethyl) phenyl]-N-[4-(2,4-dioxo-1,2,3,4-tetrahydronaphtho [1,2-b] [1,4] diazepin-5-yl) phenyl] phenyl-N-methylmethanesulfonamide;
(199) 1-(2-ethylphenyl)-N-[4-(2,4-dioxo-1,2,3,4-tetrahydronaphtho [1,2-b] [1,4] diazepin-5-yl) phenyl] phenyl-N-methylmethanesulfonamide;
(200) 1-(2,3-dimethylphenyl)-N-[4-(2,4-dioxo-1,2,3,4-tetrahydronaphtho [1,2-b] [1,4] diazepin-5-yl) phenyl] phenyl-N-methylmethanesulfonamide;
(201) 2-(2-chlorophenyl)-N-[4-(2,4-dioxo-1,2,3,4-tetrahydronaphtho [1,2-b] [1,4] diazepin-5-yl) phenyl] phenyl-N-methylethanesulfonamide;
(202) 1-(2-nitrophenyl)-N-[4-(2,4-dioxo-1,2,3,4-tetrahydronaphtho [1,2-b] [1,4] diazepin-5-yl) phenyl] phenyl-N-methylmethanesulfonamide;
(203) 1-(2-aminophenyl)-N-[4-(2,4-dioxo-1,2,3,4-tetrahydronaphtho [1,2-b] [1,4] diazepin-5-yl) phenyl] phenyl-N-methylmethanesulfonamide;
(204) 1-(2-dimethylaminophenyl)-N-[4-(2,4-dioxo-1,2,3,4-tetrahydronaphtho [1,2-b] [1,4] diazepin-5-yl) phenyl] phenyl-N-methylmethanesulfonamide;
(205) 5-[4-[(pyridin-4-yl) carbonylamino] phenyl]-1H-naphtho [1,2-b] [1,4] diazepine-2,4 (3H,5H)-dione hydrochloride;
(206) 5-[4-[2-[(pyridin-3-yl) oxy] acetylamino] phenyl]-1H-naphtho [1,2-b] [1,4] diazepine-2,4 (3H,5H)-dione hydrochloride;
(207) 5-[4-[(pyridin-3-yl) carbonylamino] phenyl]-1H-naphtho [1,2-b] [1,4] diazepine-2,4 (3H,5H)-dione hydrochloride;
(208) 5-[4-[(2-methylpyridin-3-yl) carbonylamino] phenyl]-1H-naphtho [1,2-b] [1,4] diazepine-2,4 (3H,5H)-dione hydrochloride;
(209) 5-[4-[(2-chloropyridin-3-yl) carbonylamino] phenyl]-1H-naphtho [1,2-b] [1,4] diazepine-2,4 (3H,5H)-dione;
(210) 5-[4-[2-[(pyridin-2-yl) oxy] acetylamino] phenyl]-1H-naphtho [1,2-b] [1,4] diazepine-2,4 (3H,5H)-dione;
(211) 5-[4-[[4-(trifluoromethyl) pyridin-3-yl] carbonylamino] phenyl]-1H-naphtho [1,2-b][1,4] diazepine-2,4 (3H,5H)-dione;
(212) 5-[4-[(2-chloropyridin-3-yl) carbonylamino] phenyl]-1H-[1,4] diazepino [2,3-f] isoquinoline-2,4 (3H,5H)-dione;
(213) 5-[4-[(2-chloropyridin-3-yl) carbonylamino] phenyl]-8,9,10,11-tetrahydro-1H-[1,4] diazepino [2,3-f] Isoquinoline-2,4 (3H,5H)-dione; and
(214) 5-[4-[(2-isopropylbenzoyl) amino] phenyl]-1H-naphtho [1,2-b] [1,4] diazepine-2,4 (3H,5H)-dione.

8. The solid dispersion according to claim 1, wherein the compound or a pharmacologically acceptable salt thereof is amorphous.

9. The solid dispersion according to claim 1, which has a carrier ratio of 1 to 25.

10. The solid dispersion according to claim 3, which has an SLS ratio of 0.1 to 10.

11. A method for producing the solid dispersion according to claim 1, **characterized by** producing the solid dispersion using a spray drying method.

12. A pharmaceutical composition comprising the solid dispersion according to claim 1.

13. The pharmaceutical composition according to claim 12, which is a solid preparation.
